# EUROPEAN PATENT APPLICATION

(11) **EP 2 223 914 A1**
(43) Date of publication of application: **01.09.2010**
(21) Application number: 10154463.3
(22) Date of filing: 24.02.2010
(51) Int. Cl.: C07D 231/54, C07D 491/04, A61K 31/4184, A61K 31/4188, A61K 31/4155, A61K 31/416, A61P 3/00, A61P 25/00, A61P 35/00

(54) **Tricyclic condensed pyrazole derivatives as CB1 inhibitors**

(30) Priority: 25.02.2009 IT MI20090263
(71) Applicant: Neuroscienze Pharmaness S.C. A R.L., 09010 Pula (CA) (IT)
(72) Inventor: Lazzari, Paolo, 09010, Pula (CAGLIARI) (IT); Loriga, Giovanni, 07100, SASSARI (IT); Manca, Ilaria, 07100, SASSARI (IT); Pinna, Gerard Aime, 07100, SASSARI (IT)
(74) Representative: Sama, Daniele

(57) **Abstract**

Condensed tricyclic compounds having a condensed structure containing one phenyl and one pyrazole ring linked with each other by a central ring rcomprising from five to eight atoms, having affinity for the CB1 and/or CB2 receptors, with central nervous system and/or peripheral activity, of formula (I) : 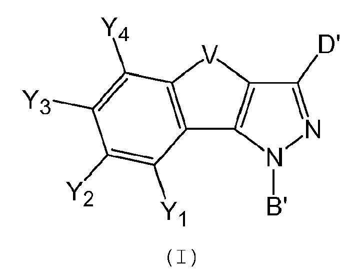 wherein the various substituents are as defined in the description. The compounds show affinity for the CB1 and/or CB2 cannabinoidergic receptors.

## Description

The present invention relates to pyrazole derivatives having affinity for the CB1 and/or CB2 cannabinoidergic receptors, the corresponding solvates and pharmaceutically acceptable salts, and the pharmaceutical compositions containing them.

More specifically the invention relates to condensed pyrazole tricyclic derivatives having affinity for the CB1 and/or CB2 cannabinoidergic receptors having in vivo activity both at a peripheral level and on the central nervous system.

Cannabinoids are compounds derived from *Cannabis sativa,* commonly known as marijuana. Among the at least 66 cannabinoid compounds characterizing marijuana, tetrahydrocannabinols (THC) and Δ⁹-tetrahydrocannabinol (Δ⁹-the) in particular, are considered to be those most active. To said compounds have indeed been correlated the properties which have brought to the use of marijuana as therapeutic agent of natural origin both in mammals and in human beings. Said properties are the following: the analgesic effect, the antiinflammatory activity, the reduction of the blood and intraocular pressure, the antiemetic activity. To tetrahydrocannabinols the negative effects which are associated to the marijuana use have furthermore been correlated, with particular reference to the perception psychological distortion, to the motory coordination loss, to the euphoria, to the sedative effect. The pharmacological action of cannabinoids appears directly correlated to their affinity towards two different classes of specific receptors belonging to the family of the "G protein-coupled" receptors: the CB1 receptors, located in the central nervous system besides that in peripheral tissues, and the CB2 receptors, found in the cerebellum (Q.J.Lu et al.; Visual Neurosci.; 2000, 17,9 1-95) but which are mostly found in peripheral tissues (M.Glass; Progr. Neuro-Psychopharmacol. & Biol. Psychiat.; 2001, 25, 743-765). In the brain the CB1 receptors are abundantly expressed in the hippocampus, in the cortical regions, in the cerebellum and inside the basal ganglia. Among the peripheral tissues wherein the CB1 receptors have been found, the testicles, the small intestine, the vesica, the deferent duct can be mentioned. The CB1 receptors have been furthermore identified both in the rat eye and in the human eye, both in the retina and in the iris and in the ciliary body (A. Porcella et al.; Molecular Brain Research; 1998, 58, 240-245; A. Porcella et al.; European Journal of Neuroscience; 2000, 12, 1123-1127). The CB2 receptors are on the contrary prevailingly located in the marginal zones of the spleen, in tonsils, besides in several cells of the immune system, as macrophages, monocytes, the cells of the bone marrow, of thymus and of pancreas. Other cells of the immune system wherein the CB2 receptors are significantly present are T4 and T8 cells, the polymorphonucleated leucocytes, in particular the cells called "natural killers" and lymphocytes B.

The compounds able to interact, as agonists or antagonists, with the CB2 receptors can therefore be used in the treatment of diseases wherein immune system cells or immune disorders are involved. The activation (modulation) of the CB2 receptors is also important in the treatment of other diseases, such as in the treatment of osteoporosis, renal ischaemia, pain, neuropathic pain, post-surgery pain, inflammatory conditions, lateral amyotrophic sclerosis.

The compounds with affinity for the CB1 receptors can be used in the treatment of eye diseases such as glaucoma, pulmonary diseases, as asthma and chronic bronchitis, inflammations as arthritis, allergies and allergic reactions, such as allergic rhinitis, contact dermatitis, allergic conjunctivitis. Said compounds can also be used in the treatment of pain, in the conditions of anxiety, behaviour disorders, delirium conditions, psychotic problems in general, furthermore for the treatment of schizophrenia, depression and in the treatment of drug and/or alcohol abuse and dependence, (for example alcoholism and tabagism). The same compounds can also be used to combat vomit, nausea, vertigoes, especially in the case of patients undergoing chemotherapy; in the treatment of neuropathies, hemicrania, stress, psychosomatic origin diseases, epilepsy, Tourette syndrome, Parkinson disease, Huntington disease, Alzheimer disease, senile dementia and in the case of recognition disorders and memory loss. Further applications of the compounds having affinity towards the CB1 receptors are the treatment of pathologies related to appetite disorders (obesity, bulimia), pathologies of the gastrointestinal tract and of the bladder, cardiovascular diseases, urinary and fertility disorders, neuroinflammatory pathologies, such as multiple sclerosis, Guillain-Barré syndrome, viral encefalitis. For example some CB1 agonist active principles are successfully used in the treatment of nausea and vomit associated to chemotherapy and in the appetite whetting in AIDS patients. Compounds having antagonist activity towards the CB1 receptors can be used for example in the treatment of psychosis, anxiety, depression, schizophrenia, obesity, neurological diseases (for example: dementia, Parkinson disease, Alzheimer disease, epilepsy, Tourette syndrome), in the conditions of memory loss, of central nervous system diseases involving the neurotransmission of cannabinoids, in fertility and erectile disorders, in the treatment of gastrointestinal and/or cardiovascular disorders.

The compounds which are effective in activating cannabinoid receptors show immunosuppressive activity and are used in the treatment of eye inflammatory conditions and autoimmune diseases, for instance uveitis and uveoretinitis (H. Xu et al., J. Leukocyte Biology, 82, 2007, 532-541). They are used also for treating retina neurodegeneration (G. Pryce et al., Brain 126 2003 2191-2202).

With reference to the wide pharmacological applications of cannabinoids, in the latest years several studies have been carried out for finding endocannabinoids and for the synthesis of new compounds capable of selectively interacting with the two subclasses of CB1 and CB2 cannabinoidergic receptors. Researches have led on the one hand to the identification of anandamide endocannabinoids (arachidonyl ethanolamide) and 2-arachidonyl glycerol, on the other hand to the preparation of different classes of synthesis compounds, agonist or antagonist towards the CB1 or CB2 receptors.

The class of the compounds having agonist activity towards the CB1 receptors (cannabimimetic activity) comprises both synthesis compounds with a basic structure directly derived from that of Δ⁹-THC, as (-)-11-OH-Δ⁸THC-dimethylheptyl (HU210) and nabilone, and compounds structurally different from Δ⁹-THC, as aminoalkylindols of the WIN 55,212-2 series (M. Pacheco et al.; J. Pharmacol. Exp. Ther.; 1991, 257, 1701-183) or as bicyclic cannabinols (non classic cannabinoids) which refer to the compound CP 55,940 (M. Glass; Progr. Neuro-Psychopharmacol. & Biol. Psychiat.; 2001, 25, 743-765). The compounds having cannabimimetic activity show in vivo the following effects: hypoactivity, hypothermia, analgesia and catalepsy (B.R. Martin et al.; Pharmacol. Biochem. Behav.; 1991, 40, 471-478; P.B. Smith et al.; J. Pharmacol. Exp. Ther.; 1994, 270, 219-227).

Clinical data have shown thah the CB1 antagonist pyrazole compound Rimonabant is effective in reducing both weight and metabolic and/or cardiovascular risk factors in patients with metabolic syndrome and/or dyslipidemia (J. P. Després et al., the New England Journal of Medicine, 2005, 353, 2121-2134, D. Tonstad, Nutrition, Metabolism and Cadiovascular Diseases 2006, 16, 156-162. The effectiveness of Rimonabant in reducing metabolic and/or cardiovascular risk factors has been shown also in patients with type 2 diabetes (A. J.Scheen et Al., Lancet 2006, 368 1660-1672).

Compounds having high affinity for the cannabinoidergic receptors and, especially, high selectivity for the CB1 receptors are described for example in EP 1,230,244. In particolar, said compounds are condensed tricyclic compounds having the following general structure: 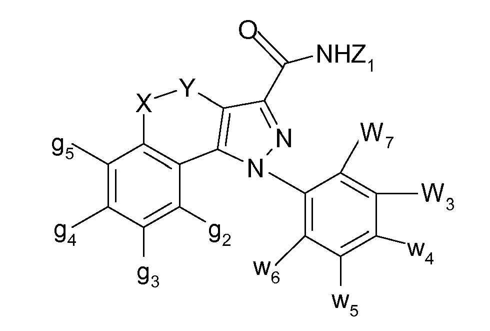 wherein Z₁, W₇, W₃, W₄, W₅, W₆, g₂, g₃ g₄, g₅ have different meanings; X-Y- represent a group selected from: -(CH₂)_{d}-CH₂-, -CH₂-S(O)_{g}-, -S(O)_{g}-CH₂-, with d equal to 1 or 2, g equal to zero, 1 or 2.

Compounds having a high affinity for the cannabinoidergic receptors and, above all, high selectivity for the CB2 receptors, are described in EP 1,230,222. In particular the compounds described in this patent are condensed tricyclic derivatives having general structure: 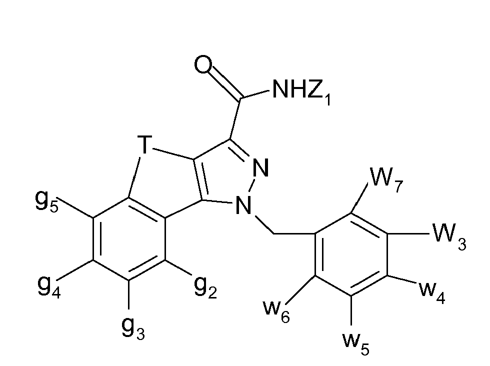 wherein: -T- represents a -(CH₂)ₘ- group, with m equal to 1 or 2; Z₁, W₂, W₃, W₄, W₅, W₆, g₂, g₃, g₄, g₅ have different meanings.

Another class of compounds having affinity towards the CB1 and/or CB2 receptors with a basic pyrazole structure wherein the pyrazole ring is part of a condensed tricyclic stucture, is represented by the derivatives described in US 2005/0282,798. These derivatives exert their activity only on the CB1 and/or CB2 cannabinoidergic receptors at a peripheral level. The compounds are unable to show any activity on the central nervous system, since they do not pass the haematoencephalic barrier.

One class of benzopyranopyrazolyl derivatives is described in USP 5,547,975 and shows the following general formula: 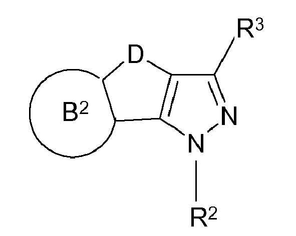 wherein B² and D have different meanings, R² is a group selected between aryl and heteroaryl, optionally substituted with different substituents, R³ is a group selected from hydrogen, halogen, haloalkyl, cyano, nitro, formyl, alkoxycarbonyl, carboxyl, carboxyalkyl, alkoxycarbonylalkyl, amidino, cyanoamidino, aminocarbonyl, alkoxy, alkoxyalkyl, aminocarbonylalkyl, N-alkylaminocarbonyl, N-arylamino-carbonyl, N,N-dialkylaminocarbonyl, N-alkyl-N-arylamino-carbonyl, alkylcarbonyl, alkylcarbonylalkyl, hydroxyalkyl, alkylthio, alkylsulphinyl, alkylsulphonyl, alkylthioalkyl, alkylsulphinylkyl, alkylsulphonylalkyl, N-alkylsulphamyl, N-arylsulphamyl, arylsulphonyl, N,N-dialkylsulphamyl, N-alkyl-N-arylsulphamyl, heterocycle.

These compounds are described for the treatment of the inflammation or disorders related to inflammation. No mention is made that these compounds have affinity for the CB1 and/or CB2 cannabinoid receptors.

A further class of condensed tricyclic compounds containing a pyrazole ring is described in WO 03/070706. The described compounds have the following general formula: 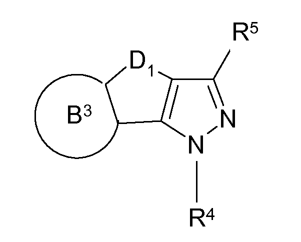 wherein D₁ has various meanings, B³ is heteroaryl, R⁴ is aryl or heteroaryl with a 5 or 6 atom ring, R⁵ is a group selected from amidine, alkylamino, aminoalkyl, NH₂, CONHR¹⁶, NHCOR⁶, CH₂-NH-COR⁶, being:
R¹⁶ a group selected from hydrogen, aryl, arylalkyl, alkyl, haloalkyl, alkenyl, alkynyl, hydroxyalkyl, aminoalkyl, alkoxy, alkoxyalkyl,
R⁶ is a group selected from hydrogen aryl, heteroaryl, alkyl, haloalkyl, alkenyl, alkynyl, hydroxyalkyl, aminoalkyl, alkylammonialkyl, alkoxy, alkoxyalkyl, heterocycloalkyl, heterocycle.

These compounds are described for the use in the treatment of cancer, inflammation and disorders related thereto. No indication is reported as to the affinity of the compounds for the CB1 and/or CB2 cannabinoid receptors.

The need was felt to have available compounds having affinity for the CB1 and/or CB2 cannabinoidergic receptors having the following combination of properties:
- activity in vitro and in vivo both at a peripheral level and on the central nervous system,
- in case of agonist compounds having affinity for the CB1, cannabinoidergic receptors activity at a peripheral level at least in the reduction of the intraocular pressure.

Compounds solving the above described technical problem have been surprisingly and unexpectedly found by the Applicant.

It is an object of the present invention tricyclic compounds having a condensed ring structure containing one phenyl and one pyrazole ring linked to each other by a central ring having from five to eight atoms, showing affinity for the CB1 and/or CB2 receptors, with central and/or peripheral activity, having formula (I): 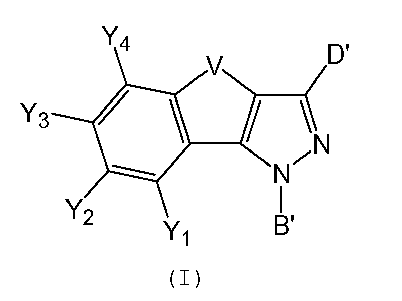 wherein:
B' is a substituent selected from phenyl, arylalkyl, arylalkenyl, heteroaryl, heteroarylalkyl, or a bivalent C₁-C₁₀ aliphatic chain, linear or branched when possible, wherein the end of the main chain not linked to the nitrogen atom is linked to W^{I} selected from hydrogen, halogen, isothiocyanate, CN, OH, OCH₃, NH₂, SO₂NH₂ or -CH=CH₂,
Y₁, Y₂, Y₃ and Y₄, equal to or different from each other, are groups selected from hydrogen, halogen C₁-C₇ alkyl, C₁-C₇ alkylthio, C₁-C₇ alkoxy, C₁-C₇ haloalkyl, C₁-C₇ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₇ alkyl chain,
V is a group selected from:
   A1 is -(CH₂)ₜ-,
   A2 is -(CH₂)ᵣ-O-(CH₂)ₛ-
   A3 is -(CH₂)ᵣ-S(O)ₚ-(CH₂)ₛ-
      wherein
      t is an integer equal to 1, 2 or 3,
      p is an integer equal to 0, 1 or 2,
      r and s, equal to or different from each other, are integers equal to 0, 1 or 2 with the proviso that r+s is equal to 0, 1, 2 or 3,
when B' has the meaning of bivalent C₁-C₁₀ aliphatic chain, linear or branched when possible, wherein the end of the main chain not linked to the nitrogen atom is linked to W^{I} as defined above, D' has the following meanings:
D1: -(CH₂)-O-(CH₂)_{z}-(Z')ᵥ-R"
   wherein z is an integer equal to 1 or 2, v is an integer equal to 0 or 1, Z' is a bivalent C₁-C₈ aliphatic chain, linear or branched when possibile, R" is selected from C₃-C₁₅ cycloalkyl, saturated or unsaturated heterocycle, aryl, or heteroaryl,
D2: -C(O)-(Z')ᵥ-R"
   wherein v, Z' and R" are as defined above,
D3: -CH(OH)-(Z')v-R"
   wherein v, Z' and R" are as defined above,
D4: -C(O)-NH-(Z')ᵥ-T'
   wherein v and Z' are as defined above and T' is a group selected from:
   - C₁-C₈ alkyl, C₁-C₇ haloalkyl with the proviso that in formula D4 v is equal to 0,
   - C₃-C₁₅ cycloalkyl,
   - monocyclic aryl or monocyclic heteroaryl,
   - NR₁R₂, wherein R₁ and R₂, equal to or different from each other, have the following meanings: hydrogen, C₁-C₇ alkyl, C₁-C₇ haloalkyl, heteroaryl, heteroarylalkyl, aryl, arylalkyl or arylalkenyl, or R₁ and R₂ with the nitrogen atom form a saturated or unsaturated heterocycle from 5 to 10 atoms,
   - C₃-C₁₅ heterocycloalkyl, containing one or more heteroatoms, equal to or different from each other selected from N, O S, with the proviso that Z' is linked to one carbon atom of the heterocycloalkyl ring,
when B' is a substituent selected from phenyl, arylalkyl, arylalkenyl, heteroaryl, heteroarylalkyl, D' has the following meanings:
D'2: -C(O)-Z'-R"
   wherein Z' and R" are as defined,
D'3: -CH(OH)-Z'-R"
   wherein Z' and R" are as defined,
D'4: -C(O)-NH-Z'-T'
Z' and T' being as defined, excluding for T' the meanings of C₁-C₈ alkyl, C₁-C₇ haloalkyl and, when in D'4 Z'= -CH₂-, T' is not 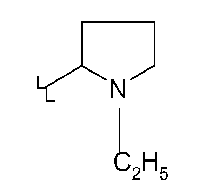
D"2: -C(O)-R", with the proviso that V=A2,
D"3: -CH(OH)-R", with the proviso that V=A2,
D"4: -C(O)-NH- T', with the proviso that V is selected from the following groups: -O- or -CH₂-O-

The compounds of formula (I) comprise the isomeric forms, both geometrical isomers and stereoisomers and mixtures thereof. Besides, the different atoms of the compounds of formula (I) can be in different isotopic forms, so as to allow the radiolabelling of said compounds.

In formula (I) when B' is phenyl, or arylalkyl, arylalkenyl, heteroaryl, heteroarylalkyl, B' can optionally be substituted with one or more groups, equal to or different from each other, selected from halogen, C₁-C₇ alkyl, C₁-C₇ alkylthio, C₁-C₇ alkoxy, C₁-C₇ haloalkyl, C₁-C₇ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₇ alkyl chain.

When Y_{1,} Y₂, Y₃ or Y₄ are phenyl, cycloalkyl, saturated or unsaturated heterocycle, heteroaryl, said phenyl, cycloalkyl, saturated or unsaturated heterocycle and heteroaryl are optionally substituted with one or more groups, equal to or different from each other, selected from halogen, C₁-C₇ alkyl, C₁-C₇ alkylthio, C₁-C₇ alkoxy, C₁-C₇ haloalkyl, C₁-C₇ haloalkoxy, cyano, nitro, SO₂NH isothiocyanate, phenyl, cycloalkyl, saturated or unsaturated heterocycle, heteroaryl, amino optionally mono- or bisubstituted with a C₁-C₇ alkyl chain.

R" in formula (I) can be substituted with one or more groups, equal to or different from each other, selected from SO₂NH₂, halogen, C₁-C₇ alkyl, C₁-C₇ haloalkyl, C₁-C₇ haloalkoxy, C₁-C₇ alkylthio or C₁-C₇ alkoxy.

T' in formula (I) can be substituted with one or more groups, equal to or different from each other, selected from halogen, cyano, nitro, C₁-C₇ alkyl, C₁-C₇ haloalkyl, C₁-C₇ haloalkoxy, C₁-C₇ alkylthio, C₁-C₇ alkoxy, SO₂NH₂, isothiocyanate, phenyl, benzyl, amino optionally mono- or bisubstituted with a C₁-C₇ alkyl chain, the phenyl and benzyl substituents being optionally substituted with one or more groups, equal to or different from each other, selected from halogen, C₁-C₇ alkyl, C₁-C₇ alkylthio, C₁-C₇ alkoxy, C₁-C₇ haloalkyl, C₁-C₇ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₇ alkyl chain.

When R₁ and R₂ of T' in the compounds of formula (I) are aromatic rings selected from heteroaryl, heteroarylalkyl, aryl, arylalkyl or arylalkenyl, or R₁ and R₂ with the nitrogen atom form an heterocycle, the armatic rings or the heterocycle can be substituted with one or more groups equal to or different from each other, selected from halogen, cyano, nitro, C₁-C₇ alkyl, C₁-C₇ haloalkyl, C₁-C₇ haloalkoxy, C₁-C₇ alkylthio, C₁-C₇ alkoxy, SO₂NH₂, isothocyanate, phenyl, benzyl, amino optionally mono- or bisubstituted with a C₁-C₇ alkyl chain, said phenyl and benzyl substituents being optionally substituted with one or more groups, equal to or different from each other, selected from halogen, C₁-C₇ alkyl, C₁-C₇ alkylthio, C₁-C₇ alkoxy, C₁-C₇ haloalkyl, C₁-C₇ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₇ alkyl chain.

Where not otherwise specified, the following definitions apply in the present invention:
by alkyl or alkyl chain it is meant a saturated C₁-C₂₀ hydrocarbon chain, linear or branched when possible,
by alkenyl or alkenyl chain it is meant a mono- or polyunsaturated C₂-C₂₀ hydrocarbon chain, preferably mono-unsaturated, linear or branched when possible,
by cycloalkyl, which ring or rings do not contain unsaturations, it is meant an aliphatic monocyclic ring, from 3 to 10 carbon atoms, preferably from 4 to 9 carbon atoms, or a polycyclic structure from 7 to 19 carbon atoms,
by heterocycloalkyl and saturated heterocycle it is meant a cycloalkyl as defined above wherein one or more carbon atoms are substituted by heteroatoms, equal to or different from each other, selected from S, O N; when the ring is monocyclic, preferably the heteroatoms are no more than 2,
by unsaturated heterocycle it is meant a cyclalkyl as defined above with one or more double bonds, with the proviso that the structure is not aromatic, and wherein at least one carbon atom is substituted by one heteroatom selected from S, O, N,
by halogen it is meant one atom selected from fluorine, chlorine, bromine, iodine,
by haloalkyl or haloalkyl chain it is meant an alkyl as defined above, wherein one or more hydrogen atoms are substituted with halogen atoms. Examples of haloalkyl are trifluoromethyl, 1-bromo-n-butyl, pentachlorethyl, etc.
by aryl it is meant an aromatic monocyclic radical, or a condensed aromatic polycyclic radical having from 6 to 20 carbon atoms,
by heteroaryl it is meant an aryl as above defined, wherein the monocyclic radical is C₅-C₆ and at least one or more carbon atoms are substituted with one or more heteroatoms, equal to or different from each other, selected from S, O N, when the radical is monocyclic preferably the heteroatoms are no more than 2,
by arylalkyl it is meant an alkyl as defined above, preferably C₁-C₇, linked to an aryl as defined above, for example a benzyl,
by arylalkenyl it is meant an alkenyl as defined above linked to an aryl as defined above,
by heteroarylalkyl it is meant an alkyl as defined abve, preferably C₁-C₇ linked to an heteroaryl as defined above,
by bivalent aliphatic chain it is meant a C₁-C₂₀ aliphatic chain, preferably C₁-C₈, saturated or unsaturated, linear or branched when possible, having at each end a free valence, wherein one or more hydrogen atoms can optionally be substituted with halogen atoms,
by compound having affinity towards the receptors it is meant a compound having in vitro and/or in vivo and/or in ex-vivo agonist, or antagonist, or partial agonist, or partial antagonist, or inverse agonist, or inverse antagonist, or inverse partial agonist activity towards the receptors. The meaning of said terms is well known to the skilled in the field.

The preferred compounds of formula (I) are those wherein:
B' is a substituent selected from phenyl, benzyl, monocyclic heteroaryl, monocyclic heteroarylalkyl, or a bivalent C₁-C₁₀ aliphatic chain, linear or branched when possibile, wherein the end of the main chain not linked to the nitrogen atom is linked to W^{I} selected from hydrogen, halogen, isothiocyanate, CN, OH, OCH₃, NH₂, SO₂NH₂ or -CH=CH₂, said phenyl, benzyl, monocyclic heteroaryl and monocyclic heteroarylalkyl being optionally substituted with one or more groups, equal to or different from each other, selected from halogen, C₁-C₇ alkyl, C₁-C₇ alkylthio, C₁-C₇ alkoxy, C₁-C₇ haloalkyl, C₁-C₇ haloalkoxy, cyano, nitro, SO₂NH₂ isothiocyanate, phenyl, cycloalkyl, saturated or unsaturated heterocycle, heteroaryl, amino optionally mono- or bisubstituted with a C₁-C₇ alkyl chain,
Y₁, Y₂, Y₃ and Y₄, equal to or different from each other, are as defined,
V is a group selected between A1 and A2,
when B' has the meaning of bivalent C₁-C₁₀ aliphatic chain, linear or branched when possible, wherein the end of the main chain not linked to the nitrogen atom is linked to W^{I} as defined above, D' is as defined,
when B' is a substituent selected from phenyl, benzyl, monocyclic heteroaryl or monocyclic heteroarylalkyl, D' has the meanings of D'2, D'4, D"2 or D"4.

The most preferred compounds of formula (I) are those wherein:
B' is a substituent selected from phenyl, benzyl, thiophene or a bivalent C₄-C₁₀ aliphatic chain, linear or branched when possible, wherein the end of the main chain not linked to the nitrogen atom is linked to W¹ selected from hydrogen, halogen, isothiocyanate, CN, OH, OCH₃, NH₂, SO₂NH₂ or -CH=CH₂, said phenyl, benzyl and thiophene being optionally substituted with one or more groups, equal to or different from each other, selected from halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₃ alkyl chain,
Y₁, Y₂, Y₃ and Y₄, equal to or different from each other, are as defined above,
V is a group selected between A1 and A2,
when B' is a bivalent C₄-C₁₀ aliphatic chain, linear or branched when possible, wherein the end of the main chain not linked to the nitrogen atom is linked to W^{I} as defined above, D' is as defined,
when B' is a substituent selected from phenyl, benzyl or thiophene, D' has the meanings of D'2, D'4, D"2 or D"4.

The still more preferred compounds of formula (I) are those wherein:
B' is a substituent selected from phenyl, benzyl, thiophene, a bivalent C₄-C₁₀ aliphatic chain, linear or branched when possible, wherein the end of the main chain not linked to the nitrogen atom is linked to W^{I} selected from hydrogen, halogen, OH, OCH₃, NH₂, SO₂NH₂, said phenyl, benzyl and thiophene being optionally substituted with one or more groups, equal to or different from each other, selected from halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂ isothiocyanate, phenyl, cycloalkyl, saturated or unsaturated heterocycle, heteroaryl, amino optionally mono- or bisubstituted with a C₁-C₃ alkyl chain,
Y₁, Y₂, Y₃ and Y₄, equal to or different from each other, are groups selected from hydrogen, halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂ isothiocyanate, phenyl, cycloalkyl, saturated or unsaturated heterocycle, heteroaryl, amino optionally mono- or bisubstituted with a C₁-C₃ alkyl chain, said phenyl, cycloalkyl, saturated or unsaturated heterocycle and heteroaryl being optionally substituted with one or more groups, equal to or different from each other, selected from halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₇ alkyl chain,
V represents a group selected from A1 or A2,
when B' is a bivalent C₄-C₁₀ aliphatic chain, linear or branched when possible, wherein the end of the main chain not linked to the nitrogen atom is linked to W^{I} as defined above, D' is as defined above,
when B' is a substituent selected from phenyl, benzyl or thiophene, D' has the meanings of D'2, D'4, D"2 or D"4, but with the proviso that:
Z' is selected from -CH₂- or -CH(CH₃)-,
R" is selected from C₃-C₁₅ cycloalkyl, saturated or unsaturated heterocycle, aryl, or heteroaryl, said C₃-C₁₅ cycloalkyl, saturated or unsaturated heterocycle, aryl, or heteroaryl being optionally substituted with one or more groups, equal to or different from each other, selected from SO₂NH₂, halogen, C₁-C₃ alkyl, C₁-C₃ haloalkyl, C₁-C₃ haloalkoxy, C₁-C₃ alkylthio, C₁-C₃ alkoxy,
T' is a group selected from:
   - C₃-C₁₅ cycloalkyl,
   - monocyclic aryl when one of the following alternative conditions is satisfied:
      V different from A1, or
      B' different from phenyl, benzyl or thiophene independently from V,
   - NR₁R₂ group, wherein R₁ and R₂, equal to or different from each other, with the nitrogen atom form a saturated or unsaturated heterocycle having from 5 to 10 atoms,
   - C₃-C₁₅ heterocycloalkyl, wherein Z' is linked to one carbon atom of the heterocycloalkyl.

T' can be substituted with one or more groups, equal to or different from each other, selected from halogen, C₁-C₃ alkyl, C₁-C₃ haloalkyl, C₁-C₃ haloalkoxy, C₁-C₃ alkylthio, C₁-C₃ alkoxy, SO₂NH₂, phenyl, benzyl, amino optionally mono- or bisubstituted with a C₁-C₃ alkyl chain, said phenyl and benzyl substituents being optionally substituted with one or more groups, equal to or different from each other, selected from halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₃ alkyl chain.

Examples of compounds of the invention of formula (I) are the compounds having formula (X') to (XXVII') reported hereinafter: 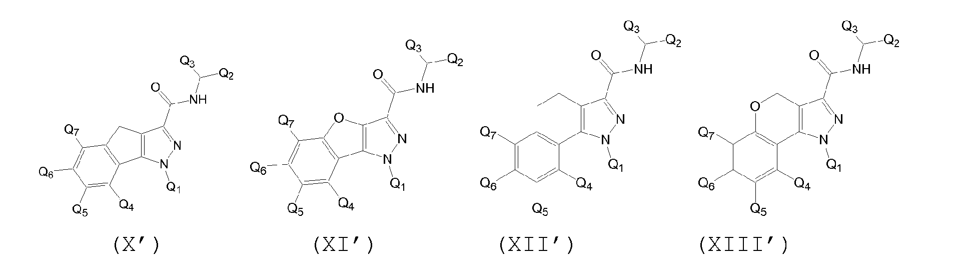 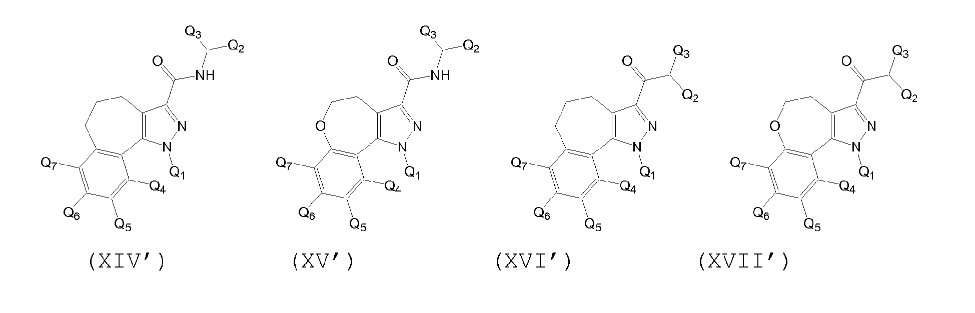 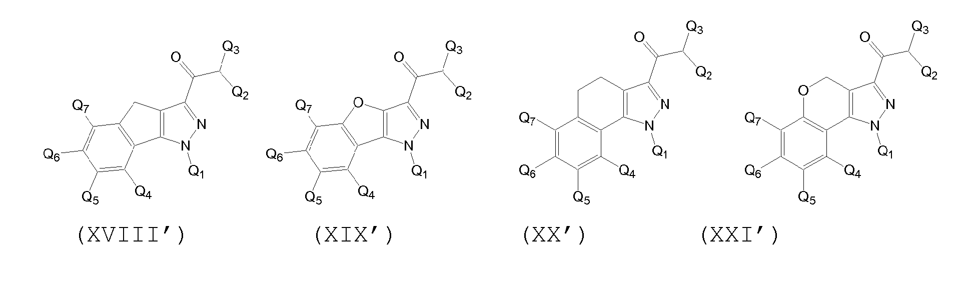 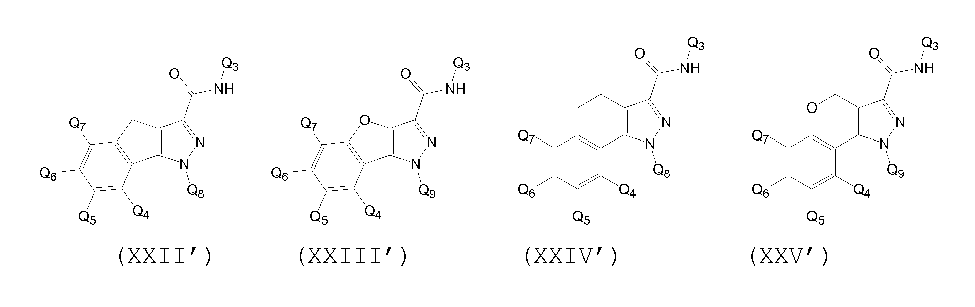 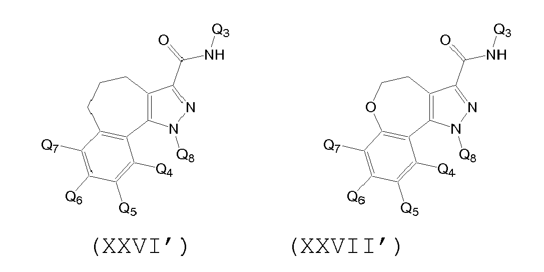 wherein:
Q₁ has the meaning of:
   Q₁A: bivalent C₄-C₁₀ aliphatic chain, linear or branched when possible, wherein the end of the main chain not linked to the nitrogen atom is linked to W^{IV} selected from hydrogen, halogen, OH, OCH₃, NH₂ or SO₂NH₂,
   Q₁B, selected from phenyl or benzyl, optionally substituted with one or more groups, equal to or different from each other, selected from halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂, isothiocyanate, phenyl, cycloalkyl, saturated or unsaturated heterocycle, heteroaryl, or amino optionally mono- or bisubstituted with a C₁-C₃ alkyl chain,
Q₈ has the meaning of Q₁A as defined above,
Q₉ has the meaning of Q₁ as defined above,
Q₂ is selected from hydrogen or methyl,
Q₄, Q₅, Q₆, Q₇, equal to or different from each other, are groups selected from hydrogen, halogen, C₁-C₃ alkyl, C₁-C₃ alkoxy, C₁-C₃ haloalkyl, cyano, SO₂NH₂, isothiocyanate, phenyl, cycloalkyl, saturated or unsaturated heterocycle, thiophene, amino optionally mono- or bisubstituted with a C₁-C₃ alkyl chain, said phenyl, cyclalkyl, saturated or unsaturated heterocycle and thiophene being optionally substituted with one or more groups, equal to or different from each other, selected from halogen, C₁-C₃ alkyl, C₁-C₃ alkoxy, C₁-C₃ haloalkyl, cyano, SO₂NH₂, isothiocyanate, amino optionally mono- or bisubstituted with a C₁-C₃ alkyl chain,
Q₃ is a group selected from the following:

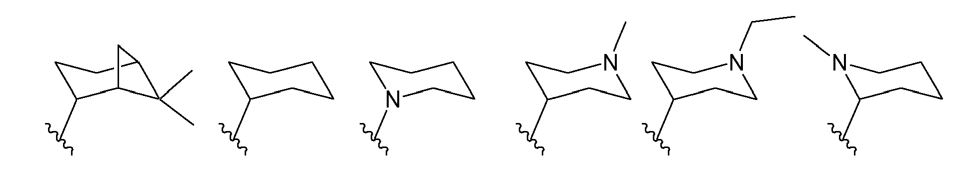

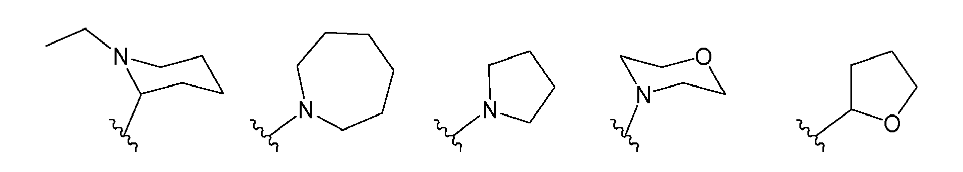

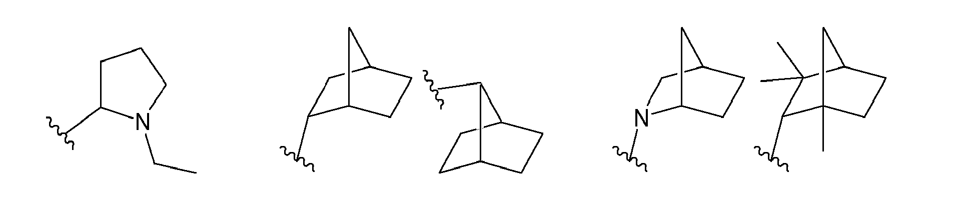

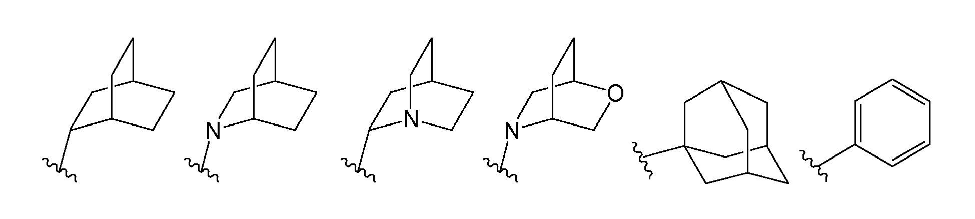

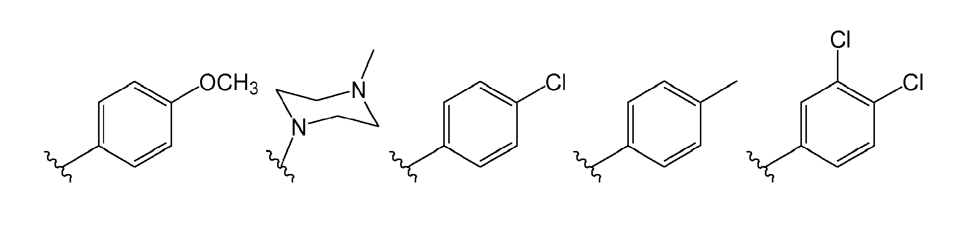

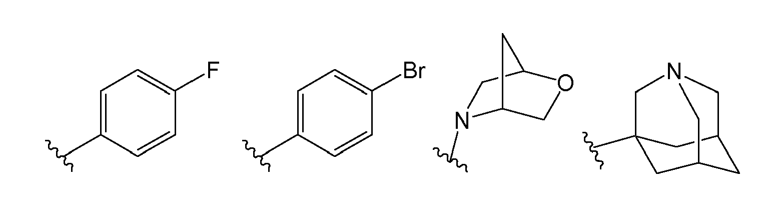

Examples of the specific compounds of the invention of formula (I) are the compounds having formula from (X") to (XCIII") reported hereinafter:

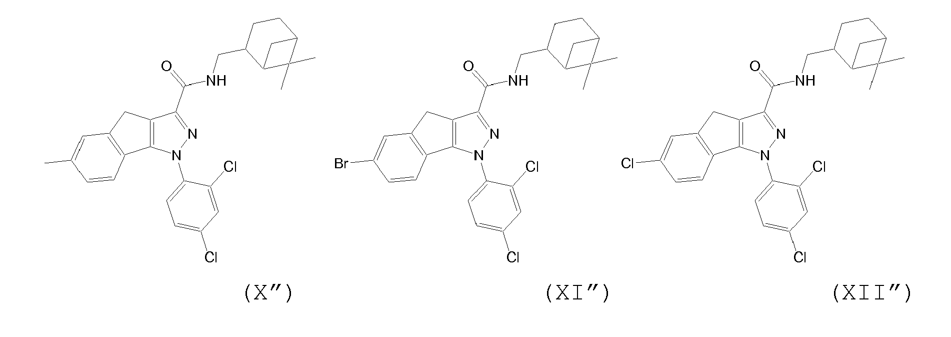

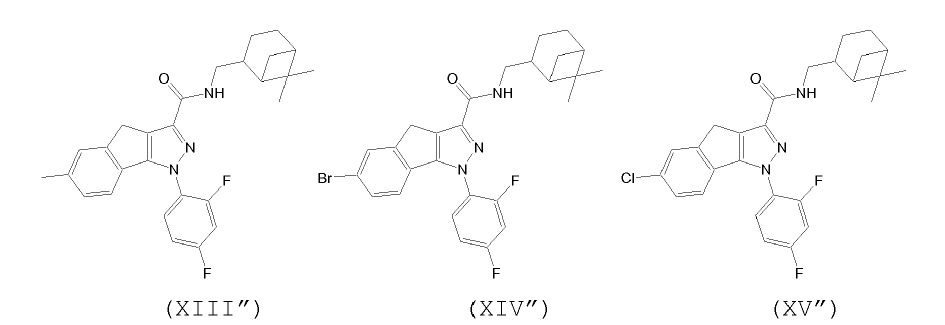

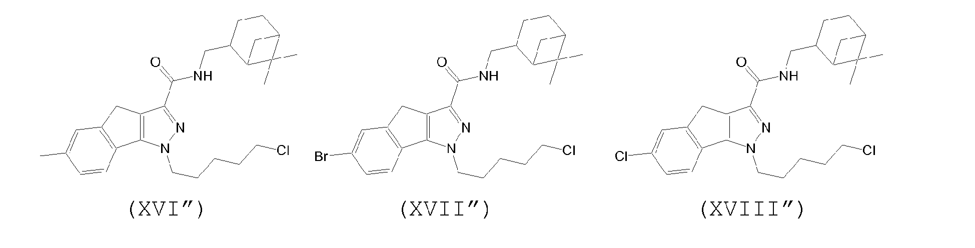

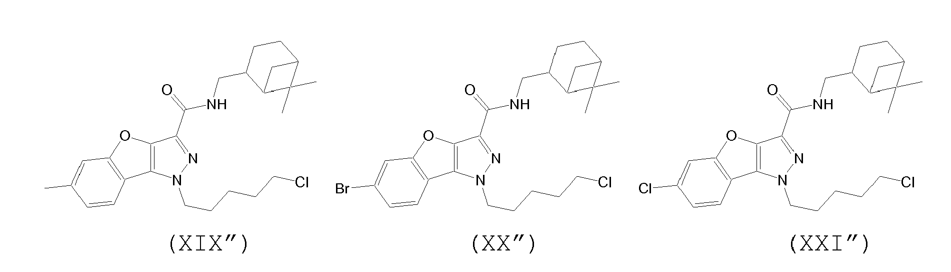

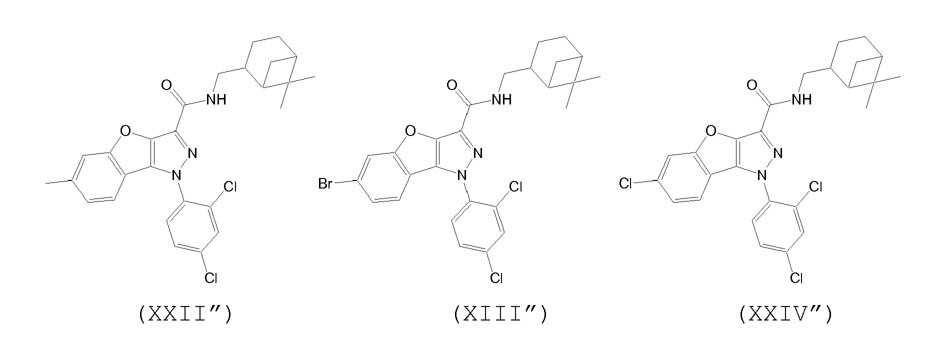

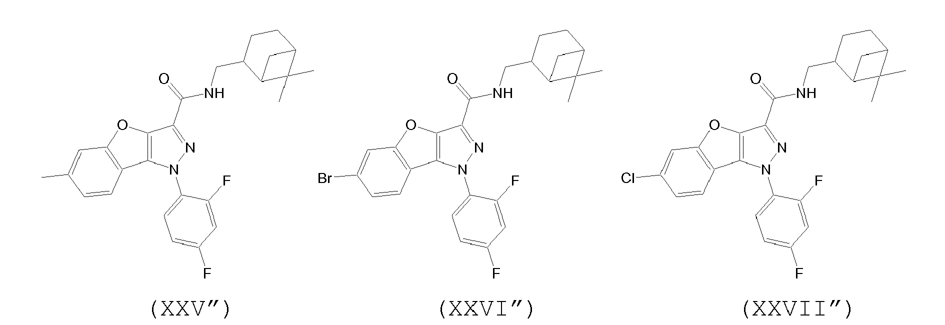

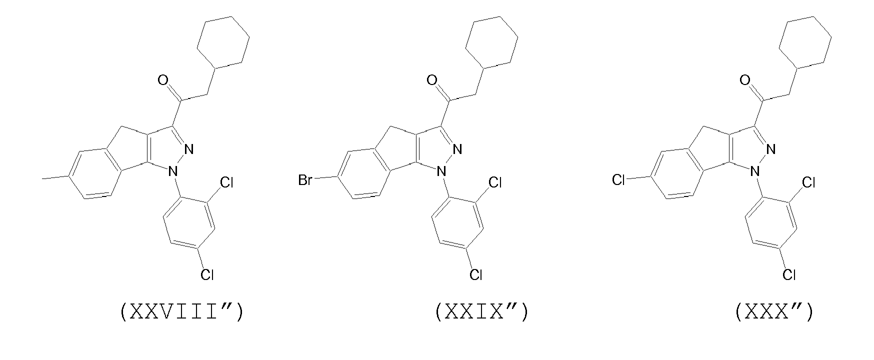

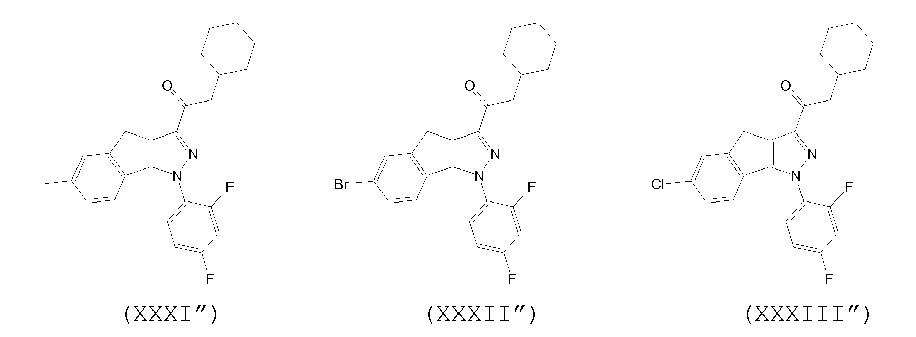

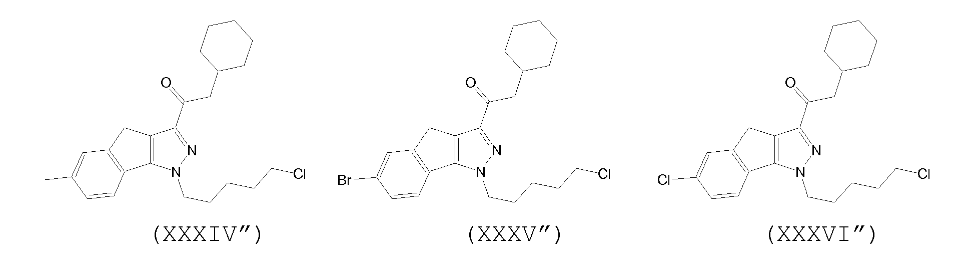

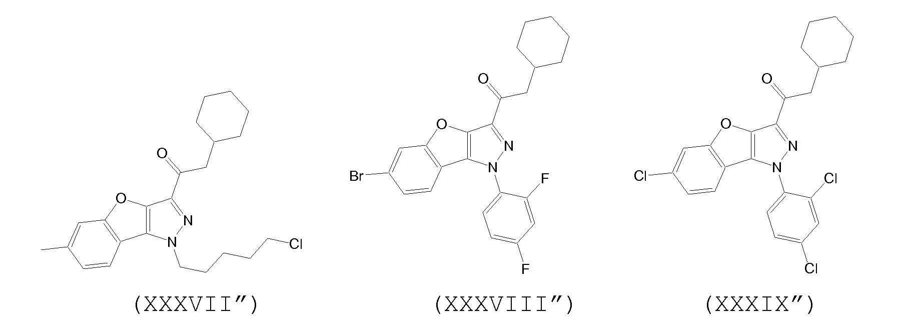

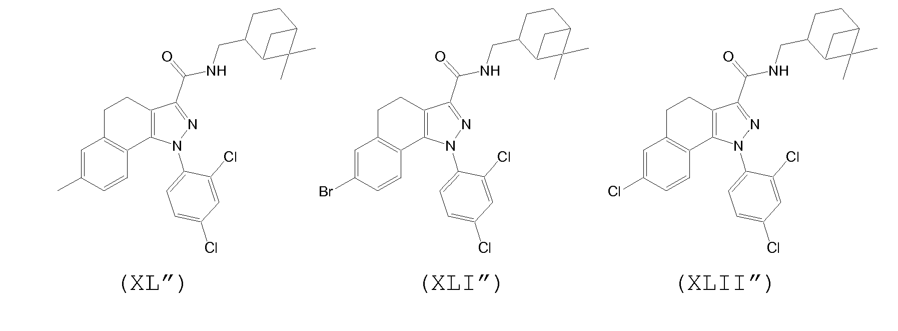

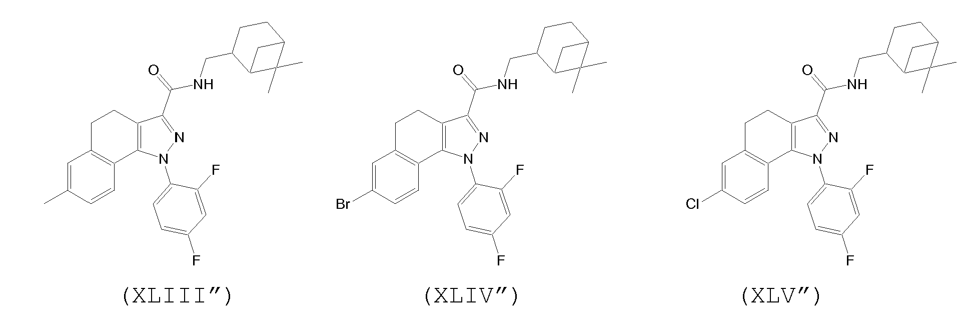

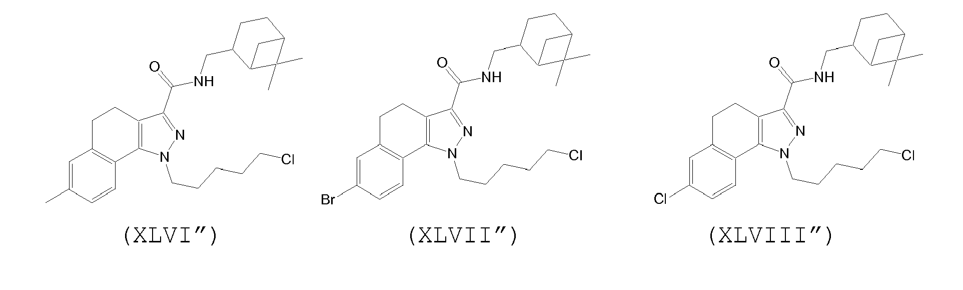

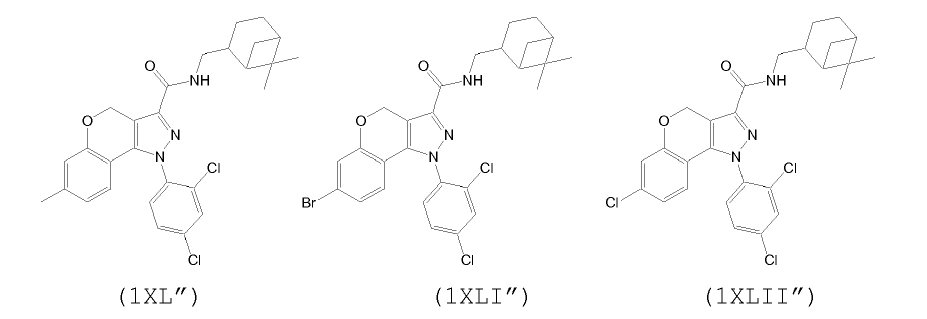

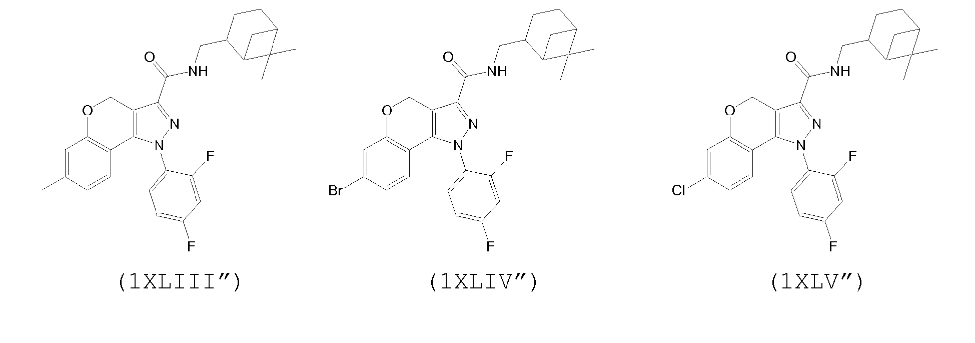

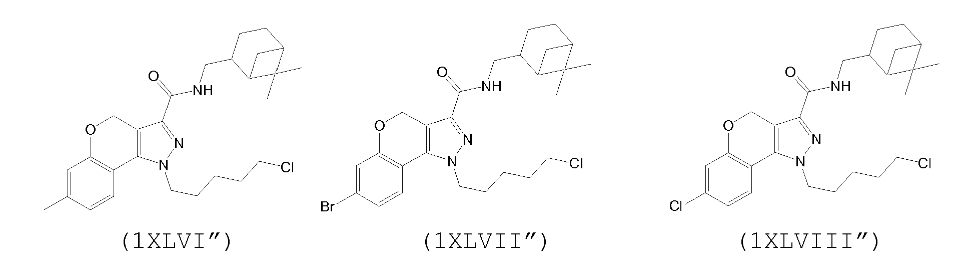

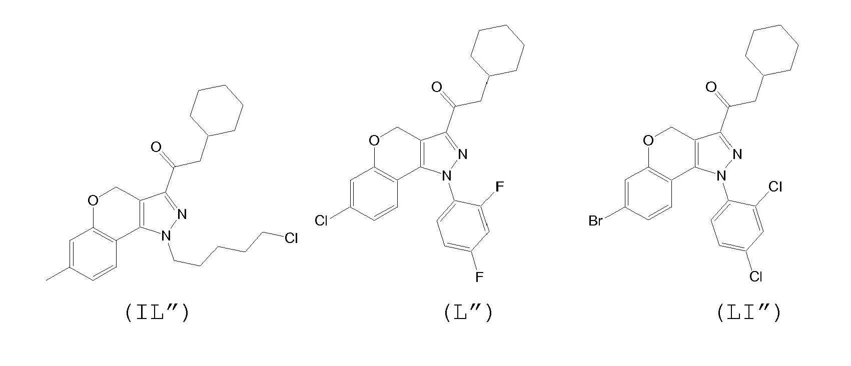

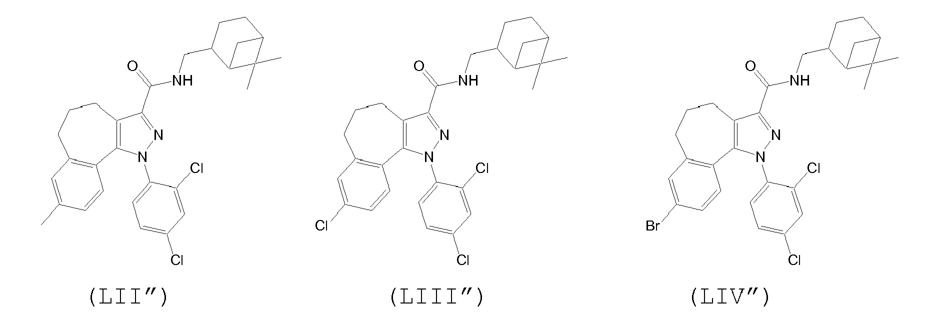

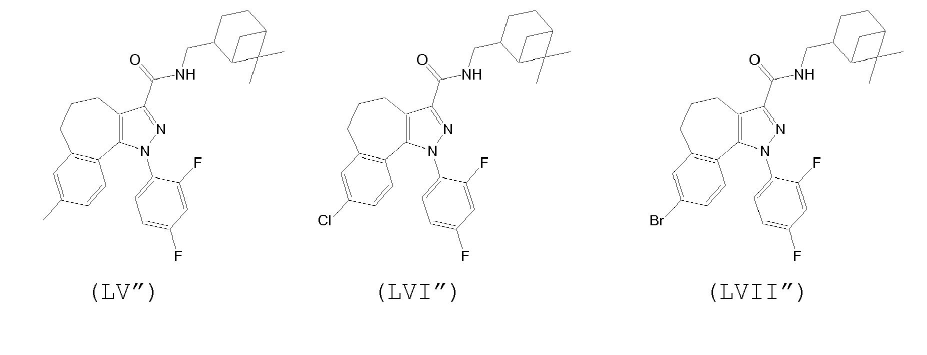

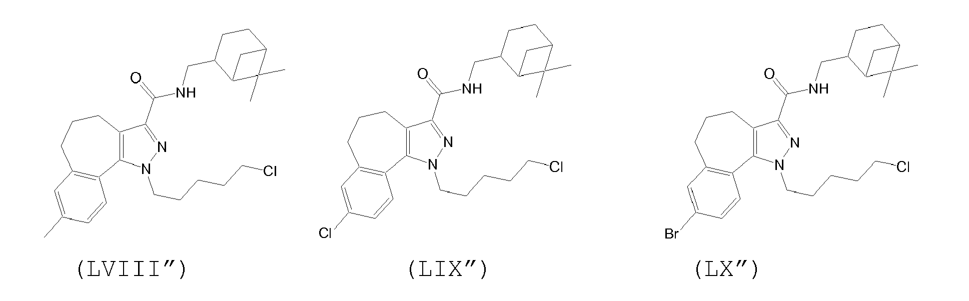

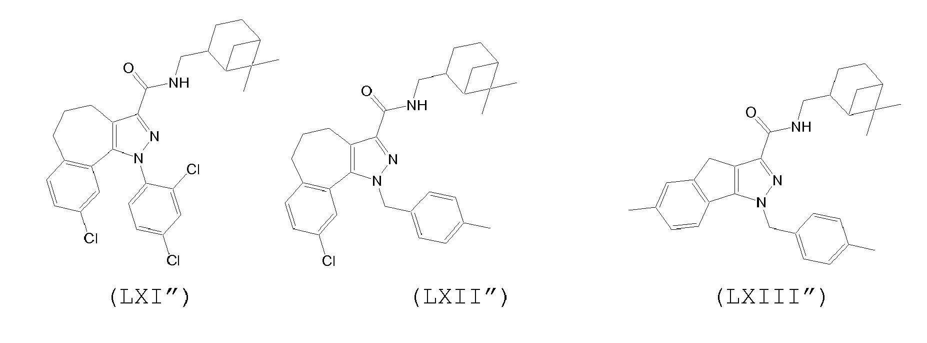

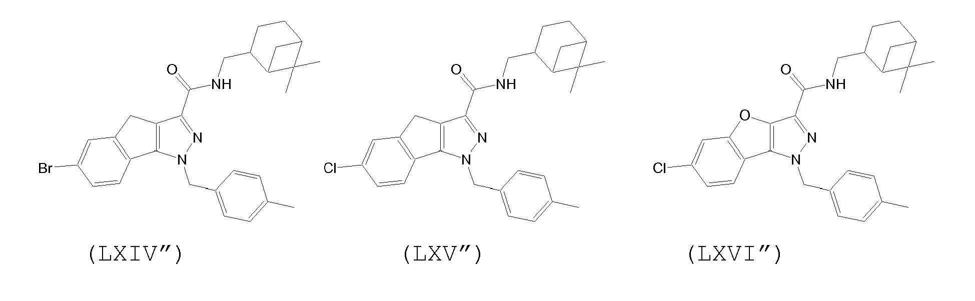

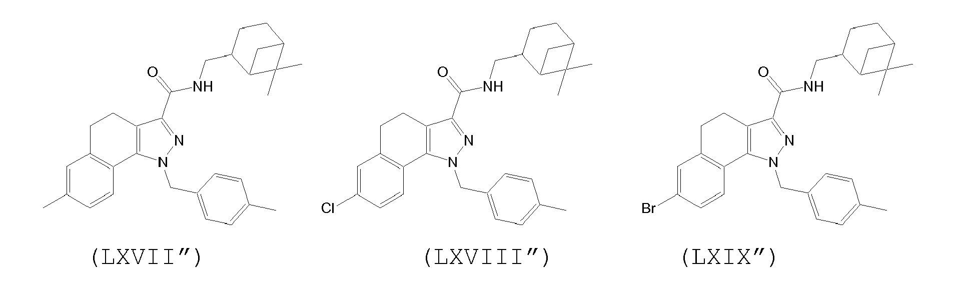

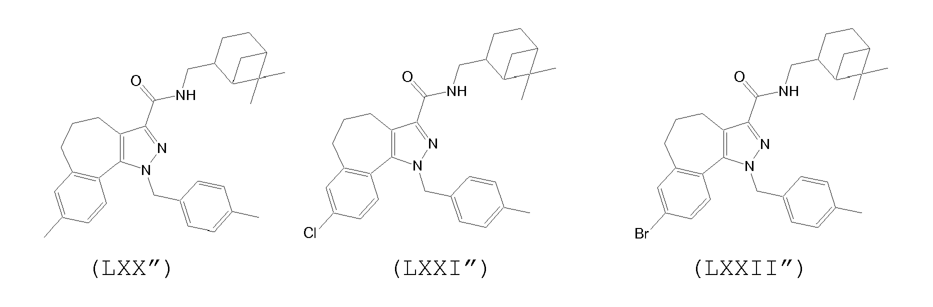

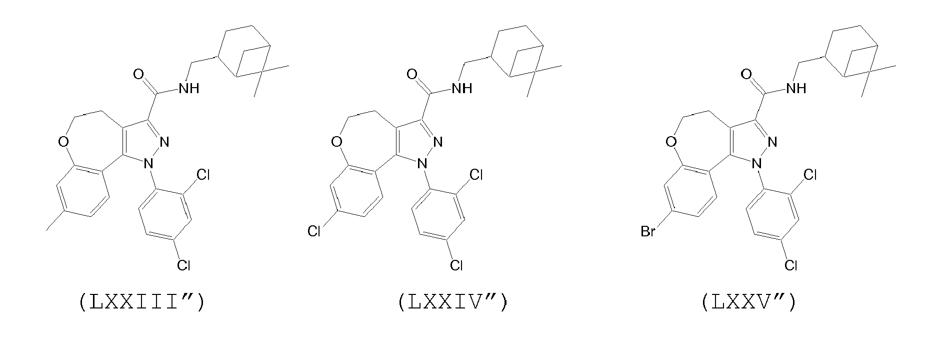

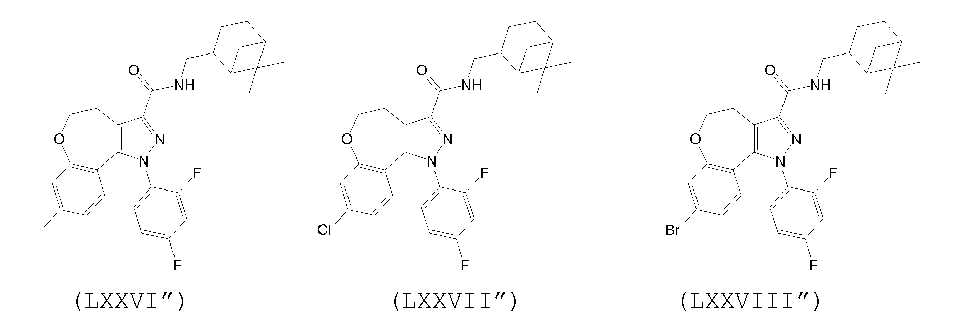

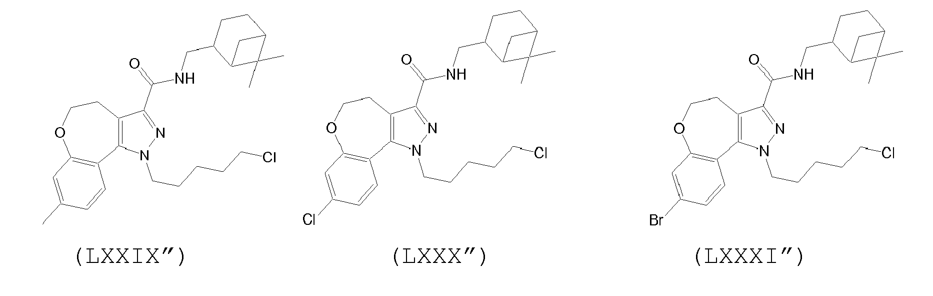

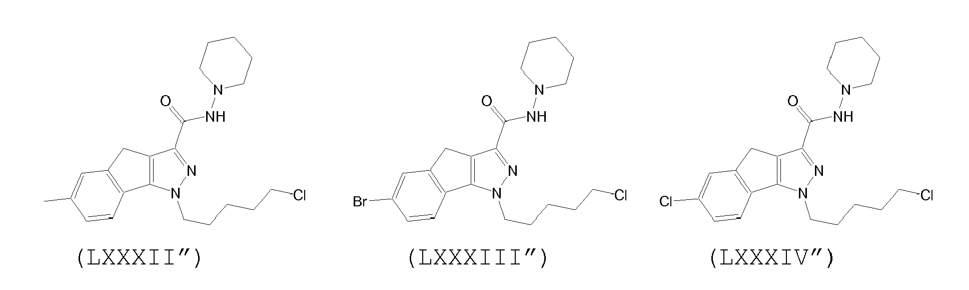

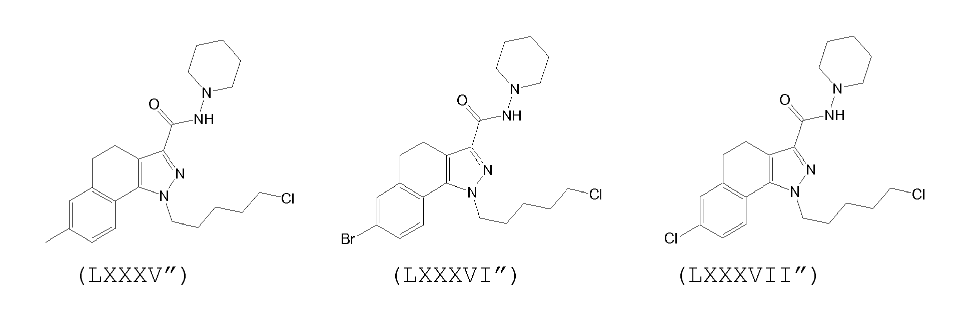

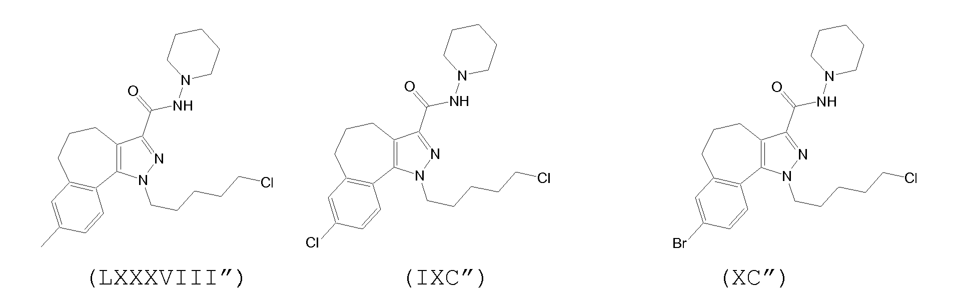

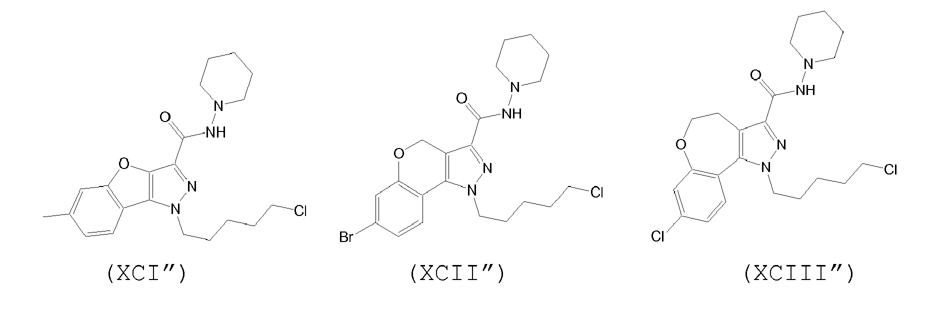

Particularly preferred compounds of the present invention are the compounds having formula (XVI"), (XVII") (XVIII"), (XIX"), (XX"), (XXI"), (XXXIV"), (XXXV") (XXXVI"), (XXXVII"), (XLVI"), (XLVII"), (XLVI"), (XLVII"), (XLVIII"), (1XLVI"), (1XLVII"), (1XLVIII"), (IL"), (LVIII"), (LVIX"), (LX"), (LXXIX"), (LXXX"), (LXXXI"), (LXXXII"), (LXXXIII"),(LXXXIV"), (LXXXV"), (LXXXVI"), (LXXXVII"), (LXXXVIII"), (IXC"), (XC"), (XCI"), (XCII"), (XCII").

As said, hydrates, solvates and pharmaceutically acceptable salts of compounds of formula (I), comprising the various optical and geometrical isomers and the mixtures thereof of the compounds of formula (I), are a further object of the present invention. The meaning of the hydrate and solvate terms is well known to the skilled in the field. In particular by hydrate it is meant a compound containing one or more molecules of hydration water, generally from 1 to 10 molecules of water. By solvate it is meant that the compound contains one or more molecules of solvent different from water.

By pharmaceutically acceptable salts the salts are meant obtained by treating the compounds of formula (I) with organic or inorganic acids acceptable from a pharmaceutical point of view. For example hydrochlorides, sulphates, fumarates, oxalates, citrates, hydrogensulphates, succinates, paratoluensulphonates can be mentioned. See the volume: "Remington, The Science and Practice of Pharmacy", vol. II, 1995, page 1457.

The metabolites derived from the administration in human beings and in animals of the compounds of formula (I) are a further object of the present invention.

Surprisingly and unexpectedly it has been found by the Applicant that the compounds of formula (I) of the invention have in vitro and/or in vivo one or more of the following activities towards the CB1 and/or CB2 cannabinoid receptors: agonist, or antagonist, or partial agonist, or partial antagonist, or inverse agonist, or inverse antagonist, or inverse partial agonist, or inverse partial antagonist.

A further object of the present invention is a process for preparing the compounds of general formula (I) carried out as follows:
i) synthesis of the acid of the following general formula (II), or optionally of a reactive derivative thereof, selected from acyl halides, anhydrides, mixed anhydrides, imidazolides, ester-amide adducts, linear or branched C₁-C₄ alkyl esters: 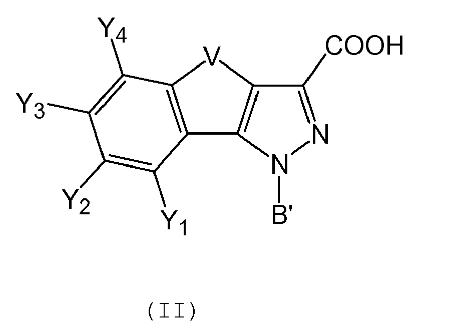 said synthesis comprising the following steps:
   - preparation of α-hydroxy-γ-ketoesters of formula (IV), wherein V, Y₁, Y₂, Y₃ and Y₄ are as previously defined, by reacting a compound of formula (III) with sodium alkoxide (RONa) and diethyloxalate in a C₁-C₃ alcoholic solvent at reflux (Claisen condensation): 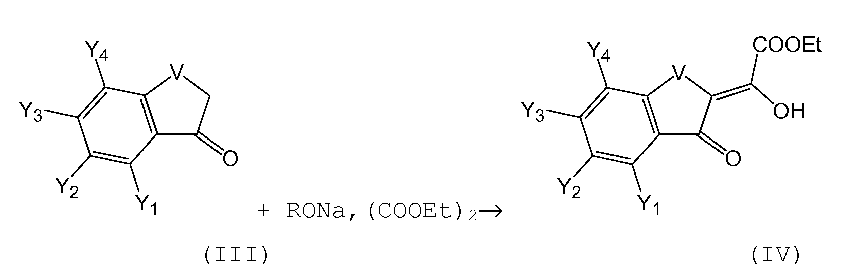
   - reaction of the compounds of formula (IV) with an hydrazine of formula (VI) wherein B' is as previously defined, said compound (VI) being optionally in the form of an hydrochloride salt in an alcoholic solvent or in acetic acid, at reflux or in acetic acid, to yield the tricyclic compound of formula (VII): 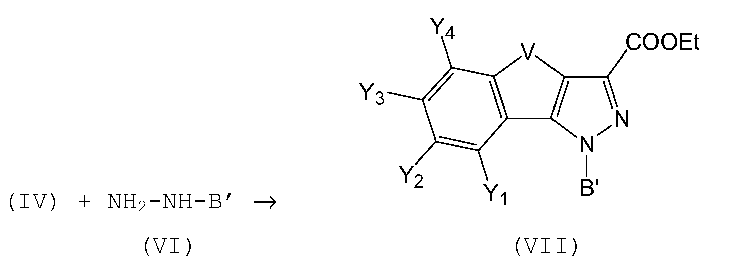
   - base hydrolysis with alkaline hydroxides in hydroalcoholic solution of the compound of formula (VII) at reflux to yield the acid of general formula (II);
   - optionally, preparation of a reactive derivative of the acid of general formula (II), said reactive derivative being as defined above,
ii) when in general formula (I) D' is a substituent having an ethereal group of formula D1, the compounds of formula (I) can be prepared starting from an acid of formula (II) or from an ester thereof, preferably an ethyl ester and reducing it in a first step, by operating at room temperature, to primary alcohol in an inert solvent (for example tetrahydrofuran) under the reaction conditions, for example by using an organic metal hydride, such as di-isobutyl aluminum hydride (DIBAL-H), or lithium and aluminum hydride LiAlH₄; then in a second step the primary alcohol is reacted at room temperature with an alkyl halide of formula R"-(Z')ᵥ -(CH2)z-Hal, wherein Hal is halogen and Z', v and z are as defined above, in the presence of an alkaline hydride, such as sodium hydride, obtaining the above mentioned compounds of formula (I) wherein D'=D1,
iii) when in general formula (I) D'=D2, the compounds of formula (I) can be prepared according to one of the following processes:
   first process, comprising:
      - reaction of an ester of the acid of general formula (II), with trialkylaluminum and with the hydrochloride salt of an amine in an inert solvent under the reaction conditions until total ester consumption and subsequent addition to the reaction mixture of the Grignard compound R"-(Z')ᵥ-MgBr, wherein Z', v and R" are as defined above, and reaction at room temperature until obtaining the compound of formula (I) with D'= D2,
   second process, comprising:
      - reaction of the acid of formula (II), or a reactive derivative thereof, with a metallorganic salt of formula (R"-(Z')ᵥ)⁻ Me⁺, wherein Me⁺ is an alkaline metal cation, in an inert solvent under the reaction conditions, obtaining the compound of formula (I) with D'=D2,
iiii) when in general formula (I) D'=D3 the synthesis is carried out in two steps:
   - preparation of the compound of formula (I) wherein D'=D2, by using one of the two alternative processes described above in iii),
   - reduction of the compound obtained in the previous step at room temperature, and isolation of the final product of formula (I) with D'=D3,
iiiii) when in general formula (I) D'=D4, the compounds of the invention are prepared by reaction of a reactive derivative of the acid of formula (II) with a compound of general formula:

   H₂N-(Z')ᵥ-T' (VIIA)

   wherein Z', v and T' are as previously defined. The reaction is carried out in an inert solvent under the reaction conditions and at room temperature.

In i) when B' has the meaning of bivalent C₁-C₁₀ aliphatic chain, linear or branched when possible, wherein the end of the main chain not linked to the nitrogen atom is linked to W^{I}, W^{I} being as defined above, the preparation of the compound (VII) can be performed by reacting compound (VI) with hydrated hydrazine in an alcoholic solvent, preferably ethanol, at reflux obtaining the intermediate (VI'): 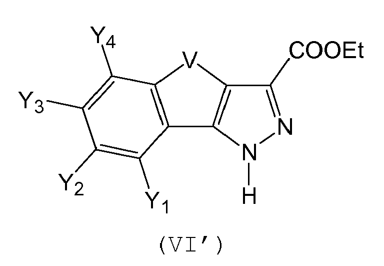 and subsequent alkylation of compound (VI') with W^{I}-B'-Z" in an inert solvent at reflux, preferably in the presence of a base, obtaining compound (VII), W¹ and B' being as defined above and Z" a leaving group, for example bromine, tosyl, mesyl.

Preferably in iii), in the first reaction of the first of the two above mentioned synthesis processes for obtaining the compounds of general formula (I) wherein D'=D2, the ethyl ester of the acid of general formula (II), Al(CH₃)₃, HN(OCH₃)CH_{3·}HCl are used, the reaction solvent being dichloromethane. Both the reactions of said synthesis process at the beginning are carried out at a temperature of 0°C and then at room temperature (20-25°C).

In iii), in the second of the two synthesis processes for obtaining the compounds of general formula (I) wherein D'=D2, preferably Me⁺ is lithium cation.

In iii) the first of the two synthesis processes is the preferred one.

Preferably the reduction reaction in iiii) is carried out with lithium and aluminum hydride or with sodium borohydride.

The compounds of formula (III) and (VIIA) are commercially available, or their preparation is described in the publications of the related art.

When in the compounds of formula (I) D' is respectively D'2, or D'3, or D'4, the process for obtaining the compounds of formula (I) as described above can be used, with the proviso that v=1.

A further object of the present invention are acids of formula (II'): 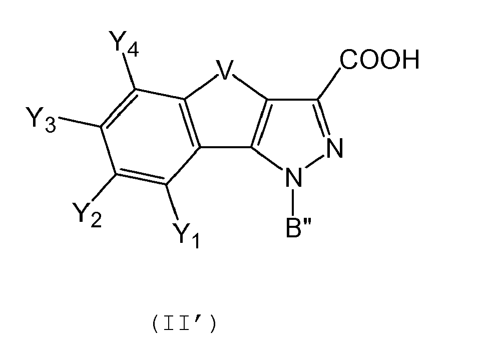 wherein:
V, Y₁, Y₂, Y₃ and Y₄ are as defined above,
B" is hydrogen or a bivalent C₁-C₁₀ aliphatic chain, linear or branched when possible, wherein the main chain end not linked to the nitrogen atom is linked to W^{II}, W^{II} being a group selected from hydrogen, halogen, isothiocyanate, CN, OH, OCH₃, NH₂, SO₂NH₂ or -CH=CH₂.

The preferred acids of formula (II') are those wherein:
V is a group selected from A1 or A2,
B" is as defined above,
Y₁, Y₂, Y₃ and Y₄, equal to or different from each other, are selected from hydrogen, halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₃ alkyl chain, said phenyl, cycloalkyl, saturated or unsaturated heterocycle and heteroaryl being optionally substituted with one or more groups, equal to or different from each other, selected from halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂, phenyl, cycloalkyl, saturated or unsaturated heterocycle, heteroaryl, isothiocyanate, or amino optionally mono- or bisubstituted with a C₁-C₇ alkyl chain.

The present invention relates furthermore to the compounds of formula (I) for preparing pharmaceutical compositions for the therapy and prophylaxis in mammals and in human beings of diseases and disorders wherein the CB1 and/or CB2 cannabinoidergic receptors are involved.

In the pharmaceutical compositions the compounds (I) are contained in the amount required for the specific pharmaceutical application.

In the pharmaceutical compositions the compounds of formula (I) can be present as such or in the form of a salt or solvate, or also as a geometrical isomer, such as cis or trans isomer, or optical isomer when it contains one or more chiral centres.

The additives of the pharmaceutical compositions are excipients, carriers, dyestuffs, preservatives, aromas, etc., the use of which in the pharmaceutical field is known. The used amounts of these additives and excipients are those known for the specific applications.

The administration of the pharmaceutical compositions can be made by oral, subcutaneous, sublingual, intramuscular, intravenous, topic, transdermal, rectal, ophthalmic, intranasal, vaginal, intraperitoneal route.

The pharmaceutical compositions of the present invention comprise for example dispersions, solutions, emulsions, powders, capsules, aerosol, suppositories, tablets, syrups, elixirs, creams, gels, ointments, plasters, etc.. See for example those described in patent application WO 2004/011,468. The pharmaceutical compositions can be obtained according to the known processes of the pharmaceutical art. For example, said pharmaceutical compositions can be obtained according to the procedures described in USP 6,028,084, herein incorporated by reference.

The pharmaceutical compositions can also be prepared by using the methods and the additives reported in patent application US 2003/0003145. In these formulations sodium alkylsulphate, or other surfactants commonly used in the pharmaceutical field, can be used.

For example pharmaceutical compositions for the oral administration of the compounds of formula (I), their isomers or the corresponding hydrates or solvates or pharmaceutically acceptable salts, contain: 0.5-20% by weight of a compound of formula (I), 0.05-0.5% by weight of sodium alkylsulphate or of another surfactant; 2.5-10% by weight of a disgregating agent, for example cellulose, sodium carboxymethylcellulose or other cellulose derivatives, the difference to 100% weight given by the other conventional excipients of oral formulations.

Pharmaceutical formulations for both the oral and intraocular administration can comprise the compounds of formula (I), their isomers, including their salts, hydrates, solvates and hydroxypropylcellulose. In particular they can comprise from 0.1 to 20% of said compounds of formula (I) and from 0.5 to 10% of hydroxypropylmethylcellulose (HPMC). The difference to 100% by weight being given by the conventional additives used in said formulations. Specific pharmaceutical formulations comprise also other excipients, such as lactose monohydrate, magnesium stearate, microcristalline cellulose, titanium oxide. Said pharmaceutical compositions can be in the form of capsules or tablets, for example. In these preparations HPMC can be present in the capsule or tablet core, and/or in the tablet shell.

Further formulations of the compounds of formula (I) comprise oil in water emulsions, wherein the active principle, as such or solubilized in an organic phase, is dispersed in an aqueous phase by using one or more amphiphilic compounds. The latter are for example surfactants, polymers soluble in oil or in water capable of forming organized structures, such as aggregates, micelles and vesicles in the liquid in which they are solubilized.

The emulsions generally contain (% by weight):
- rom 0.005 to 20% of the compounds of formula (I), their isomers or the corresponding hydrates or solvates or pharmaceutically acceptable salts,
- from 0 to 50% of one or more oils,
- from 0.01 to 50% of one or more amphiphilic compounds,
- from 0 to 50% of additives,
- from 0.01 to 99.9% of water or a saline aqueous solution, optionally buffered,
the sum of the components of the emulsions being 100% by weight.

The emulsions can be prepared for example by means of turboemulsifiers or high pressure homogenizers.

The emulsions of the present invention can be prepared by a process comprising the following steps:
- (IP"): optionally, solubilization of the compounds of formula (I) in one or more oils, optionally in the presence of additives,
- (IIP"): heating of the compounds of formula (I) or of the oily solution obtained in the optional step (IP") at temperatures in the range 35°C-80°C, more preferably 45-70°C,
- (IIIP"): addition of one or more amphiphilic compounds to water or to a saline aqueous solution, the water and the saline solution optionally containing additives,
- (IVP"): heating of the aqueous phase of step (IIIP") at temperatures in the range 35°C-80°C, more preferably 45-70°C,
- (VP"): addition, under stirring of the liquid phase obtained in step (IIP") to the aqueous phase obtained in step (IVP"), obtaining an emulsion,
- (VIP"): cooling of the emulsion at temperatures comprised between 0°C and 30°C.

Step (VP") preferably is performed by using turboemulsifiers.

The emulsions obtained in steps (VP") and (IVP") can optionally be subjected to a further homogeneization step at high pressure.

The emulsions can also be prepared by dilution of microemulsions containing the compounds of formula (I) with water or with aqueous solutions or with one or more oils. Optionally water, the aqueous solutions, the one or more oils used in the process of the present invention can contain additives.

Other pharmaceutical formulations comprising the compounds of formula (I), their isomers or the corresponding hydrates or solvates or pharmaceutically acceptable salts, are those formed of micro- and/or nano-particles of lipids, or of proteins, or of pharmaceutically acceptable polymers, wherein the compounds of formula (I), at a concentration comprised between 0.01 and 60% by weight with respect to the lipid, to the protein, or to the polymer, are incorporated inside and/or on surface of the particles.

In the case of lipid particles, those based on fatty acids or esters thereof having a melting point higher than 40°C, more preferably higher than 50°C, can for example be mentioned. For example triglycerides of fatty acids, such as tripalmitine and lanolin, can be cited. The particles can also be formed of mixtures between fatty acids or fatty acid esters having a melting point higher than 40°C and oils that are liquid at room temperature (20-25°C). Examples of the latter are medium chain triglycerides such as vegetable oils, Miglyol^{®} 812 and Miglyol^{®} 810 commercialized by Sasol. Alternatively, these particles can be formed of a surface layer of soya lecithin englobing a core of liquid lipids, formed for example of medium chain triglycerides, such as vegetable oils, Miglyol^{®} 812 and Miglyol^{®} 810.

In the case of polymeric particles, there can be mentioned for example those formed of:
natural polymers, such as proteins, albumin, polysaccharides, as chitosan and dextran,
synthetic polymers such as polyorganophosphazenes, polyanhydrides, polyamides, polyorthoesters, polyalkylcyanoacrylates, polyesters as polylactate (PLA) and polylactate/polyglycolate copolymers (PLA/PLGA).

The particles containing the compounds of formula (I) can optionally be surface modified in order to pass more easily the physiological barriers, such as the haematoencephalic barrier, and/or for achieving a longer residence time in the blood vessel system of the compounds of formula (I). The modification of the particle surface can be carried out both by chemical/physical adsorption of one or more surface modifiers, and by chemical functionalization of the polymer with one or more specific modifiers. In the latter case the modifiers are generally bound with covalent bond to the particles. See for example E. Garcia et Al., "Colloidal carriers and blood-brain barrier (BBB) translocation: A way to deliver drugs to the brain", Int. J. of Pharmaceutics 298 (2005), 274-292.

Among the modifiers there can be mentioned for example: compounds comprising polyoxyethylene or peghilated chains (PEG-based), such as Tween 80, see for example J. Kreuter, "Nanoparticulate systems for brain delivery of drugs", Advanced Drug Delivery Reviews, 47, 2001, 65-81, M.T. Peracchia et al., "Synthesis of a Novel Poly(MePEG cyanoacrylate-co-alkyl cyanoacrylate) amphiphilic copolymer for nanoparticle technology", Macromolecules, 30, 1997, 846-851, proteins, such as plasmatic proteins, for example apolipoproteins can be mentioned, see USP 2004/0131692, antibodies,
compounds which are recognized by specific receptors expressed at the physiological barrier level, such as peptide compounds, proteins, synthetic or natural compounds with a different structure than peptides. See for example L. Costantino et al., "Peptide-derivatized biodegradable nanoparticles able to cross the blood-brain barrier", Journal of Controlled Release, 108, 2005, 84-96, B. Stella et al., "Design of folic acid-coniugated nanoparticles for drug targeting", J. of Pharmaceutical Sciences 89 11, Nov. 2000 1452-1464.

The modifiers of the particle surface can be linked directly to the main polymer structure, as for example in the case of PEG chains of poly(MePEGcyanoacrylate-co-alkyl cyanoacrylate) particles, described in M.T. Peracchia et al., "Synthesis of a Novel Poly(MePEG cyanoacrylate-co-alkyl cyanoacrylate) amphiphilic copolymer for nanoparticle technology", Macromolecules, 30, 1997, 846-851.

The particle surface modifiers can also be covalently linked to the polymer through linkers having a main structure comprising saturated or unsaturated alkyl chains, linear or branched, and/or aromatic and/or polyoxyethylene chains. The linker-polymer and linker-surface modifiers bonds can be both direct bonds C-C and bonds formed by means of functional groups such as ether, amide, ester, urethane, peptide, urea groups.

The above mentioned amphiphilic compounds are selected from the following classes:
- surfactants selected from the non-ionic, anionic, cationic and amphoteric ones, optionally containing fluorine atoms,
- polymers forming organized structures such as aggregates, micelles or vesicles in the liquid wherein they are solubilized.

The preferred surfactants are the non-ionic and anionic ones. Among the non-ionic surfactants, the most preferred are those containing polyoxyalkylene chains, preferably polyoxyethylene chains. The following can for example be mentioned:
polyoxyl 35 castor oil, known for example by the trademark Cremophor^{®} EL (BASF), manufactured by ethoxylation of castor oil,
polyoxyl 40 hydrogenated castor oil, known for example by the trademark Cremophor^{®} RH40 (BASF), manufactured by ethoxylation of hydrogenated castor oil,
polyethylenglycol 15 hydroxystearate, known for example by the trademark Solutol^{®} HS15 (BASF), prepared by reaction of 15 moles of ethylene oxide with 1 mole of 12-hydroxystearic acid,
polyoxyethylene polysorbate, such as Tween^{®} 80, Tween^{®} 20, Tween^{®} 60, Tween^{®} 85,
sorbitan esters of fatty acids, as sorbitan monolaurate and sorbitan monostearate, commercialized for example under the trademark Span^{®} 20 and Span^{®} 60, respectively,
vitamin E/TPGS: tocopheryl propylenglycol 1000 succinate,
polyoxyethylen ethers of fatty acids, as those of the series Brij^{®}, as Brij^{®} 35, Brij^{®} 76, Brij^{®} 98,
PEG-12-acyloxy-stearates, see for example C.E. McNamee et al. in "Physicochemical Characterization of PEG 1500-12-acyloxy-stearate micelles and liquid cristalline phases", Langmuir, 2005, 21, 8146-8154, among the polyoxyethylen ethers of fatty acids the following can for example be mentioned:
   - PEG 1500 mono-12-capryloyloxy stearate (PEG 1500-C₁₈C₈)
   - PEG 1500 mono-12-caproyloxy stearate (PEG 1500-C₁₈C₁₀)
   - PEG 1500 mono-12-lauroyloxy stearate (PEG 1500-C₁₈C₁₂)
   - PEG 1500 mono-12-myristoyloxy stearate (PEG 1500-C₁₈C₁₄)
   - PEG 1500 mono-12-palmitoyloxy stearate (PEG 1500-C₁₈C₁₆).

Among the anionic surfactants the following can for example be mentioned: soya lecithin, for example known with the trademark Epikuron^{®} 200, bis-2-ethylhexylsulphosuccinate (AOT), sodium taurocholate.

Among cationic surfactants, hexadecyltrimethylammonium bromide (CTAB) and didodecylammonium bromide (DDAB) can for example be mentioned.

The polymers which can be used as amphiphilic compounds must be soluble in the aqueous phase and/or in the oily phase. By a soluble polymer it is meant that the polymers must reach in the phase in which they are solubilized concentrations at least equal to those allowing the formation of organized structures as aggregates, micelles, liquid crystals, vesicles. The presence of said organized structures can be detected by specific techniques of the physical chemistry of the dispersed systems, as for example Laser Light Scattering (LLS), Neutron Scattering, microscopy.

The polymers can also be used in combination with the mentioned surfactants. Also in this case the concentration of the solubilized polymer in the used liquid phase must be such to lead to the formation of the above indicated organized structures.

Said polymers are for example polyvinylpyrrolidone and vinylpyrrolidone/vinyl acetate copolymers, commercialized for example under the trademark Kollidon^{®}, as Kollidon^{®} 12PF and Kollidon^{®} 17PF (BASF), and the block copolymers containing polyoxyalkylene chains, more preferably containing polyoxyethylene chains (PEO), as for example the block copolymers PEO with polyoxypropylene chains (PPO) characterized by PEO-PPO-PEO structures, commercially available for example with the trademark Pluronic^{®} or Poloxamer^{®} or Lutrol^{®}, as Lutrol^{®} F68 and Lutrol^{®} F127 commercialized by Basf.

The oils usable for preparing emulsions or as lipids in the particles are selected from the following classes of pharmaceutically acceptable salts:
- esters of C₄-C₃₂ acids, optionally containing one or more unsaturations of ethylene type,
- C₄-C₃₂ acids, optionally containing one or more unsaturations of ethylene type, that can be used when the final composition has a pH such that the acid is not converted into the salt thereof.

The acid esters are preferably obtained by esterification of the corresponding acid, preferably an aliphatic carboxylic acid, more preferably a fatty acid, with an alcohol having an aliphatic chain, preferably C₁-C₅, or having a polyoxy-ethylene chain, or with glycerine. In this case mono-di- or triglycerides are obtained.

The following can for example be mentioned:
oleoyl macrogol 6 glyceride (unsaturated polyglycosylated glyceride), commercialized for example with the trademark Labrafil^{®} 1944 CS, (Gattefossé),
propylenglycol caprylate caprate, known for example under the trademark Labrafac^{®} PG (Gattefossé),
propylenglycol monoester of the caprylic acid, commercialized for example with the trademark Capmul^{®} PG-8 (Abitec),
glycerol oleate (for example Peceol^{®} (Gattefossé)),
medium chain mono- and diglycerides, for example capric and caprylic acid glycerides (for example Capmul^{®} MCM (Abitec), Imwitor^{®} 308 (Sasol)),
polyglycerol oleate (for example Pluro^{®} oleic (Gattefossé)),
capric/caprylic acid triglycerides (for example Miglyol^{®} 812 and Miglyol^{®} 810 (Sasol), Labrafac^{®} CC CS (Gattefossé)),
ethyl butyrate, ethyl caprylate, ethyl oleate,
tripalmitine, commercialized for example with the trademark DYNASAN^{®} 116 by Sasol.

Vegetable oils having a pharmaceutical purity containing one or more of the above mentioned esters can also be used. The soya oil can for example be mentioned.

The C₄-C₃₂ acids are preferably aliphatic carboxylic acids, more preferably fatty acids.

As fatty acid, the stearic acid can be mentioned.

As additives of the emulsions, one or more compounds can be used selected from the following classes:
- modifiers of the water and/or oil polarity,
- modifiers of the film curvature of component S),
- co-surfactants.

The modifiers of the water and/or oil polarity can for example be polyethylenglycols. Lutrol^{®}E300 and Lutrol^{®}E400 (BASF) can be mentioned. Aliphatic alcohols, for example ethanol, can also be used.

The modifiers of the film curvature of component S) are for example aliphatic alcohols, preferably C₂-C₅.

The co-surfactants can for example be surfactant compounds as defined above, or aliphatic alcohols, preferably having a chain with at least 6 carbon atoms. The following can be cited for example:
propylen glycol monolaurate, known for example with the trademark Capmul^{®} PG12 (Gattefossé) or Lauroglycol^{®} 90 (Gattefossé),
caprylocaproyl macrogol 8 glyceride (saturated ethyldiglycosylated glyceride) for example commercialized under the trademarks Labrasol^{®}, Gelucire 44-14 (Gattefossé),
diethylenglycol monoethyl ether, known for example under the trademark Transcutol^{®} (Gattefossé).

A further object of the present invention is the use of pharmaceutical compositions of the present invention for the prophylaxis and therapy in mammals and in an individual of the diseases and disorders in which the receptors of CB1 and/or CB2 cannabinoids are involved.

The diseases and disorders whcih can be treated with the pharmaceutical compositions of the present invention are the following: diseases where immune system cells are involved or immune disorders, osteoporosis, renal ischaemia, inflammatory states, pain, post surgery pain, neuropathic pain, eye diseases, pulmonary diseases as asthma and chronic bronchitis, inflammations such as arthritis, allergies and allergic reactions as for example allergic rhinitis, contact dermatitis, allergic conjunctivitis, anxiety, behaviour problems, delirium states, psychotic problems in general, schizophrenia, depression, use of abuse and/or dependence substances (for example alcoholism and tabagism), vomit, nausea, vertigo, especially in patients under chemotherapy, neuropathies, hemicrania, stress, diseases of psychosomatic origin, epilepsy, Tourette syndrome, Parkinson disease, Huntington disease, Alzheimer disease, senile dementia, cognition disorders and memory loss, pathologies associated to food intake (obesity, bulimia), gastrointestinal tract and bladder pathologies, cardiovascular diseases, urinary, erectile and fertility disorders, neuroinflammatory pathologies such as multiple sclerosis, Guillain-Barré syndrome, viral encephalitis, amyotrophic lateral sclerosis, syndrome associated to demineralization, osteoporosis.

The compounds and the pharmaceutical compositions of the present invention can also be used in the treatment of metabolic and/or cardiovascular risk factors, also in patients with metabolic syndrome and/or dyslipidemia and in patients with type 2 diabetes.

The compounds of formula (I) and thereof pharmaceutical compositions can also be used for the treatment of eye inflammatory conditions, eye autoimmune diseases, uveitis, uveoretinitis and retina neurodegeneration.

For the activity at the periphral level, in particular for the reduction of the intraocular pressure, agonist compounds having affinity for the CB1 cannabinoidergic receptors are used. The preferred compounds of the present invention of formula (I) for said use are those having affinity values for the CB1 cannabinoidergic receptors, expressed as Ki, lower than 200 nM, preferably lower than 100 nM.

The use of the pharmaceutical compositions of the present invention for the treatment of the various pathologies can be performed by using the known methods employed for said treatments.

In particular the administration of the compositions of the invention is carried out so that the amount of active principle is effective for the specific treatment. The dosages, the administration routes and the posologies are established depending on the disease typology, on the pathology severity, on the physical conditions and characteristics of the patient, such as age, weight, response to the active principle, on the pharmacokinetics and toxicology of the active principle for the specific treatment.

The preferred daily dosage is of 0.01-1,000 mg of compound of formula (I) for Kg of body weight of the mammal to be treated. In human beings, the preferred daily range is 0.1-1,000 mg of compound for Kg of body weight, still more preferred from 1 to 800 mg.

The present invention relates furthermore to the compounds of formula (I), or their isomers or the corresponding hydrates or solvates or pharmaceutically acceptable salts, for preparing drugs for the treatment in mammals and in human beings of diseases and disorders wherein the CB1 and/or CB2 cannabinoid receptors are involved.

Said compounds can therefore be used for the treatment of the same above mentioned diseases and disorders for which the pharmaceutical compositions of the invention containing the compounds of formula (I) are used.

A further object of the present invention relates to the use of the compounds of formula (I) as a medicament and in particular for the treatment of the above mentioned diseases and disorders.

The compounds of formula (I), containing radioactive isotopes and the pharmaceutical formulations thereof, can be used for identifying and labelling the receptors of the CB1 and/or CB2 cannabinoids in mammals or in human beings.

Besides, the compounds of formula (I) containing an hydroxyl group, can be used for obtaining ligands. The ligands are detectable by immunochemical methods, to be used in the separation, purification and characterization of the CB1 and/or CB2 cannabinoid receptors in identifying the corresponding active sites.

The following examples are reported for a better understanding of the present invention but are not meant to be limitative of the scope of the invention.

### EXAMPLES

### EXAMPLE 1.1

### Preparation of ethyl 9-chloro-1-(2',4'-dichlorophenyl)-1,4,5,6-tetrahydrobenzo[6,7]cyclohepta[1,2-c]pyrazol-3-carboxylate

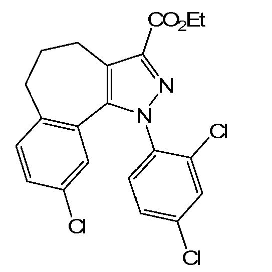

### 1.1a Preparation of ethyl α-(8-chloro-1-oxo-2,3,4,5-tetrahydro-benzocyclohepten-2-yl)-α-oxo-acetate

0.59 grams of metal sodium (25.68 mmoles) were added to 15 ml of absolute ethanol under stirring up to obtain the complete solubilization. 1.88 grams (12.84 mmoles) of diethyloxylate and a solution of 8-chloro-2,3,4,5-tetrahydrobenzocycloheptan-1-one (2.50 g, corresponding to 12.84 mmoles) in absolute ethanol (40 ml) were added to the formerly prepared solution. The reaction mixture was kept under stirring at room temperature for 5 hours and then poured into an ice and HCl 1N mixture, obtaining a white precipitate. The precipitate was filtered, washed with water and dried in the air. 3.67 g (97% yield) of product were obtained corresponding to the compound α-(8-chloro-1-oxo-2,3,4,5-tetrahydro benzocyclohepten-2-yl)-α-oxo-ethyl acetate (Compound 1.1a). Rf = 0.51 (oil ligroin/AcOEt 9.5:0.5 v/v); IR (nujol) (λ= cm⁻¹) 3435, 1731, 1698; ¹H-NMR (CDCl₃) δ 1.41 (t, 3H, J = 7.0 Hz); 2.06 (q, 2H, J = 7.0 Hz); 2.31 (t, 2H, J = 6.4 Hz); 2.71 (t, 2H, J = 7.0 Hz); 4.39 (q, 2H, J = 7.0 Hz); 7.16 (d, 1H, J = 7.8 Hz); 7.42 (dd, 1H, J = 2.0 e 7.8 Hz); 7.60 (d, 1H, J = 2.0 0 Hz); 15.37 (bs, 1H). Anal. calc. for C₁₅H₁₅ClO₄: C, 61.13; H, 5.13; Cl, 12.03. Found: C, 60.98; H, 5.12; Cl, 12.01.

### 1.1b Preparation of ethyl 9-chloro-1-(2',4'-dichlorophenyl)-1,4,5,6-tetrahydrobenzo[6,7]cyclohepta[1,2-c]pyrazol-3-carboxylate

A mixture of the compound 1.1a (1.0 g corresponding to 3.39 mmoles) and 2,4-dichlorophenylhydrazine hydrochloride (0.83 g corresponding to 3.90 mmoles) in 8 ml of glacial acetic acid was heated at reflux for 8 hours, then cooled at room temperature. A precipitate was formed that was filtered, washed with water and dried in the air to give 0.99 g (68% yield) of 9-chloro-1-(2',4'-dichlorophenyl)-1,4,5,6-tetrahydrobenzo [6,7]cyclohepta[1,2-*c*]pyrazol-3-ethyl carboxylate (compound 1.1b). Rf = 0.43 (oil ligroin/AcOEt 9:1 v/v); IR (nujol) (λ= cm⁻¹) 1709; ¹H-NMR (CDCl₃) δ 1.43 (t, 3H, J = 7.0 Hz); 2.10-2.35 (m, 2H); 2.66 (t, 2H, J = 6.8 Hz); 3.10-3.40 (m, 2H); 4.46 (q, 2H, J = 7.2 Hz); 6.65 (s, 1H); 7.15-7.30 (m, 2H); 7.35-7.50 (m, 2H); 7.57 (d, 1H, J = 9.0 Hz). Anal. calc. for C₂₁H₁₇Cl₃N₂O₂: C, 57.89; H, 3.93; Cl, 24.41; N, 6.43. Found: C, 57.74; H, 3.92; Cl, 24.39; N, 6,41.

### EXAMPLE 1.2

### Preparation of ethyl 9-chloro-1-(4'-methylbenzyl)-1,4,5,6-tetrahydrobenzo [6,7]cyclohepta[1,2-c]pyrazol-3-carboxylate

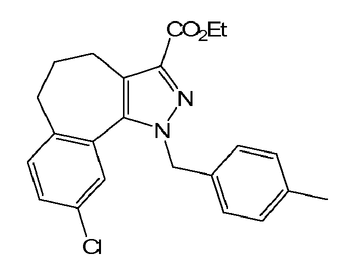

The same procedure described for the preparation of compound 1.1b was repeated, but substituting 2,4-dichloro phenylhydrazine hydrochloride with 4-methylbenzylhydrazine hydrochloride (3.90 mmoles). After filtration, a solid was recovered that after washing and drying, was identified as the compound ethyl 9-chloro-1-(4'-methylbenzyl)-1,4,5,6-tetrahydrobenzo[6,7] cyclohepta[1,2-*c*]pyrazol-3-carboxylate. Yield: 69%. Rf = 0.40 (oil ligroin/AcOEt 8.5:1.5 v/v); IR (nujol) (λ= cm⁻¹) 1719; ¹H-NMR (CDCl₃) 1.42 (t, 3H, J = 7.2 Hz); 2.15-2.39 (m, 2H); 2.37 (s, 3H); 2.64 (t, 2H, J = 6.7 Hz); 3.03-3.35 (m, 2H); 3.56 (s, 2H); 4.45 (q, 2H, J = 7.2 Hz); 6.61 (d, 1H, J = 8.0 Hz); 7.02 (dd, 1H, J = 2.0 and 8.0 Hz); 7.25 (d, 1H, J = 2.0 Hz); 7.26-7.34 (m, 4H). Anal. calc. for C₂₃H₂₃ClN₂O₂: C, 69.95; H, 5.87; Cl, 8.98; N, 7.09. Found: C, 69.91; H, 5.86; Cl, 8,96; N, 7.08.

### EXAMPLE 1.3

### Preparation of ethyl 8-chloro-1-(2',4'-dichlorophenyl)-1,4,5,6-tetrahydrobenzo[6,7]cyclohepta[1,2-c]pyrazol-3-carboxylate

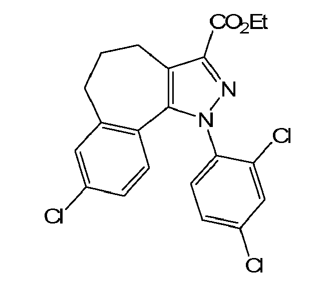

### 1.3a Preparation of ethyl α-(7-chloro-1-oxo-2,3,4,5-tetrahydro-benzocyclohepten-2-yl)-α-oxo-acetate

The same procedure described in example 1.1a was repeated but substituting the compound 8-chloro-2,3,4,5-tetrahydro-benzocycloheptan-1-one with 7-choro-2,3,4,5-tetrahydrobenzo cycloheptan-1-one (12.84 mmoles). The compound ethyl α-(7-choro-1-oxo-2,3,4,5-tetrahydro-benzocyclohepten-2-yl)-α-oxo-acetate (Compound 1.3a) was obtained with a 82% yield. Rf = 0.71 (oil ligroin/AcOEt 1:1 volume/volume); IR (nujol) (λ= cm⁻¹) 3440, 1730, 1680; ¹H-NMR (CDCl₃) δ 1.41 (t, 3H, J = 7.0 Hz); 2.07 (q, 2H, J = 6.8 Hz); 2.32 (t, 2H, J = 6.4 Hz); 2.72 (t, 2H, J = 6.8 Hz); 4.34 (q, 2H, J = 7.0 Hz); 7.22-7.37 (m, 2H); 7.58 (d, 1H, J = 8.2 Hz); 15.37 (bs, 1H). Anal. calc. for C₁₅H₁₅ClO₄: C, 61.13; H, 5.13; Cl, 12.03. Found: C, 61.01; H, 5.11; Cl, 12.00.

### 1.3b Preparation of ethyl 8-chloro-1-(2',4'-dichlorophenyl)-1,4,5,6-tetrahydrobenzo[6,7]cyclohepta[1,2-c]pyrazol-3-carboxylate

The same procedure described in example 1.1b was repeated but the compound reacted with 2,4-dichlorophenylhydrazine hydrochloride (3.90 mmoles) was the compound 1.3a (3.39 mmoles). The compound ethyl 8-chloro-1-(2',4'-dichlorophenyl)-1,4,5,6-tetrahydrobenzo[6,7]cyclohepta[1,2-*c*]pyrazol-3-carboxylate was obtained with a 68% yield. Rf = 0.39 (oil ligroin/AcOEt 8.5:1.5 v/v); IR (nujol) (λ= cm⁻¹) 1724; ¹H-NMR (CDCl₃) δ 1.43 (t, 3H, J = 7.2 Hz); 2.20-2.36 (m, 2H); 2.66 (t, 2H, J = 6.4 Hz); 3.10-3.30 (m, 2H); 4.45 (q, 2H, J = 7.2 Hz); 6.60 (d, 1H, J = 8.4 Hz); 7.02 (dd, 1H, J = 2.0 e 8.4 Hz); 7.31 (d, 1H, J = 2.0 Hz); 7.37-7.42 (m, 2H); 7.54 (d, 1H, J = 9.2 Hz). Anal. calc. for C₂₁H₁₇Cl₃N₂O₂: C, 57.89; H, 3.93; Cl, 24.41; N, 6.43. Found: C, 57.74; H, 3.92; Cl, 24.39; N, 6.41.

### EXAMPLE 1.4

### Preparation of ethyl 8-chloro-1-(4'-methylbenzyl)-1,4,5,6-tetrahydrobenzo[6,7]cyclohepta[1,2-c]pyrazol-3-carboxylate

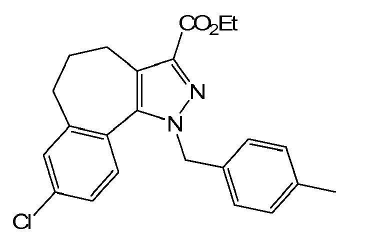

The same procedure described in example 1.1b was repeated but the compound reacting with 4-methylbenzylhydrazine hydrochloride (3.90 mmoles) was the compound 1.3a (3.39 mmoles). The compound ethyl 8-chloro-1-(4'-methylbenzyl)-1,4,5,6-tetrahydro benzo[6,7] cyclohepta-[1,2-*c*]pyrazol-3-carboxylate was obtained with a 73% yield. Rf = 0.42 (oil ligroin/AcOEt 8.5:1.5 v/v); IR (nujol) (λ= cm⁻¹) 1725; ¹H-NMR (CDCl₃) δ 1.41 (t, 3H, J = 7.1 Hz); 2.18-2.38 (m, 2H); 2.36 (s, 3H); 2.65 (t, 2H, J = 6.6 Hz); 3.05-3.32 (m, 2H); 3.55 (s, 2H); 4.44 (q, 2H, J = 7.1 Hz); 6.59 (d, 1H, J = 8.2 Hz); 7.00 (dd, 1H, J = 2.2 and 8.1 Hz); 7.22 (d, 1H, J = 2.1 Hz); 7.24-7.32 (m, 4H). Anal. calc. for C₂₃H₂₃ClN₂O₂: C, 69.95; H, 5.87; Cl, 8.98; N, 7.09. Found: C, 69.88; H, 5.85; Cl, 8.97; N, 7.07.

### EXAMPLE 1.5

### Preparation of ethyl 8-chloro-1-(2',4'-dichlorophenyl)-4,5-dihydrobenzo-1H-6oxa-cyclohepta[1,2-c]pyrazol-3-carboxylate

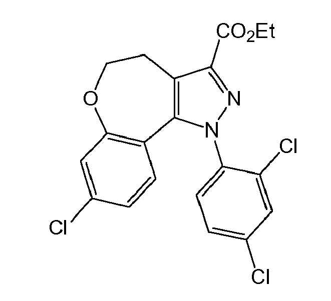

### 1.5a Preparation of the 4-(3-chlorophenoxy)butyric acid

1 eq of NaOH (flakes) was dispersed in 1 eq of 3-chlorophenol and the thus obtained dispersion heated to 170°C up to complete solubilization of the base. 1.4 eq of γ-butyrolactone were dropwise added to the solution, and the reaction mixture maintained at 170°C for 5 hours. The reaction mixture was then poured into ice and then acidified with HCl 6N. The reaction product was extracted with CHCl₃, dehydrated with Na₂SO₄ and concentrated under vacuum. The obtained residue was purified by flash chromatography (oil ligroin/ethyl acetate 4:1 volume/volume). The acid 4-(3-chloro phenoxy)butyric (yellow solid) was obtained with a 47% yield. R_{f} = 0.15 (oil ligroin/ethyl acetate 4:1); m.p. 47°C; IR (nujol) (λ= cm⁻¹) 3223 (OH), 1709 (CO); ¹H-NMR (CDCl₃) δ 2.11 (qu, 2H, J = 6.8 Hz), 2.58 (t, 2H, J = 7.4 Hz), 4.0 (t, 2H, J = 5.6 Hz), 6.76 (d, 1H, J = 8.2 Hz), 6.88 (s, 1H), 6.92 (d, 1H, J = 9.2 Hz), 7.18 (t, 1H, J = 7.8 Hz); Anal. Calc. for C₁₀HClO₃: C, 55.98; H, 5.17; Cl, 16.51. Found: C, 55.84; H, 5.16; Cl, 16.50.

### 1.5b Preparation of the compound 8-chloro-1-oxo-2,3,4,5-tetrahydrobenzocycloheptan-5-one

27.96 mmoles of the 4-(3-chlorophenoxy)butyric acid obtained in example 1.5a were added to 48 grams of polyphosphoric acid; the resulting mixture was maintained under stirring at 90°C for 2 hours and then poured on ice. The reaction mixture was extracted with CH₂Cl₂, the pooled organic phases washed with an aqueous solution of Na₂CO₃ at 10%, dehydrated on Na₂SO₄ and concentrated under vacuum. The obtained residue was purified by flash chromatography (oil ligroin/ethyl acetate 9:1 v/v). The compound 8-chloro-1-oxo-2,3,4,5-tetrahydrobenzo-cycloheptan-5-one was separated as an orange coloured oil in a 46% yield. B.p. 46-47°C/27mmHg; ¹H-NMR (CDCl₃) δ 2.22 (qu, 2H, J = 6.4 Hz), 2.89 (t, 2H, J = 6.8 Hz), 4.25 (t, 2H, J = 6.6 Hz), 7.05-7.16 (m, 2H), 7.71 (d, 1H, J = 7. 0 Hz) ; Anal. Calc. for C₁₀H₉ClO₂: C, 61.09; H, 4.61; Cl, 18.03. Found: C, 60.93; H, 4.60; Cl, 18.01.

### 1.5c Preparation of diketoester ethyl γ-(7-chloro-5-oxo-2,3,4,5-tetrahydrobenzocycloheptan-2-yl)-α-oxoacetate

2 eq of metal sodium were added to 5 ml of anhydrous ethanol. The obtained dispersion was maintained under stirring at room temperature up to complete sodium reaction. 1 eq of ethyl oxalate and 30 ml of a solution of the ketonic compound obtained in example 1.5b (1 eq) in anhydrous ethanol were added to the formerly prepared solution. The reaction mixture was maintained under stirring at room temperature for 1.5 hours and then poured on a mixture of ice + HCl 2N. The obtained solution was extracted with ethyl acetate. The organic phase was recovered and washed with water, dehydrated on Na₂SO₄ and concentrated under vacuum. The residue was purified by flash chromatography (oil ligroin/ethyl acetate 4:1 volume/volume). The compound ethyl γ-(7-chloro-5-oxo-2,3,4,5-tetrahydrobenzo-cycloheptan-2-yl)-α-oxoacetate was thus separated in a 90% yield. R_{f} = 0.46 (oil ligroin/ethyl acetate 4:1); m.p. 135 °C; IR (nujol) (λ= cm⁻¹) 1823 (CO), 1713 (CO), 1683 (CO); ¹H-NMR (CDCl₃/DMSO) δ 1.09 (t, 3H, J = 7.2 Hz), 1.27 (t, 3H, J = 6.8 Hz), 3.34-3.38 (m, 2H), 4.17 (q, 2H, J = 7.0 Hz), 6.89 (s, 1H), 7.01 (d, 1H, J = 6.2 Hz), 7.62 (d, 1H, J = 8.4 Hz) ; Anal. Calc. for (C₁₄H₁₃ClO₅): C, 56.67; H, 4.41; Cl, 11.95. Found: C, 56.58; H, 4.37; Cl, 11.94.

### 1.5d Preparation of the compound ethyl 8-chloro-1-(2',4'-dichlorophenyl)-4,5-dihydrobenzo-1H-6oxa-cyclohepta[1,2-c]-pyrazol-3-carboxylate

1 eq of the diketoester obtained in example 1.5c and 1.1 eq of 2,4-dichlorophenylhydrazine hydrochloride in 50 ml of ethanol were heated at reflux for 90 minutes. The reaction solvent was then removed under vacuum and the obtained residue purified by flash chromatography (oil ligroin/EtOAc 9:1). The compound ethyl 8-chloro-1-(2',4'-dichlorophenyl)-4,5-dihydrobenzo-1*H*-6oxa-cyclo-hepta[1,2-*c*]pyrazol-3-carboxylate was thus obtained as an orange solid (47.5% yield). *R_{f}* = 0.32 (oil ligroin/EtOAc 9:1); m.p. 130-131 °C; IR (nujol) (λ= cm⁻¹) 1712 (CO); ¹H-NMR (CDCl₃) δ 1.42 (t, 3H, J = 7.0 Hz), 3.44 (qu, 2H, J = 5.4 Hz), 4.35-4.51 (m, 4H), 6.65 (d, 1H, J = 8.6 Hz), 6.81 (d, 1H, J = 8.6 Hz), 7.14 (s, 1H) 7.39-7.44 (m, 2H), 7.49 (s, 1H); Anal. Calc. for C₂₀H₁₅Cl₃N₂O₂: C, 54.88; H, 24.30; Cl, 6.40; N, 3.45. Found: C, 54.80; H, 24.26; Cl, 6.39; N, 3.44.

### EXAMPLE 1.6

### Preparation of ethyl 7-chloro-1-(2',4'-dichlorophenyl)-6-methyl-1,4-dihydroindeno[1,2-c]pyrazole-3-carboxylate

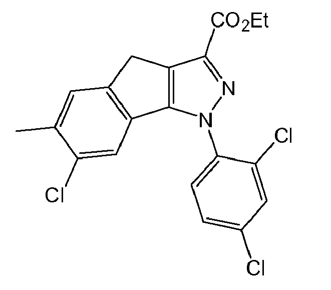

### 1.6a Preparation of ethyl 2-(6-chloro-5-methyl-1-oxo-2,3-dihydro-1H-inden-2-yl)-2-oxoacetate

Metal sodium (0.17 g, 7.5 mmol) was added in small pieces to absolute ethanol (3.5 ml) and the mixture left under stirring until complete solubilization. Diethyloxalate (0.51 ml, 3.75 mmol) was added to the alcohol solution, followed by a dropwise addition of a solution of 6-chloro-5-methylindan-1-one (12.21 mmol) in absolute ethanol (27 ml). The reaction mixture was stirred at room temperature for 9 hours. The reaction was stopped by pouring the liquid phase on a mixture of ice and HCl 1N, followed by extraction with chloroform (3 x 15 ml). The combined extracts were washed with water, dried over anhydrous sodium sulfate, filtered, and evaporated under reduced pressure. The compound ethyl 2-(6-chloro-5-methyl-1-oxo-2,3-dihydro-1*H*-inden-2-yl)-2-oxoacetate was isolated as an orange oil (96% yield), having an analytical grade purity. Rf=0.21 (petroleum ether/ethyl acetate 9/1 v/v); IR (nujol) (λ = cm⁻¹) 3440, 1730, 1680; ¹H-NMR (CDCl₃) δ 1.43 (t, 3H, J = 7.2 Hz); 2.49 (s, 3H); 3.92 (s, 2H); 4.42 (q, 2H, J = 7.2 Hz); 7.42 (s, 1H); 7.82 (s, 1H); 13.20 (bs, 1H). Anal. calc. for C₁₄H₁₃ClO₄: C, 59.90; H, 4.67; Cl, 12.63. Found: C, 58.10; H, 4.71; Cl, 12.67.

### 1.6b Preparation of ethyl 7-chloro-1-(2',4'-dichlorophenyl)-6-methyl-1,4-dihydroindeno[1,2-c]pyrazole-3-carboxylate

A mixture of compound 1.6a (0.9 g, 3.05 mmol) and 2,4-dichlorophenylhydrazine hydrochloride (0.72 g, 3.38 mmol) in ethyl alcohol (21 ml) was stirred at the reflux temperature for 8 hours. The solvent was then removed under reduced pressure and the crude ester was isolated. Purification of said compound by flash chromatography on silica gel, elution solvent petroleum ether/ethyl acetate (8.5/1.5 v/v) gave the compound ethyl 7-chloro-1-(2',4'-dichlorophenyl)-6-methyl-1,4-dihydroindeno[1,2-*c*]pyrazole-3-carboxylate as a yellow solid (99% yield).
IR (nujol) (λ = cm⁻¹) 1725; ¹H-NMR (CDCl₃) δ 1.44 (t, 3H, J = 7.0 Hz); 2.41 (s, 3H); 3.80 (s, 2H); 4.44 (q, 2H, J = 7.2 Hz); 6.93 (s, 1H); 7.43-7.75 (m, 4H). Anal. calc. for C₂₀H₁₅Cl₃N₂O₂: C, 56.96; H, 3.59; Cl, 25.22; N, 6.64. Found: C, 57.16; H, 3.61; Cl, 25.26; N, 6.67.

### EXAMPLE 1.7

### Preparation of ethyl 7-chloro-6-methyl-1-(4'-methylbenzyl)-1,4-di hydroindeno[1,2-c]pyrazole-3-carboxylate

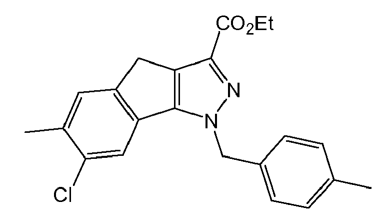

The same procedure described in Example 1.6b was repeated but that compound 1.6a was reacted with 4-methylbenzylhydrazine hydro chloride instead of 2,4-dichlorophenylhydrazine hydrochloride. Yield 95%. IR (nujol) (λ = cm⁻¹) 1724; ¹H-NMR (CDCl₃) δ 1.44 (t, 3H, J = 7.2 Hz); 2.32 (s, 3H); 2.34 (s, 3H); 3.70 (s, 2H) ; 4.45 (q, 2H, J = 7.0 Hz); 5.59 (s, 2H); 6.98-7.39 (m, 6H). Anal. calc. for C₂₂H₂₁ClN₂O₂: C, 69.38; H, 5.56; Cl, 9.31; N, 7.36. Found: C, 69.78; H, 5.53; Cl, 9.34; N, 7.39.

### EXAMPLE 1.8

### Preparation of ethyl 6-chloro-7-methyl-1-(4'-methylbenzyl)-1,4-di hydroindeno[1,2-c]pyrazole-3-carboxylate

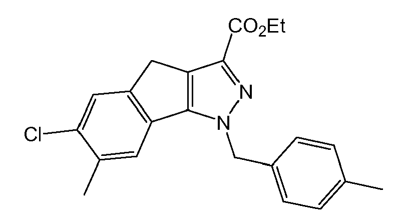

### 1.8a Preparation of ethyl 2-(5-chloro-6-methyl-1-oxo-2,3-dihydro-1H-inden-2-yl)-2-oxoacetate

The same procedure described in ex. 1.6a was repeated, but for dripping in the initial alcoholic solution 5-chloro-6-methylindan-1-one (8.99 mmol) instead of 6-chloro-5-methylindan-1-one. Yield 87%. Rf=0.50 (petroleum ether/ethyl acetate 7/3 v/v); IR (nujol) (λ = cm⁻¹) 3445, 1725, 1685; ¹H-NMR (CDCl₃) δ 1.43 (t, 3H, J = 7.2 Hz); 2.45 (s, 3H); 3.92 (s, 2H); 4.42 (q, 2H, J = 7.2 Hz); 7.54 (s, 1H); 7.71 (s, 1H); 14.50 (bs, 1H). Anal. calc. for C₁₄H₁₃ClO₄: C, 59. 90; H, 4.67; Cl, 12.63. Found: C, 57.60; H, 4.69; Cl, 12.66.

### 1.8b Preparation of ethyl 6-chloro-7-methyl-1-(4'-methylbenzyl)-1,4-dihydroindeno[1,2-c]pyrazole-3-carboxylate

The same procedure described in ex. 1.6b was repeated but for reacting the compound 1.8a with 4-methylbenzylhydrazine hydro chloride instead of 2,4-dichlorophenylhydrazine hydrochloride. Yield 88%. Rf=0.21 (petroleum ether/ethyl acetate 9:1). IR (nujol) (λ = cm⁻¹) 1725; ¹H-NMR (CDCl₃) δ 1.43 (t, 3H, J = 7.2 Hz); 2.31 (s, 3H); 2.38 (s, 3H); 3.69 (s, 2H); 4.45 (q, 2H, J = 7.0 Hz); 5.56 (s, 2H); 7.00-7.40 (m, 6H). Anal. calc. for C₂₂H₂₁ClN₂O₂: C, 69.38; H, 5.56; Cl, 9.31; N, 7.36. Found: C, 69.31; H, 5.54; Cl, 9.30; N, 7.34.

### EXAMPLE 1.9

### Preparation of the ethyl ester of the 1-(2,4-dichlorophenyl)-6-methyl-1H-benzofuro[3,2-c]pyrazole-3-carboxylic acid

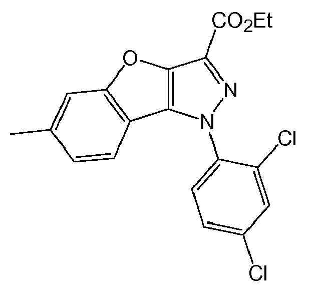

### 1.9a Preparation of 6-methylbenzofuran-3(2H)-one

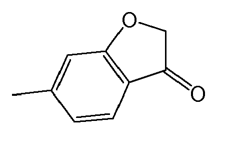

1-bromo-2-(2-hydroxy-4-methylphenyl)acetophenone was obtained according to the synthesis described by L. C. King et al. in J. Org. Chem. 29 (1964) 3459-3461, by reacting 1-(2-hydroxy-4-methyl-phenyl)-ethanone with CuBr₂ in ethyl-acetate at 77 °C.

A solution of 1-bromo-2-(2-hydroxy-4-methylphenyl)-acetophenone (1.0 g, 4.36 mmol) and sodium acetate (0.36 g, 4.38 mmol) in absolute ethanol (10 ml) was refluxed under stirring for 15 hours. The obtained mixture was poured into water and extracted with dichloromethane (3 x 10 ml). The organic phase was dried over Na₂SO₄, then concentrated under reduced pressure to obtain an oil which was purified by flash chromatography (oil ether/ethyl ether 9/1 v/v on silica gel). 0.25 g (38% yield) of a yellow solid, corresponding to 6-methylbenzofuran-3(2H)-one were recovered.

### 1.9b Preparation of ethyl 2-(3-hydroxy-6-methylbenzofuran-2yl)-2-oxoacetate

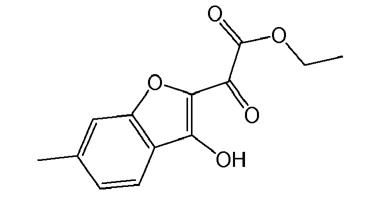

Metal sodium (0.13 g; 2.24 mmol) was added in small pieces to absolute ethanol (3 ml). The suspsension was left under reflux until complete solubilization of sodium. To the so obtained solution diethyloxalate (0.80 ml; 5.87 mmol) was added, followed by dripping a solution of 1-bromo-2-(2-hydroxy-4-methylphenyl)acetophenone (0.39 g; 2.63 mmol) in absolute ethanol (30 ml). The reaction mixture was kept under stirring at room temperature for 20 hours, then poured in a mixture of ice and HC1 1N. The aqueous solution is extracted with chloroform (3 x 20 ml). The organic phase was dried over Na₂SO₄, then concentrated under reduced pressure to obtain an oil which is triturated with oil ether/ethyl ether. 0.47 g (73% yield) of the compound are recovered under the form of a yellow solid. Rf=0.48 (dichloromethane/ acetone 7/3); m.p.: 113-115 °C; IR (nujol) (λ = cm⁻¹) 3406 (OH as tautomer mixture), 1691 (COOEt), 1651 (C=O); ¹H-NMR (CDCl₃) δ 1.50 (t, J=7.2 Hz, 3H), 2.51 (s,3H), 4.56 (q, J=7.2 Hz, 2H), 7.13 (d, J=8.4 Hz, 1H), 7.26 (s, 1H), 7.71 (d, J=8.4 Hz, 1H), 11.87 (brs, 1H); API-ESI (Atmospheric Pressure Ionization - Electron Spray Ionization) calc. for 248.23, found 248.10.

### 1.9c Preparation of (Z)-ethyl 2-(2-(2,4-dichlorophenyl)-hydrazone)-2-(3-hydroxy-6-methylbenzofuran-2-yl)acetate

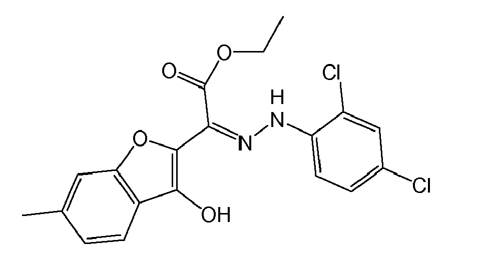

A solution in absolute ethanol (1.15 ml) of the compound obtained in Ex. 1.9b (1.07g; 4.31 mmol)and 2,4-dichlorophenylhydrazine hydrochloride (1.20 g; 5.60 mmol) was prepared. The solution was reacted at the reflux temperature for 1.5 hours, then cooled to room temperature and poured on ice. The resulting precipitate was filtered under reduced pressure, then air dried to obtain 1.37 g (78% yield) of a solid residue corresponding to (Z)-ethyl 2-(2-(2,4-dichlorophenyl)hydrazone)-2-(3-hydroxy-6-methylbenzofuran-2-yl)acetate. Rf=0.42 (oil ether/ethyl acetate 9.5/0.5 v/v on silica gel); m.p.: 190-192 °C; IR (nujol) (λ = cm⁻¹) 3423 (OH as tautomer mixture), 1619 (COOEt); ¹H-NMR (CDCl₃) δ 0.83 (t, J=7.4 Hz, 3H), 2.46 (s, 3H), 4.07 (q, J=7.0 Hz, 2H), 6.93 (d, J=0.8 Hz), 7.35 (s, 2H), 7.60 (d, J=8.6 Hz, 2H), 12.80 (s, 1H); API-ESI calc. for 407.25, found 407.10.

### 1.9d Preparation of the ethyl ester of the 1-(2,4-dichlorophenyl)-6-methyl-1H-benzofuro[3,2-c]pyrazole-3-carboxylic acid

To a solution in toluene (6 ml) of the compound prepared in Ex. 1.9d (0.5g; 1.23 mmol) a catalytic amount of p-toluensulfonic acid (0.023 g, 0.123 mmol) was added. The obtained mixture was reacted at the reflux temperature for 30 hours, then the solvent was removed under reduced pressure. The residue was purified by flash chromatography (oil ether/ethyl ether 8/2 v/v on silica gel). 0.25 g (52% yield) of a yellow solid, corresponding to ethyl ester of 1-(2,4-dichlorophenyl)-6-methyl-1H-benzofuro[3,2-*c*]pyrazole-3-carboxylic acid were recovered. Rf=0.30 (oil ether/ethyl ether 8/2 volume/volume on silica gel); m.p.: 138-140 °C; IR (nujol) (λ=cm⁻¹) 1635 (COOEt); ¹H-NMR (CDCl₃) δ 1.49 (t, J=7.2 Hz, 3H), 2.51 (s, 3H), 4.55 (q, J=7.4 Hz, 2H), 7.10 (d, J=8.6 Hz, 1H), 7.31 (d, J=8.0 Hz, 1H), 7.43-7.48 (m, 2H), 7.64-7.67 (m, 2H); API-ESI calc. for 389.23, found 389.05.

### EXAMPLE 2.1

### Preparation of the 9-chloro-1-(2',4'-dichlorophenyl)-1,4,5,6-tetrahydrobenzo[6,7]cyclohepta[1,2-c]pyrazol-3-carboxylic acid

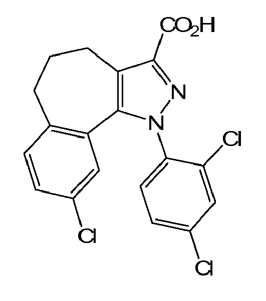

1.00 grams (2.29 mmoles) of the ester obtained in example 1.1b were solubilized in 15 ml of EtOH/H₂O 1:1 (v/v). 1.67 grams (29.77 mmoles) of solid KOH were added to the formerly prepared solution. The reaction mixture was kept under stirring at the reflux temperature for 4 hours and then poured into a mixture of ice + HCl 1N. The so obtained white precipitate was filtered, washed with water and dried in the air obtaining 0.91 g of the 9-chloro-1-(2',4'-dichlorophenyl)-1,4,5,6-tetrahydrobenzo [6,7]cyclohepta[1,2-*c*] pyrazol-3-carboxylic acid (Compound 2.1). Yield 98%. Rf = 0.60 (CHCl₃/MeOH 8.5:1.5 volume/volume); IR (nujol) (λ= cm⁻¹) 3419, 1716; ¹H-NMR (CDCl₃) δ 2.25-2.27 (m, 2H); 2.67 (t, 2H, J = 6.4 Hz); 3.07-3.32 (m, 2H); 4.78 (bs, 1H); 6.65 (d, 1H, J = 1.8 Hz); 7.20-7.32 (m, 2H); 7.40-7.50 (m, 2H); 7.57 (d, 1H, J = 9.0 Hz). Anal. calc. for C₁₉H₁₃Cl₃N₂O₂: C, 55.98; H, 3.21; Cl, 26.09; N, 6.87. Found: C, 55.85; H, 3.19; Cl, 26.05; N, 6.86.

### EXAMPLE 2.2

### Preparation of the 9-chloro-1-(4'-methylbenzyl)-1,4,5,6-tetrahydrobenzo[6,7]cyclohepta[1,2-c]pyrazol-3-carboxylic acid

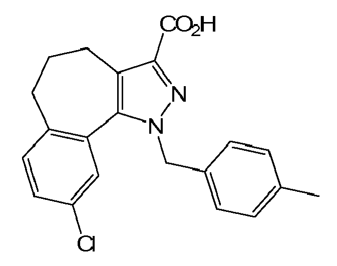

The same procedure described in example 2.1 was repeated, but substituting the ester obtained in example 1.1 with that of example 1.2. The 9-chloro-1-(4'-methylbenzyl)-1,4,5,6-tetrahydro-benzo[6,7]cyclohepta[1,2-*c*]pyrazol-3-carboxylic acid was obtained with a 94% yield. Rf = 0.36 (oil ligroin/AcOEt 8.5:1.5 v/v); IR (nujol) (λ= cm⁻¹) 3422, 1717; ¹H-NMR (CDCl₃) δ 2.18-2.38 (m, 2H); 2.36 (s, 3H); 266 (t, 2H, J = 6.8 Hz); 3.05-3.36 (m, 2H); 3.58 (s, 2H); 6.63 (d, 1H, J = 8.1 Hz); 7.01 (dd, 1H, J = 2.2 and 8.1 Hz); 7.22 (d, 1H, J = 2.1 Hz); 7.24-7.35 (m, 4H). Anal. calc. for C₂₁H₁₉ClN₂O₂: C, 68.76; H, 5.22; Cl, 9.66; N, 7.64. Found: C, 68.61; H, 5.21; Cl, 9.64; N, 7.62.

### EXAMPLE 2.3

### Preparation of the 8-chloro-1-(2',4'-dichlorophenyl)-1,4,5,6-tetrahydrobenzo[6,7]cyclohepta[1,2-c]pyrazol-3-carboxylic acid

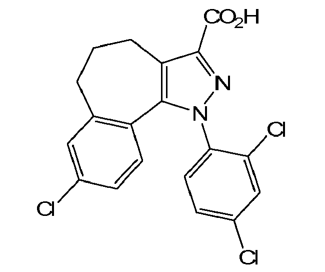

The same procedure described in example 2.1 was repeated, but substituting the ester obtained in example 1.1 with that of example 1.3. The 8-chloro-1-(2',4'-dichlorophenyl)-1,4,5,6-tetrahydrobenzo[6,7]cyclo hepta[1,2-c]pyrazol-3-carboxylic acid was thus obtained with a 94% yield. IR (nujol) (λ= cm⁻¹) 3410, 1715; ¹H-NMR (CDCl₃) δ 2.25-2.30 (m, 2H); 2.68 (t, 2H, J = 6.4 Hz); 3.10-3.23 (m, 2H); 4.50 (bs, 1H); 6.61 (d, 1H, J = 8.4 Hz); 7.03 (dd, 1H, J = 2.2 and 8.2 Hz); 7.32 (d, 1H, J = 2.0 Hz); 7.39-7.44 (m, 2H); 7.52 (d, 1H, J = 8.0 Hz) . Anal. calc. for C₁₉H₁₃Cl₃N₂O₂: C, 55.98; H, 3.21; Cl, 26.09; N, 6.87. Found: C, 55.82; H, 3.20; Cl, 26.06; N, 6.85.

### EXAMPLE 2.4

### Preparation of the 8-chloro-1-(4'-methylbenzyl)-1,4,5,6-tetrahydrobenzo[6,7]cyclohepta[1,2-c]pyrazol-3-carboxylic acid

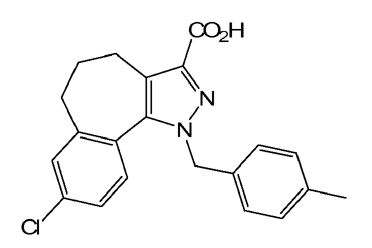

The same procedure described in example 2.1 was repeated, but substituting the ester obtained in example 1.1 with that of example 1.4. The 8-chloro-1-(4'-methylbenzyl)-1,4,5,6-tetrahydrobenzo[6,7]cyclohepta[1,2-*c*]pyrazol-3-carboxylic acid was thus obtained with a 93% yield. IR (nujol) (λ= cm⁻¹) 3412, 1716; ¹H-NMR (CDCl₃) δ 2.15-2.36 (m, 2H); 2.35 (s, 3H); 2.66 (t, 2H, J = 6.8 Hz); 3.04-3.31 (m, 2H); 3.54 (s, 2H); 6.57 (d, 1H, J = 8.1 Hz); 7.01 (dd, 1H, J = 2.0 and 8,1 Hz); 7.23 (d, 1H, J = 2.0 Hz); 7.22-7.31 (m, 4H). Anal. calc. for C₂₁H₁₉ClN₂O₂: C, 68.76; H, 5.22; Cl, 9.66; N, 7.64. Found: C, 68.69; H, 5.20; Cl, 9.65; N, 7.63.

### EXAMPLE 2.5

### Preparation of the 8-chloro-1-(2',4'-dichlorophenyl)-4,5-dihydrobenzo-1H-6oxa-cyclohepta[1,2-c]pyrazol-3-carboxylic acid

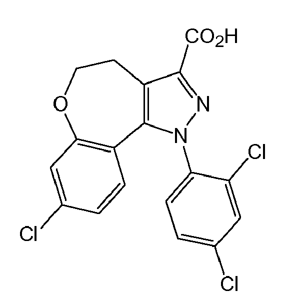

An amount equal to 1 equivalent of the ester obtained in example 1.5 was dispersed in 10 ml of CH₃OH. To said dispersion 7 ml of CH₃OH containing 2 eq of potassium hydroxide were added. The methanol solution thus prepared was maintained at reflux for 12 hours and then poured into a mixture of ice and HC1 1N. A yellow precipitate was thus obtained which was filtered, washed with water and then dried under nitrogen flow. The compound 8-chloro-1-(2',4'-dichlorophenyl)-4,5-dihydrobenzo-1*H*-6oxa-CyClohepta[1,2-c]pyrazol-3-carboxylic acid was isolated with a 91.2% yield. *R_{f}*=0.38 (CHCl₃/MeOH 9:1); m.p. 230-231°C; IR (nujol) (λ= cm⁻¹) 1689 (CO); ¹H-NMR (CDCl₃/DMSO) δ 3.10-3.45 (br s, 3H, 10H exchang. with D₂O ), 6.67 (d, 1H, J = 8.4 Hz), 6.83 (d, 1H, J = 8.2 Hz), 7.13 (s, 1H), 7.44-7.50 (m, 3H); Anal. Calc. for C₁₈H₁₁Cl₃N₂O₂: C, 52.77; H, 2.70; Cl, 25.96; N, 6.83. Found: C, 52.65; H, 2.69; Cl, 25.94; N, 6.81.

### EXAMPLE 2.6

### Preparation of 7-chloro-1-(2',4'-dichlorophenyl)-6-methyl-1,4-di hydroindeno[1,2-c]pyrazole-3-carboxylic acid

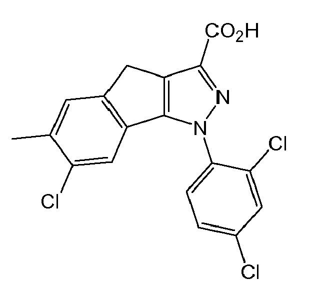

A mixture of ethyl ester 1.6 (0.64 g, 1.47 mmol) and KOH (0.17 g, 2.94 mmol) in methanol (12 ml) was refluxed for 12 hours. After said period of time a solution was formed, that was then cooled to room temperature and poured on a mixture of ice and HCl 1N. A precipitate was separated. The precipitate was filtered, washed with water, and dried under vacuum. It was obtained the compound 7-chloro-1-(2',4'-dichlorophenyl)-6-methyl-1,4-dihydroindeno[1,2-*c*]pyrazole-3-carboxylic acid as a white solid. Yield 98%. IR (nujol) (λ.= cm⁻¹) 3410, 1690; ¹H-NMR (DMSO) δ 2.41 (s, 3H); 3.79 (s, 2H); 6.94 (s, 1H); 7.35-7.75 (m, 4H). Anal. calc. for C₁₈H₁₁Cl₃N₂O₂: C, 54.92; H, 2.82; Cl, 27.02; N, 7.12. Found: C, 54.78; H, 2.80; Cl, 26.99; N, 7.11.

### EXAMPLE 2.7

### Preparation of 7-chloro-6-methyl-1-(4'-methylbenzyl)-1,4-dihydro indeno[1,2-c]pyrazole-3-carboxylic acid

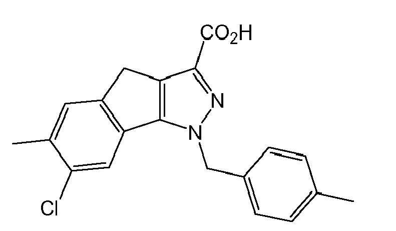

The same procedure of ex. 2.6 was repeated to convert the ethyl ester compound of ex. 1.7 into the corresponding acid. The compound 7-chloro-6-methyl-1-(4'-methylbenzyl)-1,4-dihydroindeno [1,2-*c*] pyrazole-3-carboxylic acid was obtained (yield 96%). IR (nujol) (λ.= cm⁻¹) 3410, 1690; ¹H-NMR (DMSO) δ 2.27 (s, 3H); 2.36 (s, 3H); 3.66 (s, 2H); 5.67 (s, 2H); 6.99-7.40 (m, 4H); 7.51 (s, 1H) ; 7.57 (s, 1H) ; 12.70 (bs, 1H) . Anal. calc. for C₂₀H₁₇ClN₂O₂: C, 68.09; H, 4.86; Cl, 10.05; N, 7.94. Found: C, 68.02; H, 4.84; Cl, 10.03; N, 7.93.

### EXAMPLE 2.8

### Preparation of 6-chloro-7-methyl-1-(4'-methylbenzyl)-1,4-dihydro indeno[1,2-c]pyrazole-3-carboxylic acid

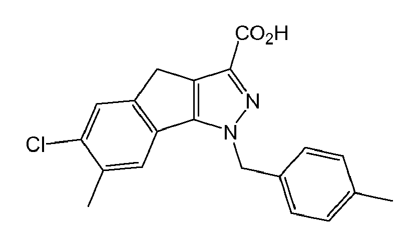

The same procedure of ex. 2.6 was repeated to convert the ethyl ester of ex. 1.8 into the corresponding acid. The compound 6-chloro-7-methyl-1-(4'-methylbenzyl)-1,4-dihydroindeno[1,2-*c*] pyrazole-3-carboxylic acid was isolated (yield 85%) . IR (nujol) (λ.= cm⁻¹) 3410, 1690; ¹H-NMR (DMSO) δ 2.26 (s, 3H); 2.36 (s, 3H); 3.66 (s, 2H); 5.67 (s, 2H); 7.16 (m, 4H); 7.40-7.70 (m, 2H); 12.70 (bs, 1H). Anal. calc. for C₂₀H₁₇ClN₂O₂: C, 68.09; H, 4.86; Cl, 10.05; N, 7.94. Found: C, 68.07; H, 4.85; Cl, 10.02; N, 7.92.

### EXAMPLE 2.9

### Preparation of 1-(2,4-dichlorophenyl)-6-methyl-1H-benzofuro-[3,2-c]pyrazole-3-carboxylic acid

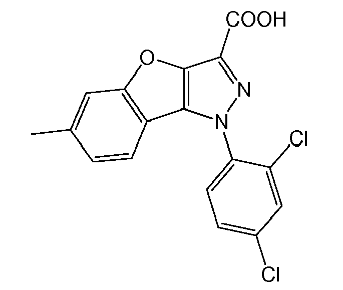

The compound obtained in Ex. 1.9 (0.17 g, 0.44 mmol) and potassium hydroxide (0.32 g, 5.7 mmol) were reacted in ethanol/water 1/1 (v/v) solution (5.6) ml at the reflux temperature for 4 hours. The reacted mixture was cooled to room temperature and then poured into a mixture of ice and HCl 1N. A precipitate was formed, that was filtered under reduced pressure, washed with water and air-dried. A solid residue corresponding to 1-(2,4-dichlorophenyl)-6-methyl-1H-benzofuro[3,2-c]pyrazole-3-carboxylic acid was obtained. Yield was quantitative. Rf=0.30 (chloroform/methanol 8/2 v/v on silica gel); m.p.: 228-230°C; IR (nujol) (λ = cm⁻¹) 3417 (OH), 1637 (COOH); ¹H-NMR (CDCl₃) δ 2.51 (s, 3H), 3.57 (bs, 1H), 7.13 (d, J=8.2 Hz, 2H), 7.34 (d, J=7.8 Hz), 7.45-7.55 (m, 2H), 7.67-7.72 (m, 2H); API-ESI calc. for 361.18, found 360.15.

### EXAMPLE 3.1

### Preparation of N-myrtanyl-9-chloro-1-(2',4'-dichlorophenyl)-1,4,5,6-tetrahydrobenzo[6,7]cyclohepta[1,2-c]pyrazol-3-carboxamide

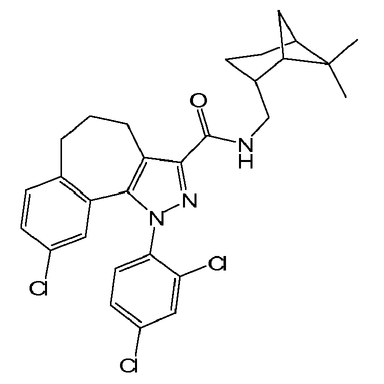

0.70 grams (1.72 mmoles) of the acid obtained in example 2.1, *N*-(3-dimethylamminopropyl)-*N*'-ethylcarbodiimide hydrochloride (EDC) (1.2 eq) and hydrate 1-hydroxybenzotriazol (HOBt) (1.2 eq) were added to 6 ml of CH₂Cl₂. The obtained mixture was maintained under stirring at room temperature for one hour. To the mixture a solution of (-)-*cis*-myrtanylamine (2 eq) in CH₂Cl₂ (6 ml) was dropwise added. The resulting reaction mixture was maintained under stirring at room temperature for 14 hours and then concentrated under vacuum. The residue was purified by flash chromatography (oil ligroin /AcOEt 8.5:1.5 v/v) to obtain the compound N-myrtanyl-9-chloro-1-(2',4'-dichlorophenyl) -1,4,5,6-tetrahydrobenzo-[6,7]-cyclohepta [1,2-*c*]pyrazol-3-carboxamide with a 87% yield. Rf = 0.50 (oil ligroin/AcOEt 6:4 v/v); IR (nujol) (λ= cm⁻¹) 3405, 1664; ¹H-NMR (CDCl₃) δ 1.08 (s, 3H); 1.20 (s, 3H); 1.53-1.61 (m, 5H); 1.84-2.03 (m, 4H); 2.19-2.30 (m, 4H); 2.63 (t, 2H, J = 6.4 Hz); 3.37-3.46 (m, 2H); 6.61 (s, 1H); 6.93 (bt, 1H, J = 5.5 Hz); 6.98-7.10 (m, 1H); 7.17-7.23 (m, 2H); 7.49-7.55 (m, 2H). Anal. calc. for C₂₉H₃₀Cl₃N₃O: C, 64.15; H, 5.57; Cl, 19.59; N, 7.74. Found: C, 64.03; H, 5.56; Cl, 19.57; N, 7.73.

### EXAMPLE 3.2

### Preparation of N-myrtanyl-9-chloro-1-(4'-methylbenzyl)-1,4,5,6-tetrahydrobenzo[6,7]cyclohepta[1,2-c]pyrazol-3-carboxamide

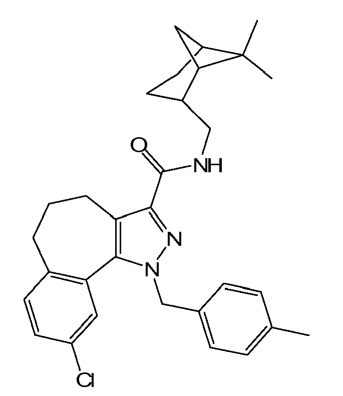

The same procedure described in example 3.1 was repeated, but substituting the acid obtained in example 2.1 with the compound obtained in example 2.2. At the end of the reaction the compound N-myrtanyl-9-chloro-1-(4'-methylbenzyl)-1,4,5,6-tetrahydrobenzo[6,7]cyclo-hepta[1,2-*c*] pyrazol-3-carboxamide was obtained with a 60% yield. Rf = 0.48 (oil ligroin/AcOEt 7:3 v/v); IR (nujol) (λ= cm⁻¹) 3408, 1659; ¹H-NMR (CDCl₃) δ 1.15 (s, 3H); 1.19 (s, 3H); 1.90-2.14 (m, 11H); 2.31 (s, 3H); 2.42-2.46 (m, 2H); 2.85-2.89 (m, 2H); 3.39-3.45 (m, 2H); 5.38 (s, 2H); 6.94-7.22 (m, 7H). Anal. calc. for C₂₉H₃₀Cl₃N₃O: C, 64.15; H, 5.57; Cl, 19.59; N, 7.74. Found: C, 64.03; H, 5.56; Cl, 19.57; N, 7.73.

### EXAMPLE 3.3

### Preparation of N-myrtanyl-8-chloro-1-(2',4'-dichlorophenyl)-4,5-dihydrobenzo-1H-6oxa-cyclohepta[1,2-c]pyrazol-3-carboxamide

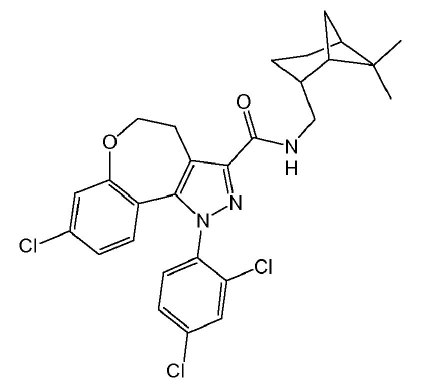

An amount equal to 1 eq of the acid obtained in example 2.5, *N*-(3-dimethylamminopropyl)-*N*'-ethylcarbodiimide hydrochloride (EDC) (1.2 eq) and 1-hydroxybenzotriazole hydrate (HOBt) (1.2 eq) were added to 2 ml of CH₂Cl₂. The obtained mixture was kept under stirring at room temperature for 30 minutes. Then a solution of (-)-*cis*-myrtanylamine (2 eq) in CH₂Cl₂ (2 ml) was dropwise added to the mixture. The resulting reaction mixture was kept under stirring at room temperature for 10 hours and then concentrated under vacuum. The residue from concentration was purified by flash chromatography (oil ligroin /AcOEt 9:1 volume/volume) obtaining the compound N-myrtanyl-8-chloro-1-(2',4'-dichlorophenyl)-4,5-dihydrobenzo-1*H*-6oxa-cyclo-hepta[1,2-*c*] pyrazol-3-carboxamide (white solid) with a 90% yield. Rf = 0.31 (oil ligroin/AcOEt 9:1 v/v); m.p. 142-143 142-143 °C; IR (nujol) (λ= cm⁻¹) 3423 (NH), 1676 (CO); ¹H-NMR (CDCl₃) δ 0.82-0.94 (m, 1H), 1.07 (s, 3H), 1.19 (s, 3H), 1.45-1.62 (m, 1H), 1.82-2.04 (m, 4H), 2.21-2.43 (m, 2H), 3.26-3.57 (m, 4H), 4.35-4.42 (m, 2H), 6.60 (d, 1H, J= 8.6 Hz), 6.81 (d, 1H, J= 8.6 Hz), 6.90-6.97 (m, 1H), 7.14 (s, 1H), 7.31-7.39 (m, 2H), 7.54 (br s, 1H, NH, exchang. with D₂O) ; ¹³C-NMR (CDCl₃) δ 19.84 (CH₂), 23.22 (CH₃), 26.00 (CH₂), 27.08 (CH₂), 27.96 (CH₃), 33.26 (CH₂), 38.70 (C), 41.32 (CH), 41.50 (CH), 43.80 (CH), 44.57 (CH₂), 73.56 (CH₂), 120.03 (C), 122.75 (CH), 123.44 (CH), 128.01 (CH), 128.33 (CH), 130.32 (CH), 130.67 (CH), 132.93 (C), 134.61 (C), 136.05 (C), 137.16 (C), 139.05 (C), 144.51 (C), 159.62 (C), 162.25 (CO); Anal. Calc. for C₂₈H₂₇Cl₃N₃O₂: C, 61.83; H, 5.00; Cl, 19.55; N, 7.72. Found: C, 61.79; H, 4.99; Cl, 19.53; N, 7.71.

### EXAMPLE 3.4

### Preparation of N-myrtanyl-8-chloro-1-(2',4'-dichlorophenyl)-1,4,5,6-tetrahydrobenzo[6,7]cyclohepta[1,2-c]pyrazol-3-carboxamide

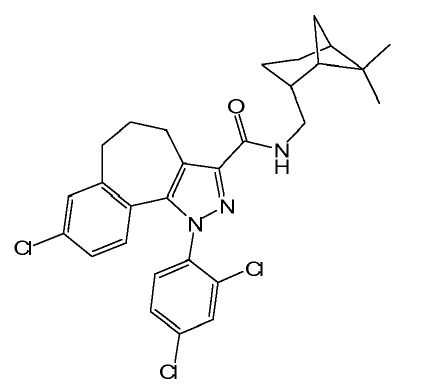

The same procedure described in example 3.1 was repeated, but substituting the acid obtained in example 2.1 with the compound obtained in example 2.3. At the end of the reaction the compound N-myrtanyl-8-chloro-1-(2',4'-dichlorophenyl)-1,4,5,6-tetrahydrobenzo[6,7]cyclohepta[1,2-*c*] pyrazol-3-carboxamide was obtained with a 81% yield. Rf = 0.48 (oil ligroin/AcOEt 6:4 v/v); IR (nujol) (λ= cm⁻¹) 3400, 1662; ¹H-NMR (CDCl₃) δ 1.07 (s, 3H); 1.21 (s, 3H); 1.52-1.63 (m, 5H); 1.82-2.05 (m, 4H); 2.15-2.29 (m, 4H); 2.64 (t, 2H, J = 6.6 Hz); 3.35-3.45 (m, 2H); 6.59 (d, 1H, J = 8.0 Hz); 7.00 (dd, 1H, J = 2.2 and 8.0 Hz); 7.25-7.32 (m, 1H); 7.37-7.43 (m, 3H); 7.51 (bt, 1H, J = 5.4 Hz). Anal. calc. for C₂₉H₃₀Cl₃N₃O: C, 64.15; H, 5.57; Cl, 19.59; N, 7.74. Found: C, 64.09; H, 5.55; Cl, 19.56; N, 7.72.

### EXAMPLE 3.5

### Preparation of N-cyclohexylmethyl-8-chloro-1-(2',4'-dichloro phenyl)-1,4,5,6-tetrahydrobenzo[6,7]cyclohepta[1,2-c]pyrazole-3-carboxamide

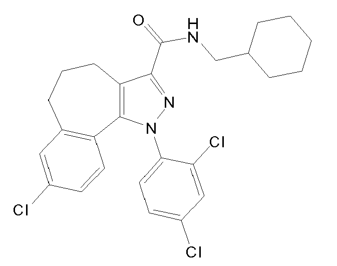

A mixture of the carboxylic acid compound of ex. 2.3 (0.2 g, 0.49 mmol), of *N*-(3-dimethylaminopropyl)-*N*'-ethylcarbodiimidehydro chloride (EDC) (1.2 eq) and 1-hydroxybenzotriazole hydrate (HOBt) (1.2 eq) in CH₂Cl₂ (5 ml) was stirred at room temperature for 1 hour. A solution of cyclohexylmethylamine (2 eq) in CH₂Cl₂ (3 ml) was then dripped and the reaction mixture stirred at room temperature for 14 hours. The solvent was removed under reduced pressure. The residue was purified by flash chromatography (petroleum ether/ethyl acetate 9/1 v/v) obtaining 0.11 g (44% yield) of the compound *N*-cyclohexylmethyl-8-chloro-1-(2',4'-dichlorophenyl)-1,4,5,6-tetrahydrobenzo[6,7]cyclohepta[1,2-*c*] pyrazole-3-carboxamide as a white solid. Rf=0.45 (petroleum ether/ethyl acetate 9:1 volume/volume). IR (nujol) (λ= cm⁻¹) 3410, 1670; ¹H-NMR (CDCl₃) δ 0.92-1.03 (m, 2H); 1.10-1.32 (m, 4H); 1.53-1.84 (m, 5H); 2.20-2.31 (m, 2H); 2.60-2.72 (m, 3H); 2.80-3.15 (m, 1H); 3.20-3.30 (m, 2H); 6.58 (d, 1H, J = 8.0 Hz); 6.97-7.08 (m, 2H); 7.30 (d, 1H, J = 1.6 Hz); 7.40 (dd, 1H, J = 1 .7 and 8.4 Hz); 7.42-7.48 (m, 2H). Anal. calc. for C₂₆H₂₆Cl₃N₃O: C, 62.10; H, 5.21; Cl, 21.15; N, 8.36. Found: C, 62.03; H, 5.20; Cl, 21.13; N, 8.34.

### EXAMPLE 3.6

### Preparation of N-(1-adamantylmethyl)-8-chloro-1-(2',4'-dichloro phenyl)-1,4,5,6-tetrahydrobenzo[6,7]cyclohepta[1,2-c]pyrazole-3-carboxamide

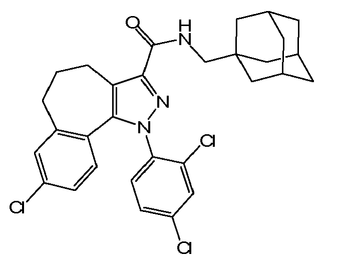

The same procedure of ex. 3.5 was repeated, but the carboxylic acid of ex. 2.3 was reacted with 1-adamantylmethylamine instead of cyclohexylmethylamine. Yield 59%. Rf=0.41 (petroleum ether/ethyl acetate 9/1 v/v). IR (nujol) (λ = cm⁻¹ ) 3405, 1669; ¹H-NMR (CDCl₃) δ 1.57 (bs, 6H); 1.61-1.75 (m, 6H); 1.95-2.02 (m, 3H); 2.20-2.30 (m, 2H); 2.63-2.73 (m, 3H); 2.82-3.18 (m, 3H); 6.58 (d, 1H, J = 8.4 Hz); 6.97-7.09 (m, 2H); 7.30 (bs, 1H); 7.40 (dd, 1H, J = 1.9 and 8.4 Hz); 7.44-7.50 (m, 2H). Anal. calc. for C₃₀H₃₀Cl₃N₃O: C, 64.93; H, 5.45; Cl, 19.17; N, 7.57. Found: C, 64.81; H, 5.44; Cl, 19.10; N, 7.55.

### EXAMPLE 3.7

### Preparation of N-tetrahydrofurfuryl-8-chloro-1-(2',4'-dichloro phenyl)-1,4,5,6-tetrahydrobenzo[6,7]cyclohepta[1,2-c]pyrazole-3-carboxamide

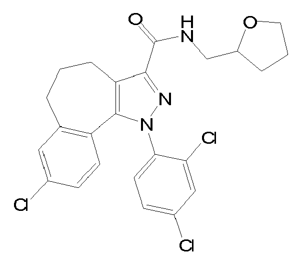

The same procedure of ex. 3.5 was repeated but using tetrahydrofurfurylamine instead of cyclohexylmethylamine. Yield 54%. Rf=0.38 (petroleum ether/ethyl acetate 7/3 v/v). IR (nujol) (λ = cm⁻¹) 3409, 1667; ¹H-NMR (CDCl₃) δ 1.58-1.74 (m, 1H); 1.84-1.96 (m, 2H); 1.97-2.08 (m, 1H); 2.19-2.31 (m, 2H); 2.61-2.72 (m, 2H); 2.75-3.28 (m, 2H); 3.31-3.41 (m, 1H); 3.66-3.81 (m, 2H); 3.84-3.94 (m, 1H); 4.04-4.12 (m, 1H); 6.57 (d, 1H, J = 8.0 Hz); 7.00 (dd, 1H, J = 1.9 and 8.4 Hz); 7.27-7.32 (m, 2H); 7.39 (dd, 1H, J = 1.9 and 8.4 Hz); 7.41-7.48 (m, 2H). Anal. calc. for C₂₄H₂₂Cl₃N₃O₂: C, 58.73; H, 4.52; Cl, 21.67; N, 8.56. Found: C, 58.69; H, 4.51; Cl, 21.65; N, 8.54.

### EXAMPLE 3.8

### Preparation of N-myrtanyl-7-chloro-1-(2',4'-dichlorophenyl)-6-methyl-1,4-dihydroindeno[1,2-c]pyrazole-3-carboxamide

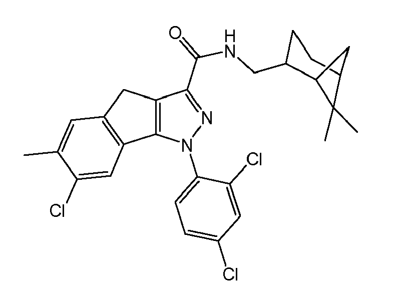

The same procedure of ex. 3.5 was followed but using in the first reaction synthesis the carboxylic acid compound of ex 2.6 instead of that of ex. 2.3, and substituting the dropwise addition of cyclohexylmethylamine with that of (-)-*cis-*myrtanylamine. Yield 78%. Rf=0.26 (petroleum ether/ethyl acetate 9/1 v/v). IR (nujol) (λ = cm⁻¹) 3378, 1683; ¹H-NMR (CDCl₃) δ 0.90-1.75 (m, 11H); 1.75-2.20 (m, 4H); 2.41 (s, 3H); 3.30-3.63 (m, 2H); 3.84 (s, 2H); 3.87-4.00 (m, 1H); 6.94 (s, 1H); 7.40-7.62 (m, 3H); 7.68 (d, 1H, J = 1.8 Hz). Anal. calc. for C₂₈H₂₈Cl₃N₃O: C, 63.58; H, 5.34; Cl, 20.11; N, 7.94. Found: C, 63.98; H, 5.37; Cl, 20.31; N, 7.98.

### EXAMPLE 3.9

### Preparation of N-(1-cyclohexylethyl)-7-chloro-1-(2',4'-dichloro phenyl)-6-methyl-1,4-dihydroindeno[1,2-c]pyrazole-3-carboxamide

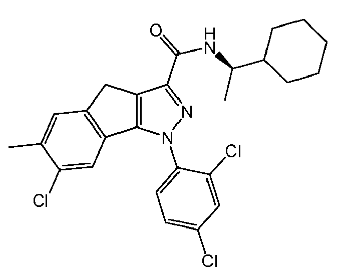

The same procedure of ex. 3.8 was repeated but that the solution dripped in the reaction mixture contained R-(-)-1-cyclohexylethylamine instead of (-)-*cis*-myrtanylamine. Yield 20%. Rf=0.49 (petroleum ether/ethyl acetate 9/1 v/v). IR (nujol) (λ = cm⁻¹) 3305, 1646; ¹H-NMR (CDCl₃) δ 1.00-2.94 (m, 14H); 2.41 (s, 3H); 3.84 (s, 2H); 3.97-4.20 (m, 1H); 6.77 (d, 1H, J = 9.0 Hz); 6.94 (s, 1H); 7.43 (s, 1H); 7.48 (dd, 1H, J = 1.8 and 8.6 Hz); 7.56 (d, 1H, J = 8.6 Hz); 7.68 (d, 1H, J = 2.0 Hz). Anal. calc. for C₂₆H₂₆Cl₃N₃O: C, 62.10; H, 5.21; Cl, 21.15; N, 8.36. Found: C, 62.40; H, 5.23; Cl, 21.18; N, 8.38.

### EXAMPLE 3.10

### Preparation of N-(1-adamantylmethyl)-7-chloro-1-(2',4'-dichlor ophenyl)-6-methyl-1,4-dihydroindeno[1,2-c]pyrazole-3-carboxamide

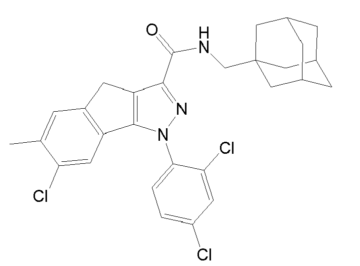

The same procedure of ex. 3.8 was repeated but that the solution dripped in the reaction mixture contained 1-adamantylmethylamine instead of (-)-*cis*-myrtanylamine. Yield 58%. Rf=0.35 (petroleum ether/ethyl acetate 9/1 v/v). IR (nujol) (λ = cm⁻¹) 3252, 1643; ¹H-NMR (CDCl₃) δ 1.32-2.14 (m, 15H); 2.41 (s, 3H); 3.14 (d, 2H, J = 6.7 Hz); 3.84 (s, 2H); 6.95 (s, 1H); 6.99 (bs, 1H); 7.43 (s, 1H); 7.48 (dd, 1H, J = 2.2 and 8.6 Hz); 7.56 (d, 1H, J = 8.6 Hz); 7.68 (d, 1H, J = 1.8 Hz). Anal. calc. for C₂₉H₂₈Cl₃N₃O: C, 64.39; H, 5.22; Cl, 19.66; N, 7.77. Found: C, 64.69; H, 5.25; Cl, 19.69; N, 8.06.

### EXAMPLE 3.11

### Preparation of N-myrtanyl-7-chloro-6-methyl-1-(4'-methylbenzyl)-1,4-dihydroindeno[1,2-c]pyrazole-3-carboxamide

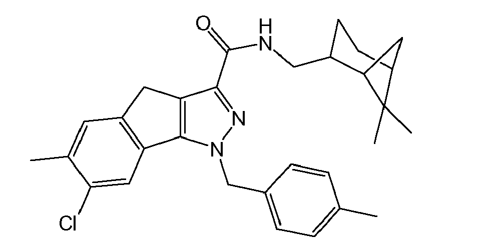

The same procedure of ex. 3.5 was repeated but that the carboxylic acid prepared in ex. 2.7 instead of that of ex. 2.3 was reacted, and the solution dripped in the reaction mixture contained (-)-*cis*-myrtanylamine instead of cyclohexylmethylamine. Yield 50%. Rf=0.45 (petroleum ether/ethyl acetate 8/2 v/v). IR (nujol) (λ = cm⁻¹) 3356, 1679; ¹H-NMR (CDCl₃) δ 1.02 (s, 3H); 1.14 (s, 3H); 1.40-1.70 (m, 2H); 1.75-2.05 (m, 4H); 2.25 (s, 3H); 2.31 (s, 3H); 3.25-3.55 (m, 2H); 3.68 (s, 2H); 5.41 (s, 2H); 6.86 (bs, 1H); 7.00-7.30 (m, 6H). Anal. calc. for C₃₀H₃₄ClN₃O: C, 73.83; H, 7.02; Cl, 7.26; N, 8.61. Found: C, 73.77; H, 7.00; Cl, 7.25; N, 8.60.

### EXAMPLE 3.12

### Preparation of N-myrtanyl-6-chloro-7-methyl-1-(4'-methylbenzyl)-1,4-dihydroindeno[1,2-c]pyrazole-3-carboxamide

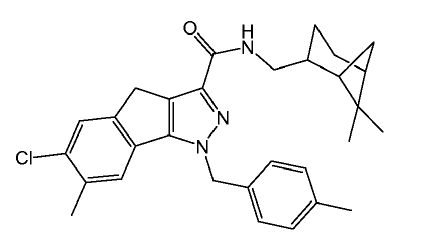

The same procedure of ex. 3.5 was repeated but that the carboxylic acid prepared in ex. 2.8 instead of that of ex. 2.3 was reacted, and the solution dripped in the reaction mixture contained (-)-*cis*-myrtanylamine instead of cyclohexylmethylamine. Yield 68%. Rf=0.30 (petroleum ether/ethyl acetate 9/1 v/v). IR (nujol) (λ = cm⁻¹) 3381, 1674; ¹H-NMR (CDCl₃) δ 1.09 (s, 3H); 1.21 (s, 3H); 1.50-1.80 (m, 2H); 1.90-2.10 (m, 4H); 2.22-2.50 (m, 6H); 3.25-3.60 (m, 2H); 3.76 (s, 2H); 5.51 (s, 2H); 6.92 (bs, 1H); 7.00-7.50 (m, 6H). Anal. calc. for C₃₀H₃₄ClN₃O: C, 73.83; H, 7.02; Cl, 7.26; N, 8.61. Found: C, 73.80; H, 7.01; Cl, 7.24; N, 8.58.

### EXAMPLE 3.13

### Preparation of 8-chloro-1-(2',4'-dichlorophenyl)-3-(1-oxo-2-cyclo hexyleth-1-yl)-1,4,5,6-tetrahydrobenzo[6,7]-cyclohepta-[1,2-c] pyrazole

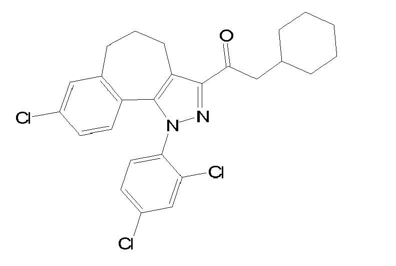

### 3.13a Preparation of N-methoxy-N-methyl-8-chloro-1-(2',4'-dichloro phenyl)-1,4,5,6-tetrahydrobenzo[6,7]cyclohepta[1,2-c]pyrazole-3-carboxamide

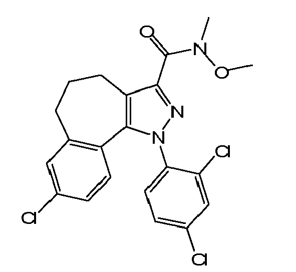

Trimethylaluminum (0.92 ml of a 2 M solution of the compound in hexane, 1.84 mmol) was added dropwise to a suspension of dimethylhydroxylamine hydrochloride (0.18 g, 1.84 mmol) in CH₂Cl₂ (3 ml) at 0 °C. The reaction mixture was stirred at 0 °C for 45 minutes, then at room temperature for 40 minutes. After this period of time a clear solution was obtained. Under stirring to said solution a solution in CH₂Cl₂ (2 ml) of the compound obtained in Ex. 1.3 (0.4 g, 0.92 mmol) was added dropwise. Stirring was continued for further 4 hours at room temperature. The reaction mixture was cooled to 0 °C, and 10% HCl was carefully added dropwise. The mixture was extracted with CH₂Cl₂, washed with water, brine, dried over Na₂SO₄, and filtered. The residue isolated after evaporation of the solvent under reduced pressure was purified by flash chromatography (petroleum ether/ethyl acetate 7/3 volume/volume) obtaining 0.33 g of the compound N-methoxy-N-methyl-8-chloro-1-(2',4'-dichlorophenyl)-1,4,5,6-tetrahydro-benzo [6,7]cyclohepta[1,2-*c*]pyrazole-3-carboxamide as a white solid (80% yield). Rf=0.38 (petroleum ether/ethyl acetate 7/3). IR (nujol) (λ = cm⁻¹) 1681; ¹H-NMR (CDCl₃) δ 2.21-2.30 (m, 2H); 2.64-2.75 (m, 4H); 3.46 (s, 3H); 3.80 (s, 3H); 6.60 (d, 1H, J = 8.3 Hz); 7.02 (dd, 1H, J = 2.2 and 8.3 Hz); 7.30 (d, 1H, J = 1.6 Hz) ; 7.36 (dd, 1H, J = 2.2 and 8.3 Hz); 7.40 (d, 1H, J = 8.3 Hz); 7.45 (d, 1H, J = 2.2 Hz). Anal. calc. for C₂₁H₁₈Cl₃N₃O₂: C, 55.96; H, 4.03; Cl, 23.60; N, 9.32. Found: C, 55.82; H, 4.02; Cl, 23.57; N, 9.30.

### 3.13b Preparation of 8-chloro-1-(2',4'-dichlorophenyl)-3-(1-oxo-2-cyclohexyleth-1-yl)-1,4,5,6-tetrahydrobenzo[6,7]-cyclohepta[1,2-c]pyrazole

3.86 ml of a 0.5 M cyclohexylmethylmagnesium bromide solution in THF were added dropwise at 0°C under a nitrogen atmosphere to 6 ml of THF solution containing 0.29 g (0.64 mmol) of the compound obtained in ex. 3.13a. The temperature of the reaction mixture was slowly raised to room temperature and stirred at this temperature for 24 hours. At the end of this period the temperature of the mixture was lowered to 0°C. 15 ml of a saturated NH₄Cl aqueous solution previously conditioned at 0 °C were added dropwise. The temperature of the reaction mixture was slowly raised to room temperature and then diluted with ethylacetate (15 ml). The aqueous and the organic phases were thus separated. The aqueous layer was extracted with ethylacetate (3x10 ml), and the combined organic layers were washed with water, dried (Na₂SO₄), and filtered. After evaporation of the solvent under reduced pressure a residue was recovered, that was purified by flash chromatography (petroleum ether/diethyl ether 9/1 v/v). 80 mg (26% yield) of compound 8-chloro-1-(2',4'-dichlorophenyl)-3-(1-oxo-2-cyclohexyl eth-1-yl)-1,4,5,6-tetrahydrobenzo[6,7]-cyclohepta[1,2-*c*]pyrazole were recovered as a white solid. Rf=0.56 (petroleum ether/diethyl ether 9/1). IR (nujol) (λ = cm⁻¹) 1685; ¹H-NMR (CDCl₃) δ 0.98-1.11 (m, 2H); 1.13-1.38 (m, 4H); 1.62-1.81 (m, 4H); 1.97-2.10 (m, 1H); 2.18-2.29 (m, 2H); 2.62-2.71 (m, 2H); 2.85-3.18 (m, 4H); 6.58 (d, 1H, J = 8.3 Hz); 7.00 (dd, 1H, J = 2.2 and 8.3 Hz); 7.30 (d, 1H, J = 2.2 Hz); 7.40 (dd, 1H, J = 2.2 and 8.3 Hz); 7.44-7.48 (m, 2H). Anal. calc. for C₂₆H₂₅Cl₃N₂O: C, 64.01; H, 5.17; Cl, 21.80; N, 5.74. Found: C, 63.89; H, 5.16; Cl, 21.77; N, 5.72.

### EXAMPLE 3.14

### Preparation of 8-chloro-1-(2',4'-dichlorophenyl)-3-(1-hydroxy-2-cyclohexyleth-1-yl)-1,4,5,6-tetrahydrobenzo[6,7]cyclohepta[1,2-c] pyrazole

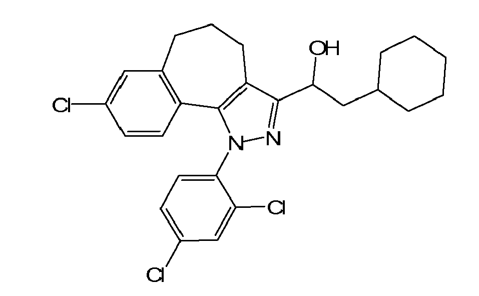

To a suspension in methyl alcohol (3 ml) of the keto compound prepared in Example 3.13 (60 mg, 0.12 mmol) sodium borohydride (10 mg, 0.25 mmol) was added. The mixture was stirred at room temperature for 2 hours, then diluted with CHCl₃ and washed with water. The organic layer was recovered and dried over anhydrous sodium sulfate. The organic phase was then filtered and concentrated under reduced pressure. 60 mg (99% yield) of the compound 8-chloro-1-(2',4'-dichlorophenyl)-3-(1-hydroxy-2-cyclo hexyleth-1-yl)-1,4,5,6-tetrahydrobenzo-[6,7]cyclohepta[1,2-*c*] pyrazole were recovered as a white solid. Rf=0.34 (petroleum ether/ethyl acetate 8/2 volume/volume). IR (nujol) (λ = cm⁻¹) 3315; ¹H-NMR (CDCl₃) δ 0.84-1.05 (m, 2H); 1.08-1.24 (m, 4H); 1.34-1.78 (m, 5H); 1.80-1.90 (m, 2H); 2.14-2.26 (m, 2H); 2.46-2.72 (m, 4H); 5.00 (bs, 1H); 6.60 (d, 1H, J = 8.3 Hz); 7.01 (dd, 1H, J = 2.2 and 8.3 Hz); 7.29 (d, 1H, J = 2.2 Hz); 7.35 (dd, 1H, J = 2.2 and 8.9 Hz); 7.40-7.45 (m, 2H). Anal. calc. for C₂₆H₂₇Cl₃N₂O: C, 63.75; H, 5.56; Cl, 21.71; N, 5.72. Found: C, 63.68; H, 5.55; Cl, 21.69; N, 5.71.

### EXAMPLE 3.15

### Preparation of N-myrtanyl-1-(2',4'-dichlorophenyl)-6-methyl-1,4-dihydroindeno[1,2-c]pyrazole-3-carboxamide

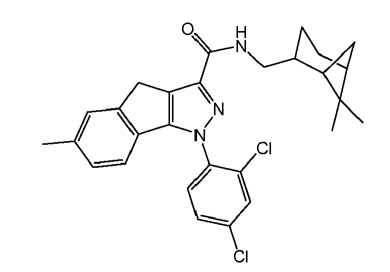

The same procedure of ex. 3.5 was repeated but that the starting mixture contained 1-(2',4'-dichlorophenyl)-6-methyl-1,4-dihydro indeno[1,2-*c*]pyrazole-3-carboxylic acid, prepared as reported in literature (Mussinu J.M., Ruiu S., Mulè A.C., Pau A., Carai M.A.M., Loriga G., Murineddu G., Pinna G.A., Bioorg. Med. Chem. 2003, 11, 251-263) instead of the carboxylic acid prepared in the ex. 2.3 and that the solution dripped in the reaction mixture contained (-)-*cis-*myrtanylamine instead of cyclohexyl methyl amine. Yield 56%. Rf = 0.50 (petroleum ether/ethyl acetate 8.5/1.5 v/v). IR (nujol) (λ = cm⁻¹) 3350, 1685; ¹H-NMR (CDCl₃) δ 0.93 (d, 1H, J = 12.0 Hz); 1.11 (s,3H), 1.23 (s,3H), 1.55-1.68 (m, 3H); 1.84-2.09 (m, 4H); 2.30-2.45 (m, 1H); 2.42 (s, 3H); 3.38-3.46 (m, 1H); 3.50-3.58 (m, 1H); 3.88 (s, 2H); 6.90 (d, 1H, J = 7.9 Hz); 6.97 (bt, 1H, J = 6.4 Hz); 7.06 (bd, 1H, J = 7.7 Hz); 7.40 (bs, 1H) ; 7.49 (dd, 1H, J = 2.0 and 7.9 Hz) ; 7.57 (d, 1H, J = 7.8 Hz); 7.68 (d, 1H, J = 2.2 Hz). Anal. calc. for C₂₈H₂₉Cl₂N₃O: C, 68.01; H, 5.91; Cl, 14.34; N, 8.50. Found: C, 67.89; H, 5.90; Cl, 14.31; N, 8.48.

### EXAMPLE 3.16

### Preparation of N-cyclohexylmethyl-1-(2',4'-dichlorophenyl)-6-methyl 1,4-dihydroindeno[1,2-c]pyrazole-3-carboxamide

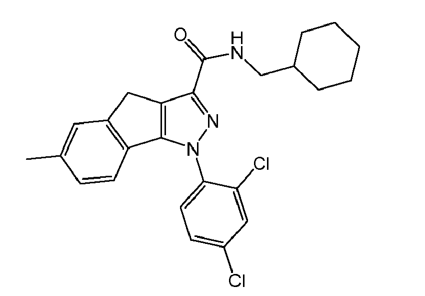

The same procedure of ex. 3.15 was repeated but that the solution dripped in the reaction mixture contained cyclohexylmethylamine instead of (-)-*cis*-myrtanylamine. Yield 84%. Rf=0.26 (petroleum ether/ethyl acetate 9:1 volume/volume). IR (nujol) (λ = cm⁻¹) 3360, 1685; ¹H-NMR (CDCl₃) δ 0.93-1.05 (m, 2H); 1.10-1.30 (m, 3H); 1.53-1.84 (m, 6H); 2.39 (s, 3H); 3.25-3.32 (m, 2H); 3.85 (s, 2H); 6.87 (d, 1H, J = 7.8 Hz) ; 6.98 (bt, 1H, J = 6.6 Hz) 7.03 (bd, 1H, J = 7.8 Hz); 7.37 (bs, 1H); 7.45 (dd, 1H, J = 2.2 and 8.1 Hz); 7.54 (d, 1H, J = 7.9 Hz); 7.63 (d, 1H, J = 2.2 Hz). Anal. calc. for C₂₅H₂₅Cl₂N₃O: C, 66.08; H, 5.55; Cl, 15.60; N, 9.25. Found: C, 66.01; H, 5.54; Cl, 15.58; N, 9.24.

### EXAMPLE 3.17

### Preparation of N-(1-adamantylmethyl)-1-(2',4'-dichlorophenyl)-6-methyl-1,4-dihydroindeno[1,2-c]pyrazole-3-carboxamide

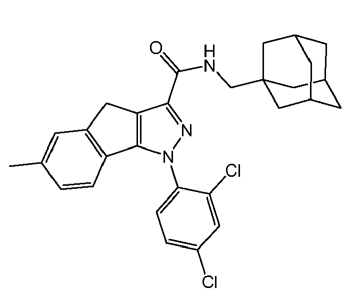

The same procedure of ex. 3.15 was repeated but that the solution dripped in the reaction mixture contained 1-adamantylmethylamine instead of (-)-*cis*-myrtanylamine. Yield 77%. Rf=0.31 (petroleum ether/ethyl acetate 9/1 v/v). IR (nujol) (λ = cm⁻¹) 3374, 1682; ¹H-NMR (CDCl₃) δ 1.55-1.76 (m, 12H); 1.98 (bs, 3H); 2.38 (s, 3H); 3.14 (d, 2H, J = 7.8 Hz); 3.85 (s, 2H); 6.88 (d, 1H, J = 7.8 Hz) ; 6.97 (bt, 1H, J = 6.4 Hz); 7.02 (bd, 1H, J = 7.7 Hz); 7.37 (bs, 1H); 7.45 (dd, 1H, J = 2.1 and 8.0 Hz); 7.55 (d, 1H, J = 7.8 Hz); 7.65 (d, 1H, J = 2.0 Hz). Anal. calc. for C₂₉H₂₉Cl₂N₃O: C, 68.77; H, 5.77; Cl, 14.00; N, 8.30. Found: C, 68.72; H, 5.76; Cl, 13.98; N, 8.28.

### EXAMPLE 3.18

### Preparation of 1-(2',4'-dichlorophenyl)-6-methyl-3-(1-oxo-2-cyclo hexyleth-1-yl)-1,4-dihydroindeno[1,2-c]pyrazole

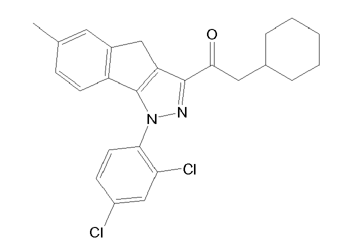

### 3.18a Preparation of N-methoxy-N-methyl-1-(2',4'-dichlorophenyl)-6-methyl-1,4-dihydroindeno[1,2-c]pyrazole-3-carboxamide

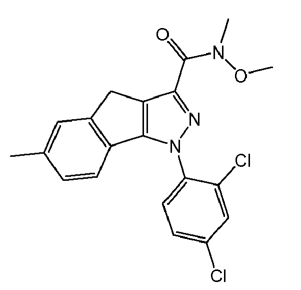

The same procedure of ex. 3.13a was repeated but that instead of the ester compound of ex. 1.3, ethyl 1-(2',4'-dichlorophenyl)-6-methyl-1,4-dihydroindeno[1,2-*c*]pyrazole-3-carboxylate, prepared according to the procedure reported by Mussinu J.M. et al. in Bioorg. Med. Chem. 2003, 11, 251-263, was used. Yield 44%. Rf=0.31 (petroleum ether/ethyl acetate 7.5/2.5 v/v). IR (nujol) (λ = cm⁻¹) 1684; ¹H-NMR (CDCl₃) δ 2.40 (s, 3H); 3.54 (bs, 3H); 3.80 (s, 2H); 3.82 (s, 3H); 6.90 (d, 1H, J = 7.8 Hz); 7.04 (bd, 1H, J = 7.7 Hz); 7.37 (bs, 1H); 7.44 (dd, 1H, J = 2.2 and 8.5 Hz); 7.56 (d, 1H, J = 8.5 Hz); 7. 65 (d, 1H, J = 2.2 Hz). Anal. calc. for C₂₀H₁₇Cl₂N₃O₂: C, 59.71; H, 4.26; Cl, 17.63; N, 10.45. Found: C, 59.67; H, 4.25; Cl, 17.62; N, 10.43.

### 3.18b Preparation of 1-(2',4'-dichlorophenyl)-6-methyl-3-(1-oxo-2-cyclohexyleth-1-yl)-1,4-dihydroindeno[1,2-c]pyrazole

The same procedure of ex. 3.13b was repeated but using the compound of ex. 3.18a instead of the carboxamide compound of ex. 3.13a. Yield 70%. Rf=0.62 (petroleum ether/diethyl ether 9:1 v/v). IR (nujol) (λ = cm⁻¹) 1686; ¹H-NMR (CDCl₃) δ 1.00-1.40 (m, 5H); 1.56-1.85 (m, 5H); 2.02-2.16 (m, 1H); 2.40 (s, 3H); 2.96 (d, 2H, J = 6.9 Hz); 3.82 (s, 2H); 6.89 (d, 1H, J = 7.8 Hz); 7.04 (bd, 1H, J = 7.5 Hz); 7.38 (bs, 1H); 7.47 (dd, 1H, J = 2.2 and 8.4 Hz); 7.57 (d, 1H, J = 8.4 Hz); 7.66 (d, 1H, J = 2.1 Hz). Anal. calc. for C₂₅H₂₄Cl₂N₂O: C, 68.34; H, 5.51; Cl, 16.14; N, 6.38. Found: C, 68.26; H, 5.50; Cl, 16.12; N, 6.37.

### EXAMPLE 3.19

### Preparation of 1-(2',4'-dichlorophenyl)-6-methyl-3-(1-hydroxy-2-cyclohexyleth-1-yl)-1,4-dihydroindeno[1,2-c]pyrazole

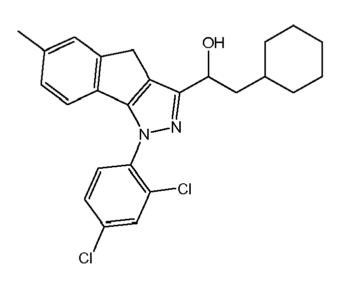

The same procedure of ex. 3.14 was repeated but using the keto compound of ex. 3.18 instead of the keto compound of ex. 3.13. Yield 99%. Rf=0.39 (petroleum ether/ethyl acetate 8/2 v/v). IR (nujol) (λ = cm⁻¹) 3325; ¹H-NMR (CDCl₃) δ 0.90-1.32 (m, 6H); 1.49-1.60 (m, 1H); 1.61-1.92 (m, 7H); 2.39 (s, 3H); 3.65 (d, 2H, J = 3.1 Hz); 4.99-5.05 (m, 1H); 6.88 (d, 1H, J = 7.8 Hz); 7.03 (bd, 1H, J = 7.7 Hz); 7.33 (bs, 1H); 7.40 (dd, 1H, J = 2.3 and 8.4 Hz); 7.50 (d, 1H, J = 8.4 Hz); 7.61 (d, 1H, J = 2.2 Hz). Anal. calc. for C₂₅H₂₆Cl₂N₂O: C, 68.03; H, 5.94; Cl, 16.09; N, 6.35. Found: C, 67.94; H, 5.93; Cl, 16.03; N, 6.34.

### EXAMPLE 3.20

### Preparation of N-piperidinyl-1-(2,4-dichlorophenyl)-6-methyl-1H-benzofuro[3,2-c]pyrazole-3-carboxamide

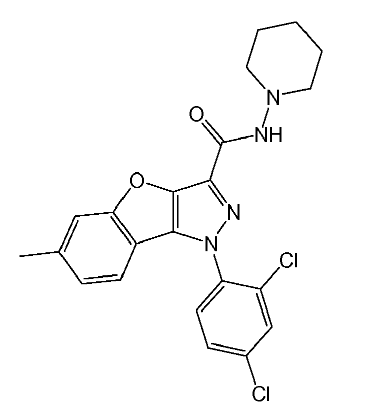

To the solution in methylene chloride (2 ml) of the acid obtained in ex. 2.9 (0.09 g; 0.25 mmol) HOBt (0.04 g; 0.30 mmol) and EDC (0.06 g, 0.30 mmol) were added. The resulting mixture was stirred at room temperature for 1 hour, then a solution of aminopiperidine (0.50 mmol) in 3 ml of CH₂Cl₂, was added. The resulting mixture was stirred at room temperature for 22 hours, then the solvent was removed under reduced pressure. The oily residue was purified by flash chromatography (oil ether/ethyl ether 6/4 v/v on silica gel). 0.09 g (81% yield) of a white solid corresponding to N-piperidinyl-1-(2,4-dichlorophenyl)-6-methyl-1*H*-benzofuro[3,2-c] pyrazole-3-carboxamide were recovered. Rf=0.10 (oil ether/ethyl ether 6/4 on silica gel); m.p.: 155-157°C; IR (nujol) (λ = cm⁻¹) 3434 (NH), 1648 (C=O) ; ¹H-NMR (CDCl₃) δ 0.86 (m, 2H), 1.25-1.98 (m, 4H), 2.49 (s, 3H) 2.92 (m, 4H), 7.09 (d, J=7.6 Hz, 1H), 7.29 (m, 2 H), 7.47 (m, 2H), 7.62 (m, 2H); ¹³C-NMR (CDCl₃) δ 21.9, 23.2, 25.2, 57.0, 113.9, 119.0, 124.4, 125.4, 128.2, 128.8, 129.2, 130.3, 130.5, 135.6, 136.0, 136.2, 137.8, 157.5, 162.9; API-ESI calc. for 443.33, found 443.10.

### EXAMPLE 3.21

### Preparation of N-pirrolidinyl-1-(2,4-dichlorophenyl)-6-methyl-1H-benzofuro[3,2-c]pyrazole-3-carboxamide

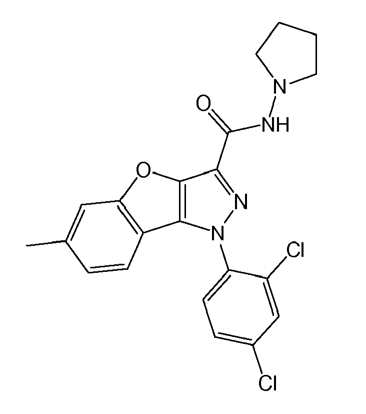

The same procedure described in ex. 3.20 was repeated, but the acid prepared in the ex. 2.9 was reacted with aminopirrolidine instead of aminopiperidine. Yield 94%. m.p. 90-92°C; IR (nujol) (λ = cm⁻¹) 3405, 1677; ¹H-NMR (CDCl₃) δ 1.78-1.94 (m, 5H); 2.50 (s,3H); 3.07 (m, 4H); 7.09 (d, 1H, J = 7.6 Hz); 7.27 (m, 1H); 7.48 (m, 2H); 7.63 (m, 2H). API-ESI calc. 429.30, found: 429.05.

### EXAMPLE 3.22

### Preparation of N-morpholin-4yl-1-(2,4-dichlorophenyl)-6-methyl-1H-benzofuro[3,2-c]pyrazole-3-carboxamide

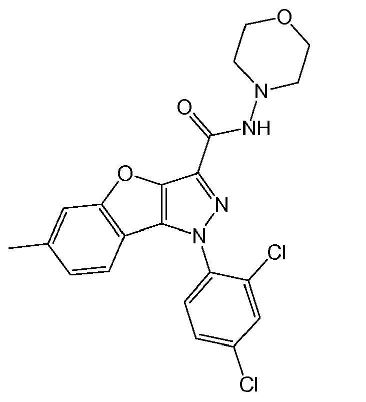

The same procedure described in ex. 3.20 was repeated but the acid prepared in ex. 2.9 was reacted with morpholin-4-ylamine instead of aminopiperidine. Yield 99%. m.p. 178-180°C; IR (nujol) (λ=cm⁻¹) 3430, 1673; ¹H-NMR (CDCl₃) δ 2.50 (s,3H); 3.02 (m, 4H); 3.88 (m, 4H); 7.10 (d, 1H, J = 7.8 Hz); 7.29 (d, 1H, J = 8.2 Hz); 7.46-7.51 (m, 2H); 7.59-7.67 (m, 2H). API-ESI calc. 445.30; found: 445.15.

### EXAMPLE 3.23

### Preparation of N-cyclohexyl-1-(2,4-dichlorophenyl)-6-methyl-1H-benzofuro[3,2-c]pyrazole-3-carboxamide

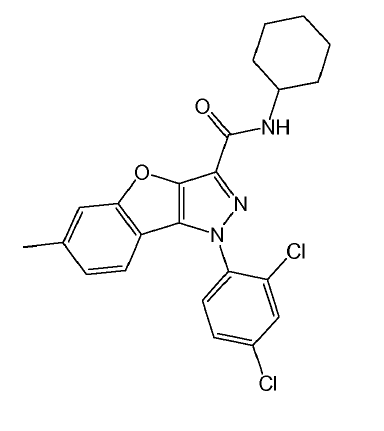

The same procedure described in ex. 3.20 was repeated but the acid prepared in ex. 2.9 was reacted with cyclohexylamine instead of aminopiperidine. Yield 99%. m.p. 140-142°C; IR (nujol) (λ = cm⁻¹) 3409, 1665; ¹H-NMR (CDCl₃) δ 0.79-2.08 (m, 10H); 2.50 (s,3H); 4.05 (m, 1H); 6.73 (d, 1H, J = 8.4 Hz); 7.09 (d, 1H, J = 7.8 Hz); 7.28 (d, 1H, J = 8.1 Hz); 7.47 (m, 2H); 7.58(m, 2H); ¹³C-NMR (CDCl₃) 21.9, 24.8, 25.6, 33.1, 47.9, 113.9, 119.1, 124.4, 128.3, 129.2, 129.9, 130.5, 135.6, 137.7, 159.3, 162.9. API-ESI calc. 442.34; Found: 442.20.

### EXAMPLE 3.24

### Preparation of N-2-isopropyl-5-methyl-cyclohexyl-1-(2,4-dichloro phenyl)-6-methyl-1H-benzofuro[3,2-c]pyrazole-3-carboxamide

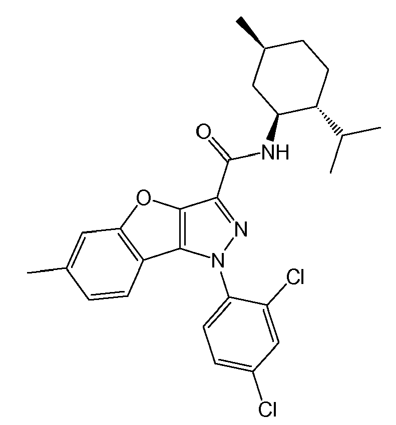

The same procedure described in ex. 3.20 was followed but the acid prepared in ex. 2.9 was reacted with 2-isopropyl-5-methyl-cyclohexylamine instead of aminopiperidine. Yield 96%. m.p. 70-72°C; IR (nujol) (λ = cm⁻¹) 3403, 1666; ¹H-NMR (CDCl₃) δ 0.86-0.99 (m, 9H); 1.14-1.32 (m, 4H); 1.50-1.74 (m, 3H); 2.03-2.14 (m, 2H), 2.50 (s,3H); 4.70 (m, 1H); 6.58 (d, 1H, J = 9.8 Hz); 7.09 (d, 1H, J = 8.0 Hz); 7.30 (d, 1H, J = 8.2 Hz); 7.47 (dd, 2H, J = 2.2, 10.6 Hz); 7.60-7.73 (m, 2H); ¹³C-NMR (CDCl₃) δ 16.2,21.2, 22.0, 22.2, 23.8, 26.9, 31.9, 34.5, 43.1, 48.2, 49.7, 113.9, 119.1, 124.4, 128.3, 129.2, 130.6, 135.6, 137.8, 159.5, 163.0. API-ESI calc. 498.44; found: 498.25.

### EXAMPLE 3.25

### Preparation of N-2,6,6-trimethyl-bicyclo[3.1.1]hept-3yl-1-(2,4-di chlorophenyl)-6-methyl-1H-benzofuro[3,2-c]pyrazole-3-carboxamide

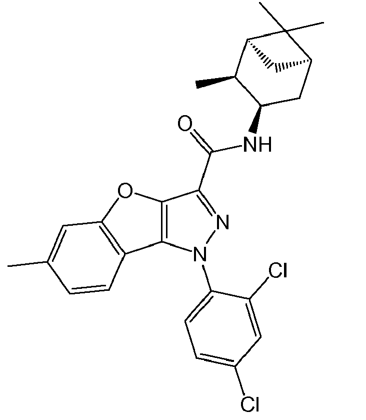

The same procedure described in ex. 3.20 was repeated but the acid prepared in ex. 2.9 was reacted with 2,6,6-trimethylbicyclo [3.1.1]hept-3ylamine instead of aminopiperidine. Yield 89%. m.p. 85-87°C; IR (nujol) (λ = cm⁻¹) 3407, 1666; ¹H-NMR (CDCl₃) δ 1.11 (s, 3H), 1.25(s, 6H), 1.69-2.00 (m, 5H), 2.49 (s, 3H), 2.70 (t, J=12 Hz, 2H), 4.53 (q, J=6.6 Hz, 1H), 6.76 (d, J=8.4 Hz, 1H), 7.09 (d, J=8.2 Hz, 1H), 7.29 (d, J=7.8 Hz, 1H), 7.45-7.49 (m, 2H), 7.60-7.66 (m, 2H). API-ESI calc. 496.43; found: 496.15.

### EXAMPLE 3.26

### Preparation of N-1,3,3-trimethyl-bicyclo[2.2.1]hept-2yl-1-(2,4-dichlorophenyl)-6-methyl-1H-benzofuro[3,2-c]pyrazole-3-carboxamide

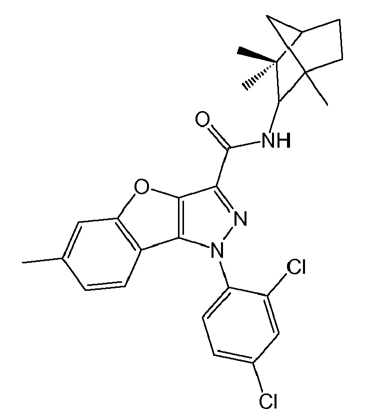

The same procedure described in ex. 3.20 was repeated but the acid prepared in ex. 2.9 was reacted with 1,3,3-trimethylbicyclo [2.2.1]hept-2yl-amine instead of aminopiperidine. Yield 96%. m.p. 84-86°C; IR (nujol) (λ = cm⁻¹) 3416, 1671; ¹H-NMR (CDCl₃) δ 0.82-1.80 (m, 16H), 2.50 (s, 3H), 3.88 (d, J= 9.4 Hz, 1H), 6.92 (d, J=10.8 Hz, 1H), 7.09 (d, J=8.0 Hz, 1H), 7.32 (d, J=8.2 Hz, 1H) 7.48 (m,2H), 7.60 (m, 2H); ¹³C-NMR (CDCl₃) δ 19.7, 21.2, 21.9, 26.0, 27.3, 30.9, 39.5, 42.7, 48.1, 48.6, 63.0, 113.9, 119.1, 124.4, 128.2, 129.1, 130.5, 135.4, 136.2, 137.7, 141.5, 146.7, 160.7, 162.9. API-ESI calc. 496.43; found: 496.35.

### EXAMPLE 3.27

### Preparation of N-1,7,7-trimethyl-bicyclo[2.2.1]hept-2yl-1-(2,4-dichlorophenyl)-6-methyl-1H-benzofuro[3,2-c]pyrazole-3-carboxamide

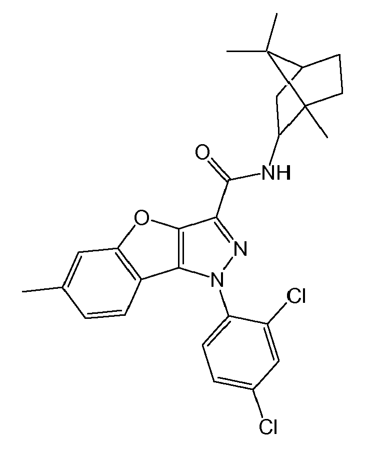

The same procedure described in ex. 3.20 was repeated but the acid prepared in ex. 2.9 was reacted with 1,7,7-trimethylbicyclo [2.2.1]hept-2ylamine instead of aminopiperidine. Yield 89%. m.p. 78-80°C; IR (nujol) (λ = cm⁻¹) 3414, 1669; ¹H-NMR (CDCl₃) δ 0.92 (s, 6H), 1.02 (s, 3H), 1.26-1.74 (m, 7H), 2.49 (s, 3H), 4.53 (m, 1H), 6.88 (d, J=9.2 Hz, 1H), 7.09 (d, J=8.00 Hz, 1H), 7.29 (m, 1H), 7.48 (dd, J=2.2, 11.0 Hz, 2H), 7.65 (m, 2H); ¹³C-NMR (CDCl₃) δ 13.8, 18.7, 19.9, 21.9, 28.1, 28.4, 37.5, 44.9, 48.2, 49.9, 53.6, 113.9, 119.1, 124.4, 128.3, 129.3, 130.5, 135.5, 136.1, 136.7, 137.7, 146.3, 160.2, 162.9. API-ESI calc. 496.43; found: 496.15.

### EXAMPLE 3.28

### Preparation of N-adamantan-1yl-1-(2,4-dichlorophenyl)-6-methyl-1H-benzofuro[3,2-c]pyrazole-3-carboxamide

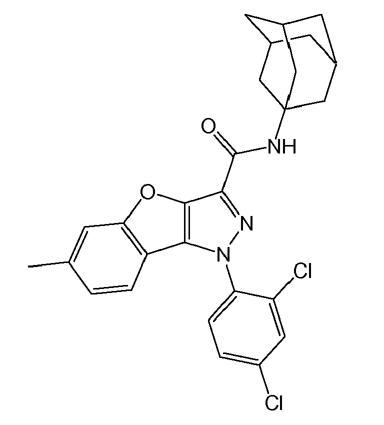

The same procedure described in ex. 3.20 was repeated but reacting the acid prepared in ex. 2.9 with adamantan-1-ylamine instead of aminopiperidine. Yield 81%. m.p. 210-212°C; IR (nujol) (λ = cm⁻¹) 3399, 1670; ¹H-NMR (CDCl₃) δ 1.68 (m, 7H), 2.18 (m, 8H), 2.49 (s, 3H), 6.58 (s, 1H), 7.08 (d, J=8.2 Hz, 1H), 7.29 (dd, J=7.8, 10.4 Hz, 1H), 7.46 (dd, J=2.2, 10.8 Hz, 2H), 7.62 (m, 2H). API-ESI calc. 494.41; found: 494.15.

### EXAMPLE 3.29

### Preparation of N-adamantan-2yl-1-(2,4-dichlorophenyl)-6-methyl-1H-benzofuro[3,2-c]pyrazole-3-carboxamide

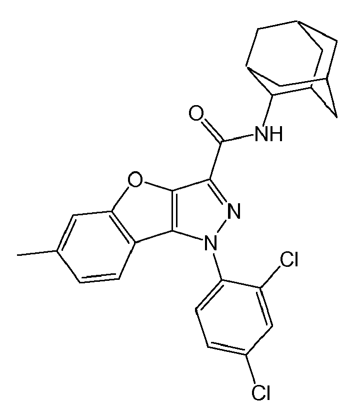

The same procedure described in ex. 3.20 was repeated but the acid prepared in ex. 2.9 was reacted with adamantan-2-ylamine instead of aminopiperidine. Yield 76%. m.p. 209-211°C; IR (nujol) (λ = cm⁻¹) 3417, 1664; ¹H-NMR (CDCl₃) δ 1.66-2.10 (m, 15 H), 2.50 (s, 3H), 4.30 (m, 1H), 7.09 (d, J=8.2 Hz, 1H), 7.20-7.33 (m, 3H), 7.48 (dd, J=2.2, 8.4 Hz, 2H), 7.64 (dd, J=2.2, 12,0 Hz, 1H). API-ESI calc. 494.41; found: 494.15.

### .EXAMPLE 4

### Affinity of the compounds of the invention towards the CB1 and CB2 cannabinoidergic receptors

The affinity of the compounds towards the CB1 and CB2 cannabinoidergic receptors was evaluated in vitro by radioreceptor binding studies using the following method.

The technique of the receptor binding allows to establish if, and with which affinity and specificity, a specific compound binds to a particular receptor. To evaluate the affinity of a specific compound to a particular receptor it is necessary to challenge in a particular tissue preparation wherein those specific receptors are present the compound to be tested with a radioactive labelled compound whose affinity for the same receptors is known. The ability of the compound under test to displace the radioactive compound from the receptor site gives an index of the affinity of the compound under test for that specific receptor. The amount of radioactivity present in the receptor-compound complex allows furthermore to stimate with great accuracy the amount of compound bound to the receptor. By said method it is therefore possible to stablish quickly the affinity of a new compound towards a specific receptor and thus to determine its pharmacological activity. With the same experimental protocol it is possible to evaluate the affinity of the compound towards other receptors and thus establish its specificity degree toward said other receptors.

The receptor binding technique, besides being used for the screening of new molecules with pharmacological activity, can give useful information on possible changes at receptor level, related for example to a prolonged exposure to drugs and/or to particular pathologies. In these conditions, indeed, changes in the amount of the receptors, or conformational changes can take place that alter the binding affinity of the agonists or antagonists, therefore affecting the functionality of the receptors themselves.

The experimentation has been carried out according to the guide lines of the European Community for the animal experimentation (EEC n. 86/609), by using laboratory animals (mice) lodged twenty per cage, under standard stabulation conditions (temperature 22±2°C, relative humidity 60%, artificial lighting with light/dark cycle of 12 hours). The food and water were ad libitum.

The procedure adopted, based on the use of the compound [³H]-CP-55,940 (New England Nuclear, Boston, MA, USA), requires the use of the mouse brain as biological tissue for the evaluation of the affinity towards the CB1 receptors and of the mouse spleen for the affinity assay for the CB2 receptors.

The animals were sacrificed by cervical dislocation and the complete brain (excluding the cerebellum) and the spleen were quickly dissected and kept in ice.

The tissue was homogeneized in 15 volumes (weight/-volume) of TME buffer (50 Mm Tris, 1 mM EDTA and 3 mM MgCl₂, pH 7.4) by an Ultra-Turrax and subsequently centrifuged for 10 minutes at 1086 x g in a centrifuge refrigerated at 4°C. The recovered supernatant was centrifuged at 45,000 x g for 30 minutes at 4°C by using a Beckman SW41 rotor and the obtained pellet was resuspended in 50 volumes of TME.

The thus obtained membranes (50-80 µg of proteins) were incubated in the presence of 1 nM of [³H]-CP55.940 for 1 hour at 30°C in a final volume of 0.5 ml of TME buffer containing 5 mg/ml of bovine serum albumin (BSA). The non specific binding was measured in the presence of CP55.940 at a 1 µM concentration.

All the experiments were carried out in polypropylene test tubes pretreated with Sigma-Cote (Sigma Chemical Co. Ltd., Poole, UK) for reducing non specific binding.

In order to determine the competitive inhibition binding curves, eight different concentrations of each compound were used. As reference compounds SR141716A was used for the CB1 receptors and SR144528 was used for the CB2 receptors.

Incubation was stopped by addition of TME buffer (at 4°C) containing 5 mg/ml of BSA, and subsequent filtration under vacuum by Whatman GFC filters pretreated with 0.5% of polyethylamine (PEI) and by using a filtering device (Brandell, Gaithersburg, MD, USA). The filters were washed 3 times with 5 ml of Tris HCl buffer (pH 7.4, 4°C) containing 1 mg/ml of BSA and separately placed in plastic vials containing 4 ml of scintillating liquid (Ultima Gold MV, Packard).

The radioactivity present in the filters was determined by a scintillator spectrophotometer (Tricarb^{®} 2100, Packard, Meridien, USA).
Protein determination was carried out by the Bradford method by using the protocol and the reactants supplied by Bio-Rad (Milano, Italy).
The experiments were carried out in triplicate and the results confirmed in five independent experiments.
The affinity of the compounds towards the CB1 and CB2 receptors has been expressed in Ki terms.

The Ki values, obtained with the compounds of the present invention in the test in vitro, are reported in Table 1. By comparison, in the Table, the affinity values of the reference compounds SR144528 and SR141716A (Rimonobant^{®}) are reported.

The Table shows that the compounds of the present invention have activity on the CB1 and/or CB2 receptors comparable with that of the reference compounds.

**TABLE 1**

| Example 4 : in vitro activity of the compounds of the invention on CB1 and CB2 receptors | | |
|---|---|---|
| Compound example | CB1 (brain) | CB2 (spleen) |
| | Ki (nM) | Ki (nM) |
| 3.1 | 20.0±5.0 | 23.3±1.6 |
| 3.3 | 21.7±3.0 | 325±38 |
| 3.4 | 2.6±0.17 | 19.6±1.5 |
| 3.8 | 2500±740 | 62.0±9.3 |
| 3.15 | 350±76 | 2.3±0.5 |
| 3.20 | 1788±330 | 12.9±1.1 |
| 3.21 | 2722±652 | 29.5±8.0 |
| 3.22 | 4064±64 | 37.7±6.3 |
| 3.23 | 1247+129 | 15.6±4.0 |
| 3.24 | 38.2±6.0 | 2.3±0.3 |
| 3.25 | 2398±629 | 3.7±0.3 |
| 3.26 | 257±7 | 2.5±0.3 |
| 3.27 | 469±64 | 3.5±0.6 |
| 3.28 | 363±92 | 28.0±6.6 |
| 3.29 | 537±132 | 17.5±0.9 |
| SR144528 (comp) | 70±10 | 0.28±0.04 |
| SR141716A (comp) | 1.8±0.075 | 514±30 |

### EXAMPLE 4.1 (COMPARISON)

### 4.1.a Preparation of 1-(2,4-dichlorophenyl)-6-methyl-1H-benzofuro [3,2-c]pyrazole-3-carboxamide

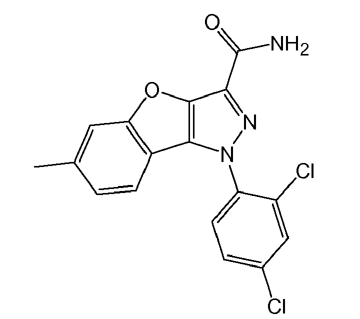

To a solution of the acid obtained in ex. 2.9 (0.09 g; 0.25 mmol) in methylene chloride (2 ml) HOBt (0.04 g; 0.30 mmol) and EDC (0.06g, 0.30 mmol) were added. The mixture was kept under stirring at room temperature for 1 hour, then 10 eq of NH₄OH (30% w in water) was added dropwise. The resulting mixture was stirred at room temperature for 30 minutes. The organic solution was washed with brine, dried over Na₂SO₄, then concentrated under reduced pressure. A crude product was obtained, that was purified by flash chromatography (oil ether/ethyl ether 10/3 v/v on silica gel) to give 1-(2,4-dichlorophenyl)-6-methyl-1*H*-benzofuro[3,2-c]pyrazole-3-carboxamide. Yield 90%. M.p.: 185-186°C; IR (nujol) (λ = cm⁻¹) 3548 (NH₂), 3287 (NH₂), 1648 (C=O); ¹H-NMR (CDCl₃) δ 2.34 (s, 3H) 6.93 (d, 1H), 7.17 (s, 1H), 7.37 (m, 1 H), 7.50 (m, 2H), 7.69 (m, 2H), 7.77 (m, 1H), 8.31 (br s, NH₂); ¹³C-NMR (CDCl₃) δ 21.6, 106.9, 111.6, 116.7, 120.8, 122.7, 123.6, 127.5, 131.0, 132.9, 133.2, 134.9, 141.3, 141.7, 145.0, 156.4, 161.6; API-ESI calc. for 360.19; found 360.05.

### 4.1.b Determination of the affinity of 1-(2,4-dichlorophenyl)-6-methyl-1H-benzofuro[3,2-c]pyrazole-3-carboxamide for CB1 and CB2 receptors

The affinity of 1-(2,4-dichlorophenyl)-6-methyl-1H-benzofuro[3,2-c]pyrazole-3-carboxamide for CB1 and CB2 receptors was determined according to the method reported in ex. 4.
It was found that said compound did not show any significant affinity towards both CB1 and CB2 receptors, as the Ki values for this compound were higher than 3.000 nM for both CB1 and CB2 receptors.
The example shows that the presence of specific substituents at the 3 position of the pyrazole ring in the condensed tricyclic structure, is critical for providing affinity, as in the case of the compounds of formula (I), for CB1 and/or CB2 receptors.

### EXAMPLE 5

### Preparation of an emulsion according to the invention

2.20 grams of Solutol^{®}HS15 (Basf) were solubilized in 20,00 grams of physiological solution. The obtained aqueous solution was heated to 70°C. By keeping under a turbulent motion by a Politron ultraturrax turboemulsifier (10,000 rpm with 7 mm probe), 2.80 g of an oily solution previously heated to 70°C were dropwise added. Said oily solution has been obtained by mixing 0.25 grams of the compound obtained in example 3.1 with 0.25 g of ethanol and 2.30 g of Miglyol^{®}810N.
At the end of the dropwise addition to the aqueous solution of Solutol^{®}HS15, agitation was prosecuted for further 15 minutes by means of the with the Politron ultraturrax turboemulsifier, obtaining at the end an emulsion in the form of a white liquid homogeneous phase. Said emulsion was poured into a glass vessel at 4°C, and it was therein kept for 30 minutes. The emulsion was then stored at room temperature for five days without noticing phase separation.

The emulsion composition (% by weight) is the following:

| | |
|---|---|
| Ethanol | 1.00 |
| Compound ex. 3.1 | 1.00 |
| Miglyol^{®}810N | 9.20 |
| Solutol^{®}HS15 | 8.80 |
| Physiological solution | 80.00 |

### EXAMPLE 6

### Preparation of an emulsion according to the invention

The same procedure described in example 5 was repeated but the amount of Solutol^{®}HS15 was reduced to 1.00 g and the oily solution (2.80 g) was substituted with 1.00 g of a solution formed of: 0.01 g of the compound obtained in example 3.3, 0.02 g of ethanol, 0.45 g of Miglyol^{®}810N, 0.52 g of Imwitor^{®}308. An emulsion in the form of a white liquid homogeneous phase was obtained. Said emulsion after a storage period at room temperature of five days, did not show any phase separation.

The emulsion composition (% by weight) is the following:

| | |
|---|---|
| Ethanol | 0.10 |
| Compound ex. 3.3 | 0.05 |
| Miglyol^{®}810N | 2.04 |
| Imwitor^{®}308 | 2.36 |
| Solutol^{®}HS15 | 4.55 |
| Physiological solution | 90.90 |

### EXAMPLE 7

### Preparation of particles of polylactate-polyglycolate (PLA-PLGA) according to the invention

10 mg of the compound obtained in example 3.4 and 100 mg of copolymer PLA-PLGA 50:50 having an average molecular weight 40,000-75,000 (Sigma Aldrich), were solubilized in 4 ml of dichloromethane. The obtained organic solution was emulsified with 8 ml of an aqueous solution at 5% by weight of polyvinyl alcohol (Sigma Aldrich), by treatment for a period of 30 minutes with an ultraturrax Politron turboemulsifier (10,000 rpm with a 7 mm probe).

At the end the dichloromethane was removed from the emulsion at 50°C in a rotating evaporator. An aqueous dispersion of PLA-PLGA particles containing the compound of formula 3.4 was thus obtained. The aqueous dispersion was subjected to three washing cycles by centrifugation in centrifuge AMI-CON test tubes, having membrane with 100,000 MWCO cut off. Each washing cycle was carried out at 4,000 rpm for 20 minutes, by adding each time 15 ml of distilled water in the upper section containing the particles.

At the end of the washing cycles the particle aqueous dispersion was lyophilized under the following conditions: temperature -40°C, pressure 5x10⁻² mbar, time 24 hours.

The obtained particles have been characterized both by transmission electronic microscopy (TEM) and by Photon Correlation Spettroscopy (PCS). The average diameters determined for the particles resulted of 140±20 nm by TEM and 176±13 nm by PCS.

The content of the compound of ex. 3.4 incorporated into the particles was determined by solubilizing a known amount of the particles in dichloromethane and then analyzing by UV/Visible spectrophotometer the obtained organic solution against a reference dichloromethane solution of the compound of ex. 3.4. The amount of compound in the lyophilized sample of nano-particles was, as percentage by weight, equal to 40% of that used in the preparation. Therefore the PLA-PLGA particles contain 4% of the compound of ex. 3.4 (4 mg in 100 mg of PLA-PLGA).

The percentage by weight of the compound of example 3.4 on the particle total is 3.84%.

## Claims

1. Condensed tricyclic compounds, containing one phenyl ring and one pyrazole ring joined by a central ring comprising from five up to eigth atoms, showing affinity for the CB1 and/or CB2 receptors, with central nervous system and/or peripheral activity, having formula (I): 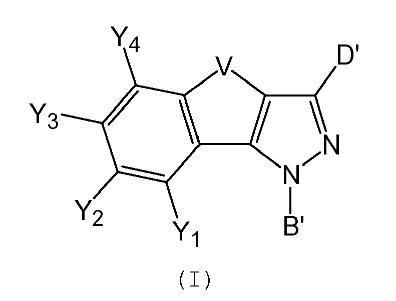 wherein:
B' is selected from phenyl, arylalkyl, arylalkenyl, heteroaryl, heteroarylalkyl, or a bivalent C₁-C₁₀ aliphatic chain, linear or branched when possibile, wherein the chain end not linked to the nitrogen atom is linked to W^{I}, selected from hydrogen, halogen, isothiocyanate, CN, OH, OCH₃, NH₂, SO₂NH₂ or -CH=CH₂,
Y₁, Y₂, Y₃ and Y₄, equal to or different from each other, are selected from hydrogen, halogen, C₁-C₇ alkyl, C₁-C₇ alkylthio, C₁-C₇ alkoxy, C₁-C₇ haloalkyl, C₁-C₇ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₇ alkyl chain,
V is selected from:
A1: -(CH₂)ₜ-,
A2: -(CH₂)ᵣ-O-(CH₂)ₛ-
A3: - (CH₂)ᵣ-S(O)ₚ-(CH₂)ₛ-
wherein
t is an integer equal to 1, 2 or 3,
p is an integer equal to 0, 1 or 2,
r and s, equal to or different from each other, are integers equal to 0, 1 or 2 with the proviso that r+s is equal to 0, 1, 2 or 3,
when B' has the meaning of bivalent C₁-C₁₀ aliphatic chain, linear or branched when possibile, wherein the chain end not linked to the nitrogen atom is linked to W^{I} as defined above, D' has the following meanings:
D1: -(CH₂)-O-(CH₂)₂-(Z')ᵥ-R"
wherein z is an integer equal to 1 or 2, v is an integer equal to 0 or 1, Z' is a bivalent C₁-C₈ aliphatic chain, linear o branched when possible, R" is selected from C₃-C₁₅ cycloalkyl, saturated or unsaturated heterocycle, aryl or heteroaryl,
D2: -C(O)-(Z')ᵥ-R"
wherein v, Z' and R" are as defined above,
D3: -CH(OH)-(Z')ᵥ-R"
wherein v, Z' and R" are as defined above,
D4: -C(O)-NH-(Z')ᵥ-T'
wherein v and Z' are as defined above and T' is a group selected from:
- C₁-C₈ alkyl, C₁-C₇ haloalkyl with the proviso that in formula D4 v is equal to 0,
- C₃-C₁₅ cycloalkyl,
- monocyclic aryl or monocyclic heteroaryl,
- NR₁R₂, wherein R₁ and R₂, equal to or different from each other, have the following meanings: hydrogen, C₁-C₇ alkyl, C₁-C₇ haloalkyl, heteroaryl, heteroarylalkyl, aryl, arylalkyl or arylalkenyl, or R₁ and R₂ with the nitrogen atom form a saturated or unsaturated heterocycle having 5 to 10 atoms,
- C₃-C₁₅ heterocycloalkyl, containing one or more heteroatoms, equal to or different from each other, selected from N, O, S, with the proviso that Z' is linked to one carbon atom of the heterocycloalkyl ring,
when B' is selected from phenyl, arylalkyl, arylalkenyl, heteroaryl, heteroarylalkyl, D' has the following meanings:
D'2: -C(O)-Z'-R"
wherein Z' and R" are as defined,
D'3: -CH(OH)-Z'-R"
wherein Z' and R" are as defined,
D'4: -C(O)-NH-Z'-T'
Z' being as defined, and excluding for T' C₁-C₈ alkyl, C₁-C₇ haloalkyl and, when in D'4 Z'= -CH₂-, T' is not: 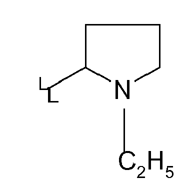
D"2: -C(O)-R", with the proviso that V=A2,
D"3: -CH(OH)-R", with the proviso that V=A2,
D"4: -C(O)-NH- T', with the proviso that V is selected from the following groups: -O- or -CH₂-O-.

2. Isomeric forms, both geometrical and stereoisomers, and their corresponding mixtures, of the compounds according to claim 1.

3. Compounds according to claims 1-2, wherein B' is phenyl, or arylalkyl, arylalkenyl, heteroaryl, heteroarylalkyl, and is optionally substituted with one or more groups, equal to or different from each other, selected from halogen, C₁-C₇ alkyl, C₁-C₇ alkylthio, C₁-C₇ alkoxy, C₁-C₇ haloalkyl, C₁-C₇ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₇ alkyl chain.

4. Compounds according to claims 1-3, wherein Y₁, Y₂, Y₃ or Y₄ are phenyl, cycloalkyl, saturated or unsaturated heterocycle, heteroaryl, said phenyl, cycloalkyl, saturated or unsaturated heterocycle and heteroaryl optionally substituted with one or more groups, equal to or different from each other, selected from halogen, C₁-C₇ alkyl, C₁-C₇ alkylthio, C₁-C₇ alkoxy, C₁-C₇ haloalkyl, C₁-C₇ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₇ alkyl chain.

5. Compounds according to claims 1-4, wherein R" is substituted with one or more groups, equal to or different from each other, selected from SO₂NH₂, halogen, C₁-C₇ alkyl, C₁-C₇ haloalkyl, C₁-C₇ haloalkoxy, C₁-C₇ alkylthio or C₁-C₇ alkoxy.

6. Compounds according to claims 1-5, wherein T' is substituted with one or more groups, equal to or different from each other, selected from halogen, cyano, nitro, C₁-C₇ alkyl, C₁-C₇ haloalkyl, C₁-C₇ haloalkoxy, C₁-C₇ alkylthio, C₁-C₇ alkoxy, SO₂NH₂, isothiocyanate, phenyl, benzyl, amino optionally mono- or bisubstituted with a C₁-C₇ alkyl chain, the phenyl and benzyl substituents being optionally substituted with one or more groups, equal to or different from each other, selected from halogen, C₁-C₇ alkyl, C₁-C₇ alkylthio, C₁-C₇ alkoxy, C₁-C₇ haloalkyl, C₁-C₇ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₇ alkyl chain.

7. Compounds according to claims 1-6, wherein R₁ and R₂ of T' are aromatic rings selected from heteroaryl, heteroarylalkyl, aryl, arylalkyl or arylalkenyl, or R₁ and R₂ with the nitrogen atom form an heterocycle, the aromatic rings or the heterocycle are substituted with one or more groups equal to or different from each other, selected from halogen, cyano, nitro, C₁-C₇ alkyl, C₁-C₇ haloalkyl, C₁-C₇ haloalkoxy, C₁-C₇ alkylthio, C₁-C₇ alkoxy, SO₂NH₂, isothiocyanate, phenyl, benzyl, amino optionally mono- or bisubstituted with a C₁-C₇ alkyl chain, the phenyl and benzyl substituents being optionally substituted with one or more groups, equal to or different from each other, selected from halogen, C₁-C₇ alkyl, C₁-C₇ alkylthio, C₁-C₇ alkoxy, C₁-C₇ haloalkyl, C₁-C₇ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₇ alkyl chain.

8. Compounds according to claims 1-7, wherein:
B' is selected from phenyl, benzyl, monocyclic heteroaryl, monocyclic heteroarylalkyl, or a bivalent C₁-C₁₀ aliphatic chain, linear or branched when possibile,
wherein the chain end not linked to the nitrogen atom is linked to W^{I}, selected from hydrogen, halogen, isothiocyanate, CN, OH, OCH₃, NH₂, SO₂NH₂ or -CH=CH₂, said phenyl, benzyl, monocyclic heteroaryl and monocyclic heteroarylalkyl being optionally substituted with one or more groups, equal to or different from each other, selected from halogen, C₁-C₇ alkyl, C₁-C₇ alkylthio, C₁-C₇ alkoxy, C₁-C₇ haloalkyl, C₁-C₇ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₇ alkyl chain,
Y₁, Y₂, Y₃ and Y₄, equal to or different from each other, are as defined,
V is selected between A1 and A2,
when B' has the meaning of bivalent C₁-C₁₀ aliphatic chain, linear or branched when possibile, wherein the chain end not linked to the nitrogen atom is linked to W^{I} as defined above, D' is as defined,
when B' is selected from phenyl, benzyl, monocyclic heteroaryl or monocyclic heteroarylalkyl, D' has the meanings of D'2, D'4, D"2 or D"4.

9. Compounds according to claims 1-8, wherein:
B' is selected from phenyl, benzyl, thiophene or a bivalent C₄-C₁₀ aliphatic chain, linear or branched when possibile, wherein the chain end not linked to the nitrogen atom is linked to W^{I} selected from hydrogen, halogen, isothiocyanate, CN, OH, OCH₃, NH₂, SO₂NH₂ or -CH=CH₂, said phenyl, benzyl and thiophene being optionally substituted with one or more groups, equal to or different from each other, selected from halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₃ alkyl chain,
Y₁, Y₂, Y₃ and Y₄, equal to or different from each other, are as defined above,
V is a group selected between A1 and A2,
when B' is a bivalent C₄-C₁₀ aliphatic chain, linear or branched when possibile, wherein the chain end not linked to the nitrogen atom is linked to W^{I} as defined above, D' is as defined,
when B' is selected from phenyl, benzyl or thiophene, D' has the meanings of D'2, D'4, D"2 or D"4.

10. Compounds according to claims 1-9, wherein:
B' is selected from phenyl, benzyl, thiophene, a bivalent C₄-C₁₀ aliphatic chain, linear or branched, wherein the chain end not linked to the nitrogen atom is linked to W^{I}, selected from hydrogen, halogen, OH, OCH₃, NH₂, SO₂NH₂, said phenyl, benzyl and thiophene are optionally substituted with one or more groups, equal to or different from each other, selected from halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₃ alkyl chain,
Y₁, Y₂, Y₃ and Y₄, equal to or different from each other, are selected from hydrogen, halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₃ alkyl chain, said phenyl, cycloalkyl, saturated or unsaturated heterocycle and heteroaryl being optionally substituted with one or more groups, equal to or different from each other, selected from halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₇ alkyl chain,
V represents a group selected from A1 or A2,
when B' is a bivalent C₄-C₁₀ aliphatic chain, linear or branched when possibile, wherein the chain end not linked to the nitrogen atom is linked to W^{I} as defined above, D' is as defined,
when B' is selected from phenyl, benzyl or thiophene, D' has the meanings of D'2, D'4, D"2 or D"4, with the proviso that:
Z' is -CH₂- or -CH(CH₃)-,
R" is selected from C₃-C₁₅ cycloalkyl, saturated or unsaturated heterocycle, aryl, or heteroaryl, said C₃-C₁₅ cycloalkyl, saturated or unsaturated heterocycle, aryl, or heteroaryl being optionally substituted with one or more groups, equal to or different from each other, selected from SO₂NH₂, halogen, C₁-C₃ alkyl, C₁-C₃ haloalkyl, C₁-C₃ haloalkoxy, C₁-C₃ alkylthio, C₁-C₃ alkoxy,
T' is a group selected from:
- C₃-Cₗ₅ cycloalkyl,
- monocyclic aryl with the proviso that one of the following two alternative conditions is satisfied:
V different from A1, or independently from V, B' different from phenyl, benzyl or thiophene,
- a group NR₁R₂, wherein R₁ and R₂, equal to or different from each other, form with the nitrogen atom one saturated or unsaturated heterocycle having from 5 to 10 atoms,
- C₃-C₁₅ heterocycloalkyl, wherein Z' is linked to one carbon atom of the heterocycloalkyl.

11. Compounds according to claims 1-10, wherein T' is substituted with one or more groups, equal to or different from each other, selected from halogen, C₁-C₃ alkyl, C₁-C₃ haloalkyl, C₁-C₃ haloalkoxy, C₁-C₃ alkylthio, C₁-C₃ alkoxy, SO₂NH₂, phenyl, benzyl, amino optionally mono- or bisubstituted with a C₁-C₃ alkyl chain, said phenyl and benzyl being optionally substituted with one or more groups, equal to or different from each other, selected from halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂, isothiocyanate, phenyl, cycloalkyl, saturated or unsaturated heterocycle, heteroaryl, amino optionally mono- or bisubstituted with a C₁-C₃ alkyl chain.

12. Compounds according to claims 1-11, selected from the following: 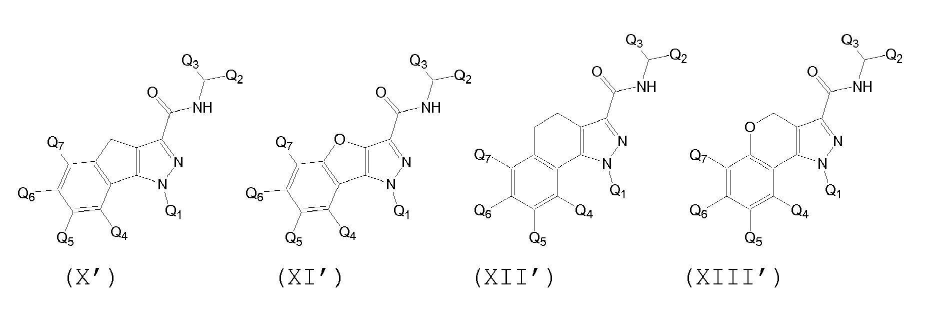 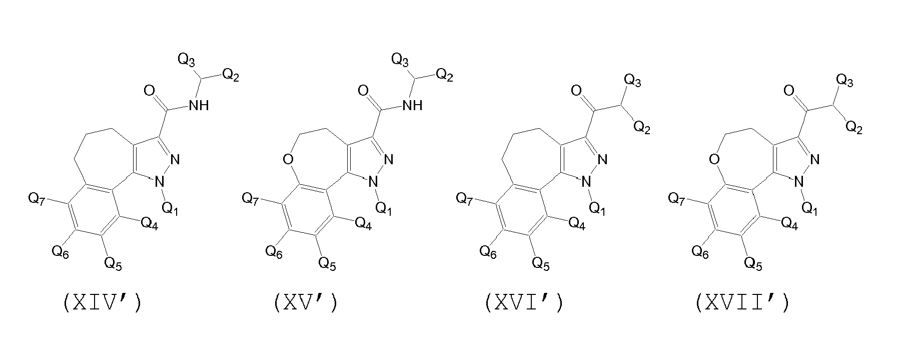 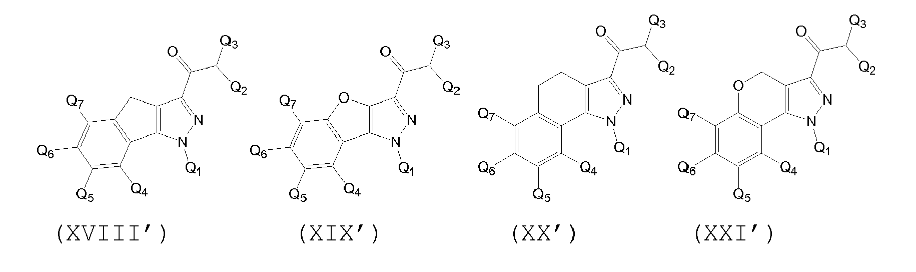 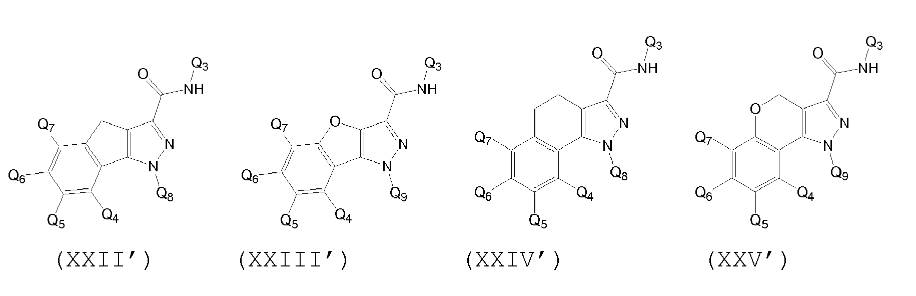 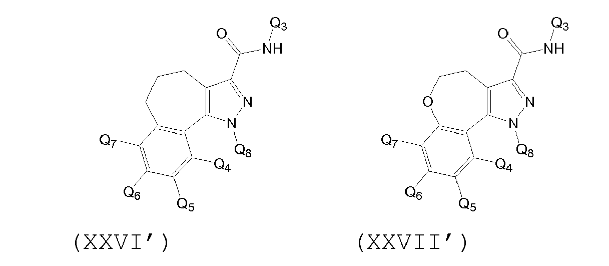 wherein:
Q₁ has the meaning of:
Q₁A: bivalent C₄-C₁₀ aliphatic chain, linear or branched when possibile, wherein the chain end not linked to the nitrogen atom is linked to W^{IV}, W^{IV} being selected from hydrogen, halogen, OH, OCH₃, NH₂ or SO₂NH₂,
Q₁B, selected from phenyl or benzyl, optionally substituted with one or more groups, equal to or different from each other, selected from halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂, isothiocyanate, phenyl, cycloalkyl, saturated or unsaturated heterocycle, heteroaryl, or amino optionally mono- or bistubstituted with one C₁-C₃ alkyl chain,
Q₈ has the meanings of Q₁A as defined above,
Q₉ has the meanings of Q₁ as defined above,
Q₂ is selected from hydrogen or methyl,
Q₄, Q₅, Q₆, Q₇, equal to or different from each other, are selected from hydrogen, halogen, C₁-C₃ alkyl, C₁-C₃ alkoxy, C₁-C₃ haloalkyl, cyano, SO₂NH₂, isothiocyanate, phenyl, cycloalkyl, saturated or unsaturated heterocycle, thiophene, amino optionally mono- or bisubstituted with a C₁-C₃ alkyl chain, said phenyl, cycloalkyl, saturated or unsaturated heterocycle and thiophene being optionally substituted with one or more groups, equal to or different frm each other, selected from halogen, C₁-C₃ alkyl, C₁-C₃ alkoxy, C₁-C₃ haloalkyl, cyano, SO₂NH₂, isothiocyanate, amino optionally mono- or bisubstituted with a C₁-C₃ alkyl chain,
Q₃ is selected from the following:

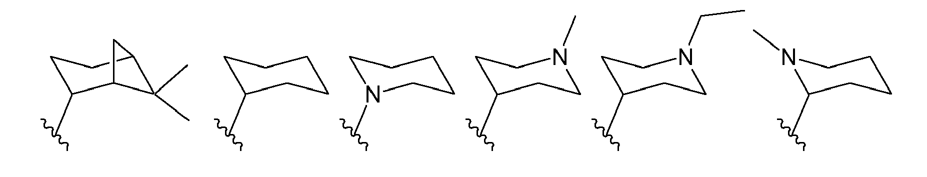

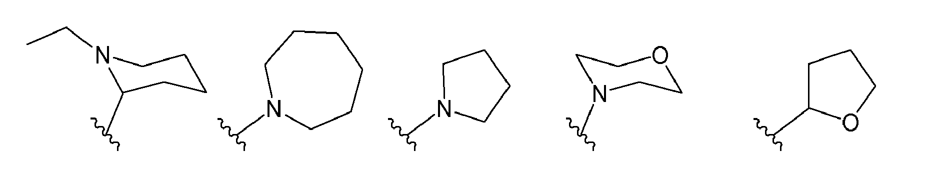

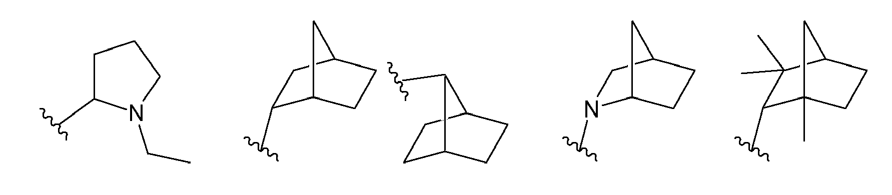

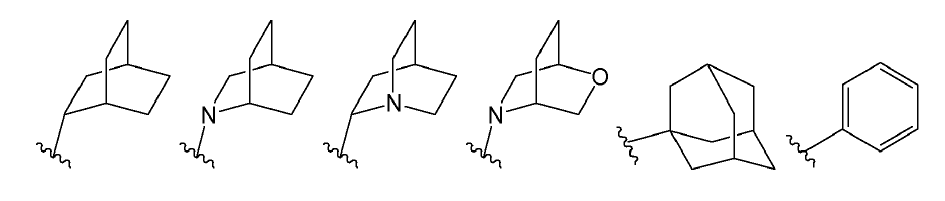

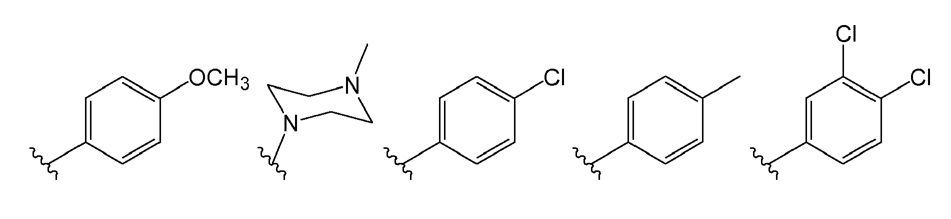

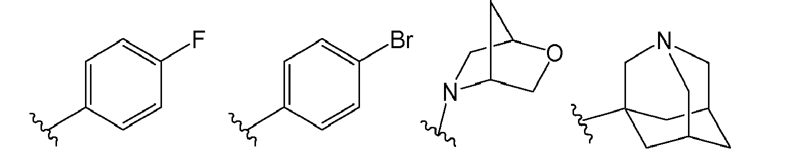

13. Compounds according to claims 1-12, selected from the following:

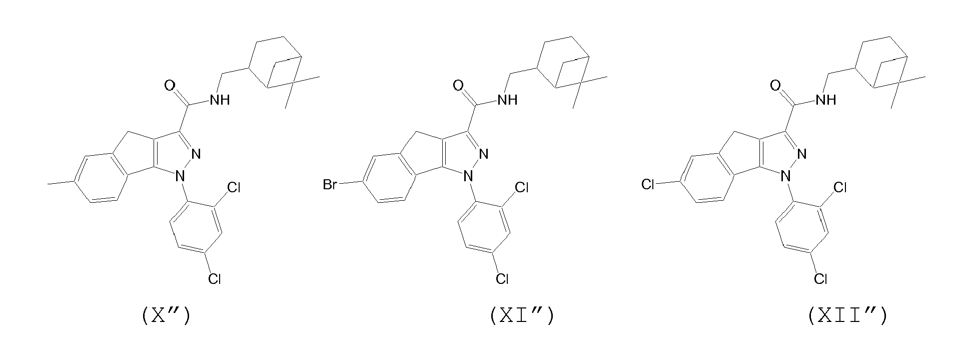

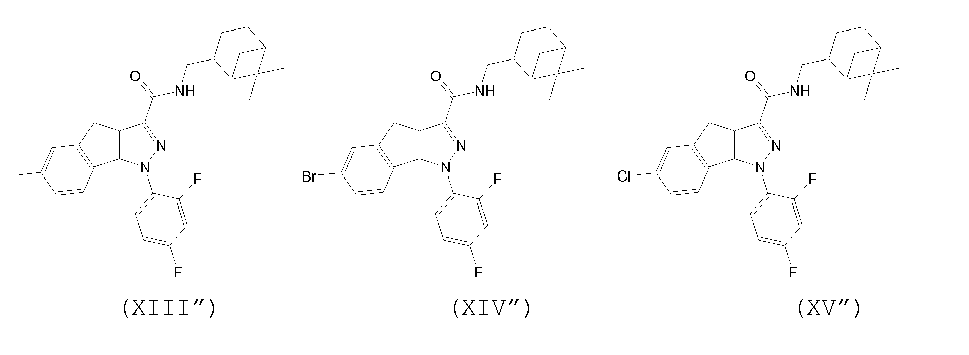

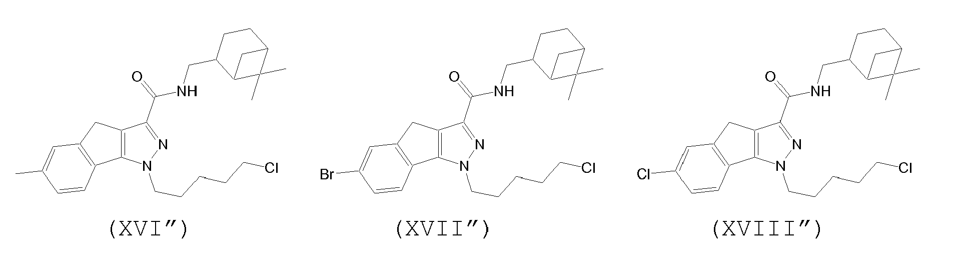

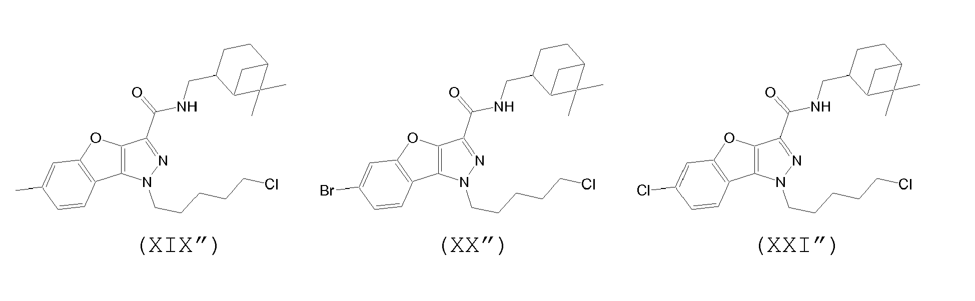

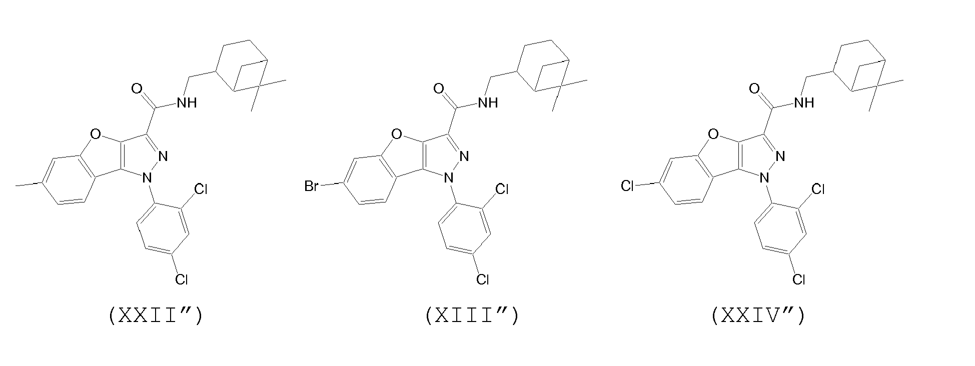

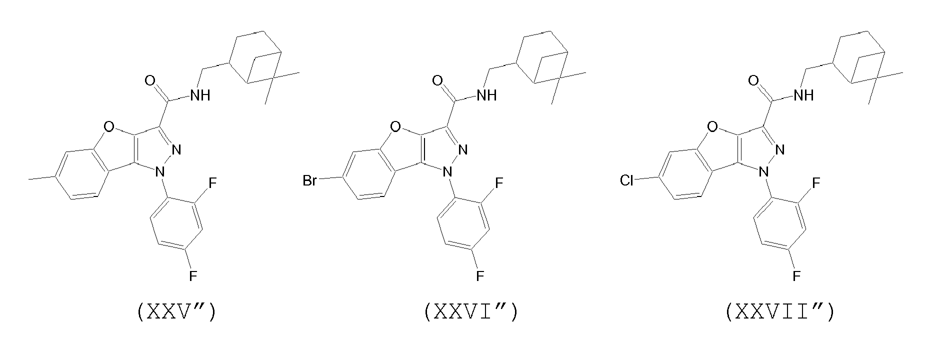

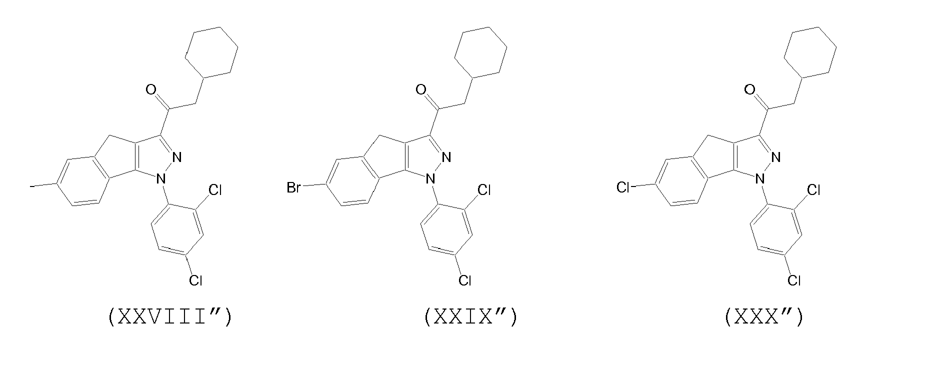

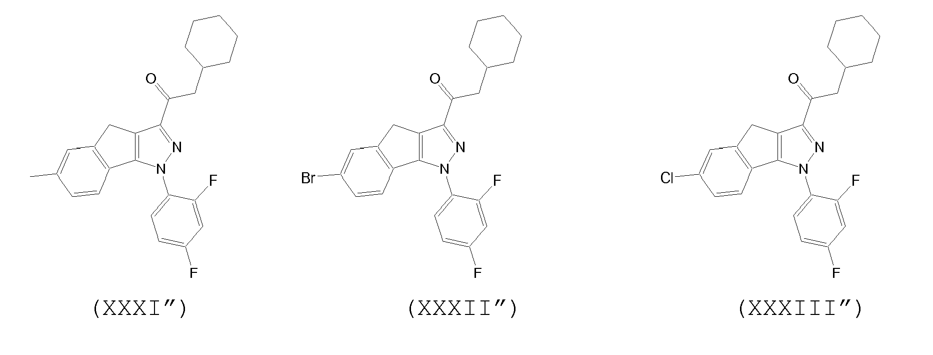

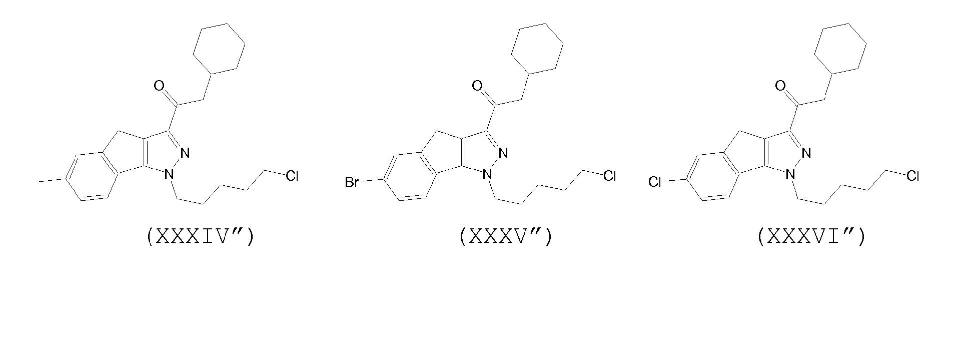

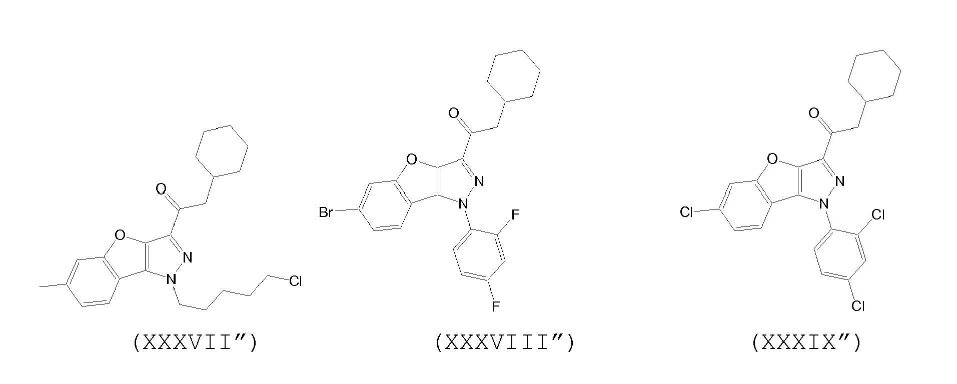

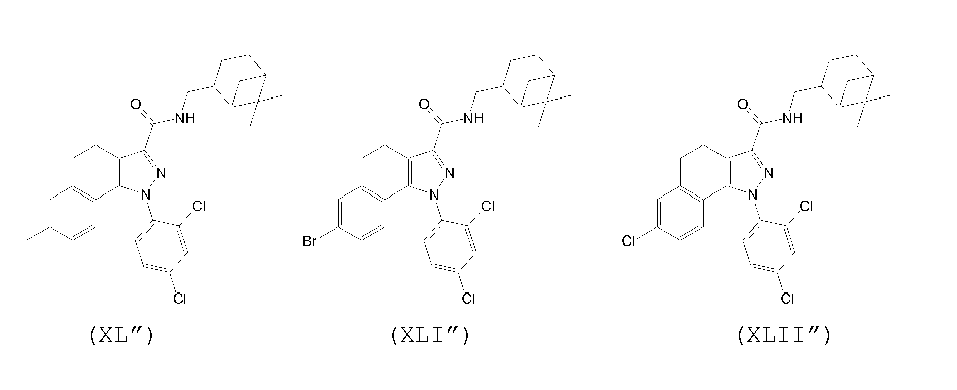

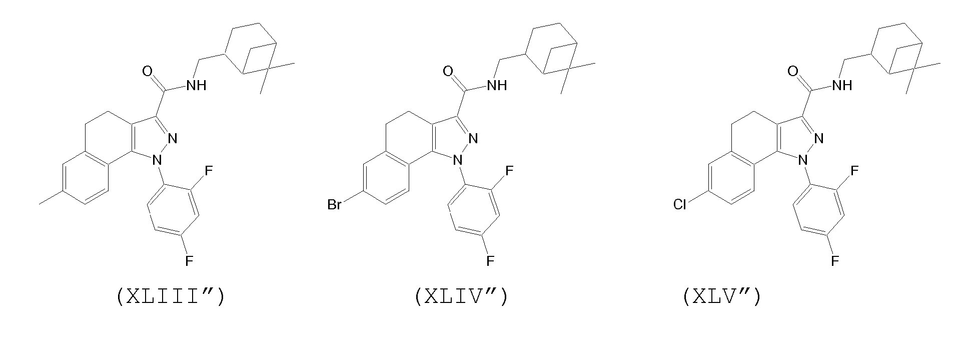

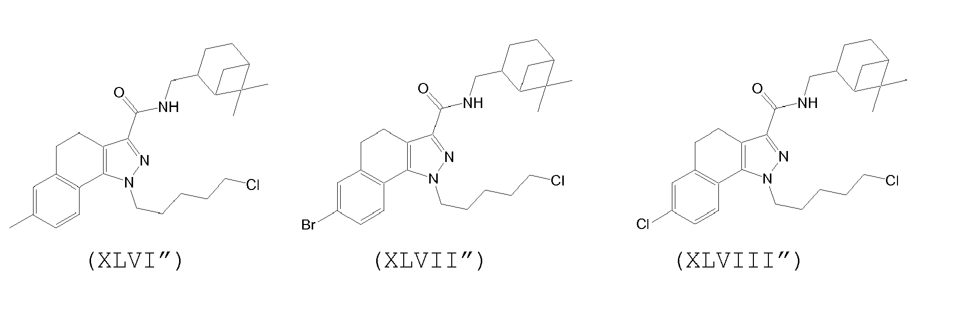

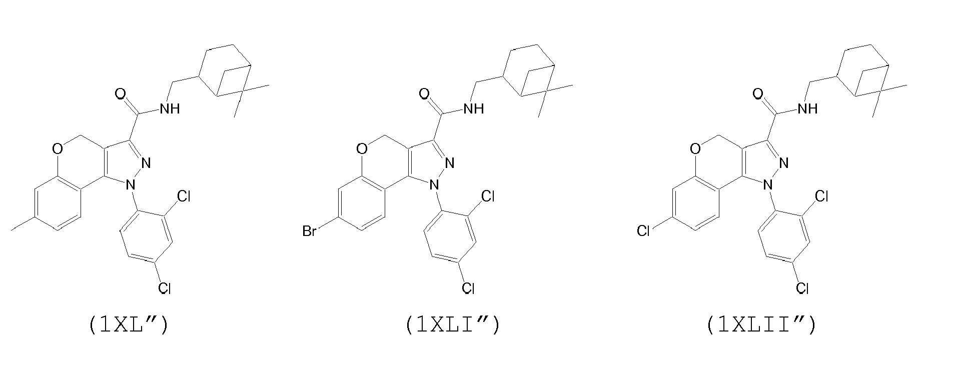

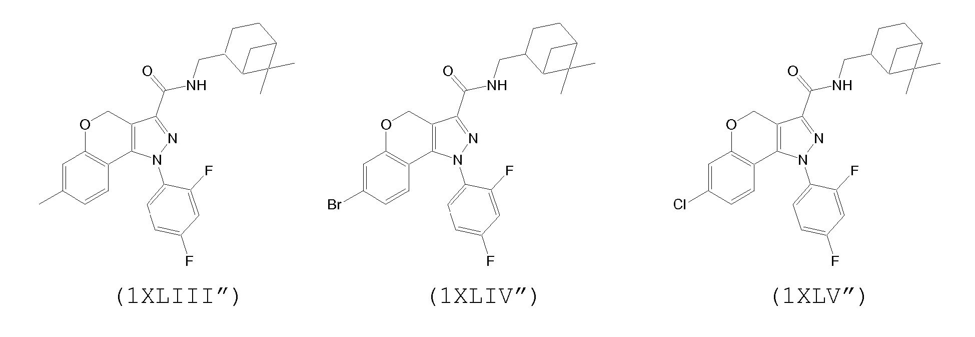

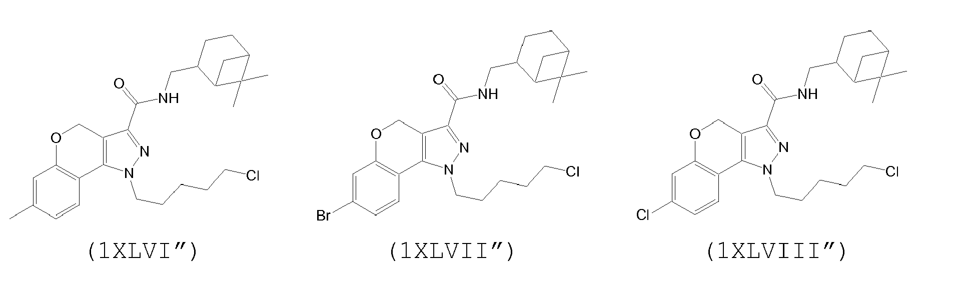

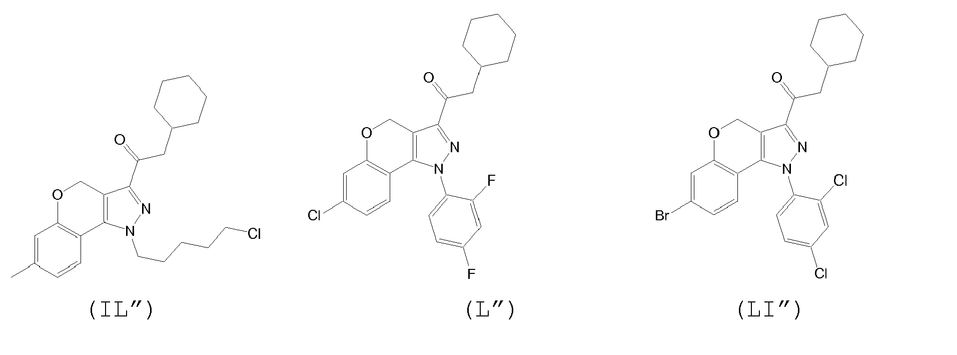

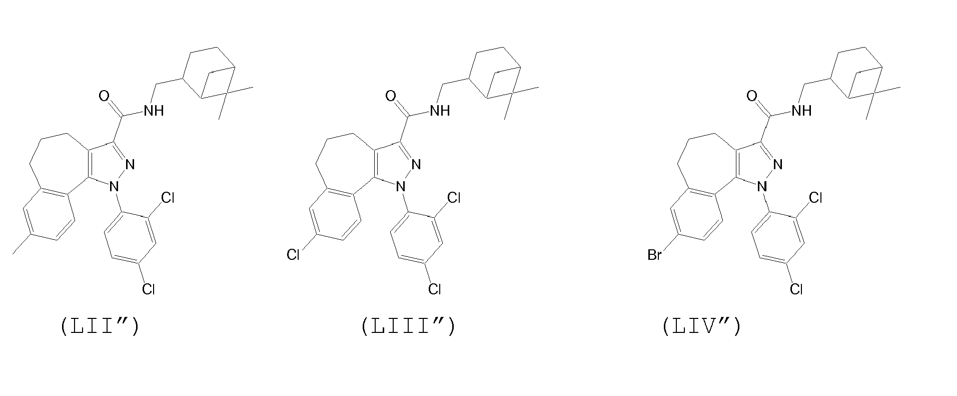

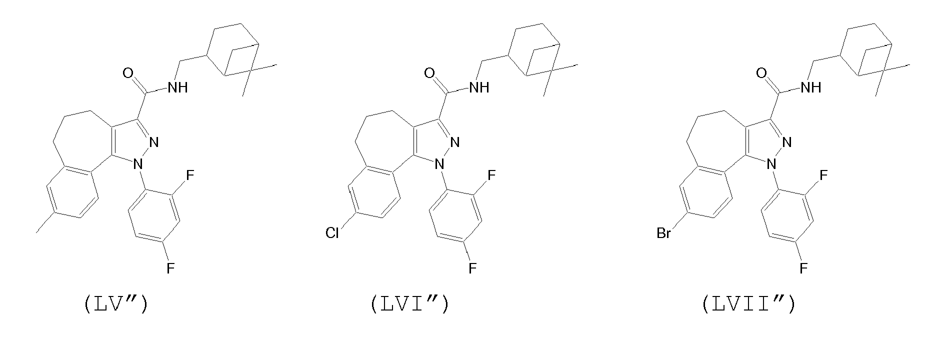

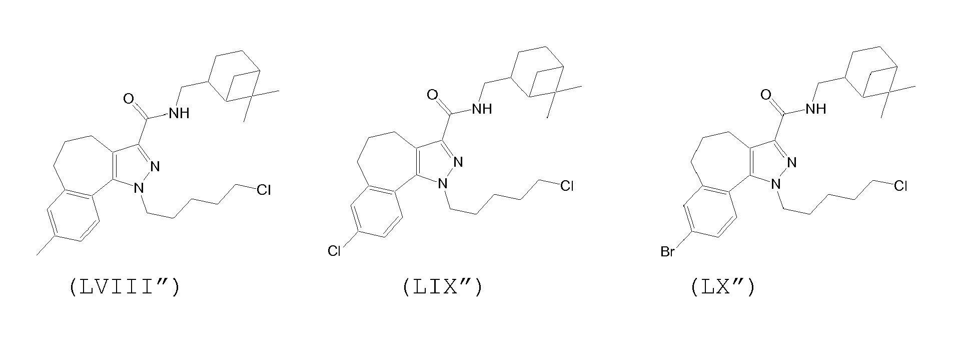

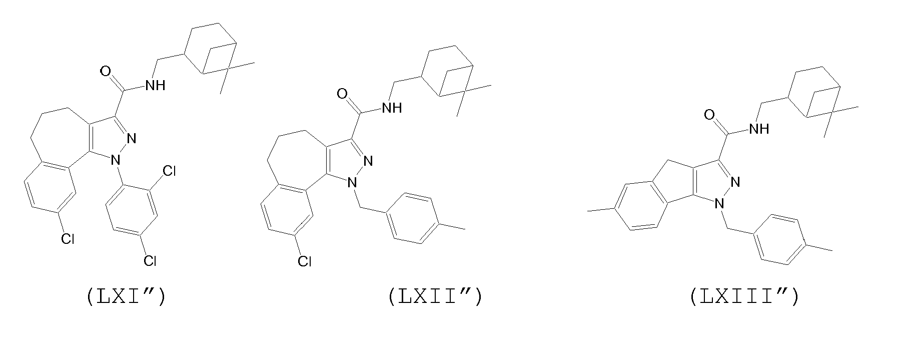

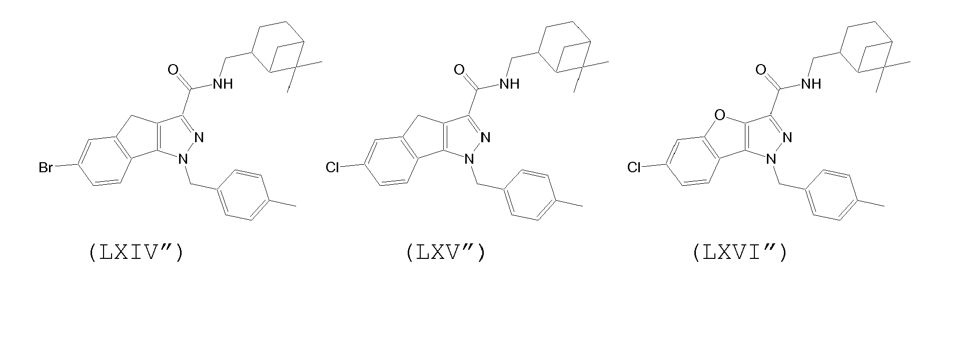

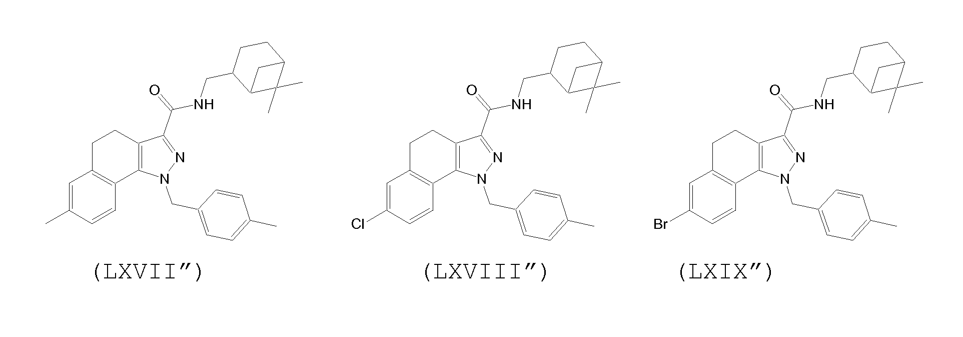

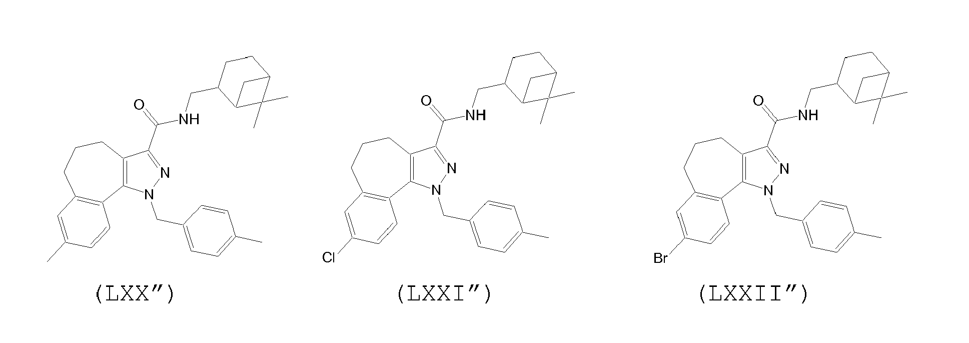

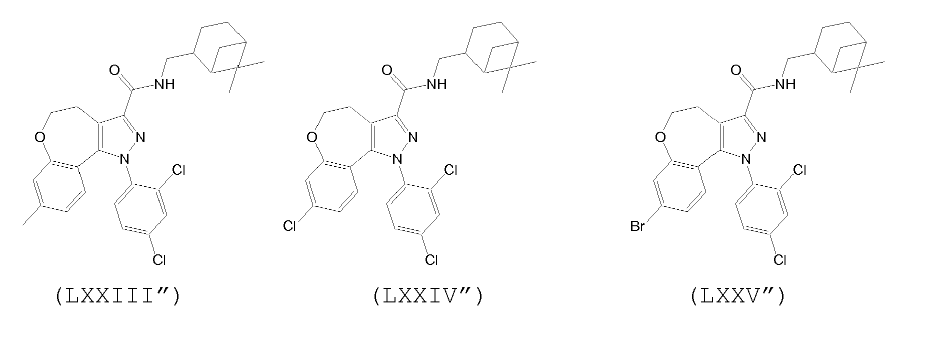

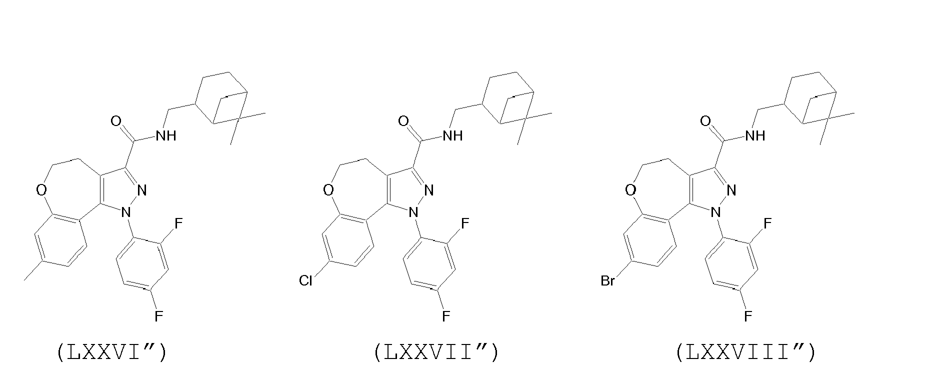

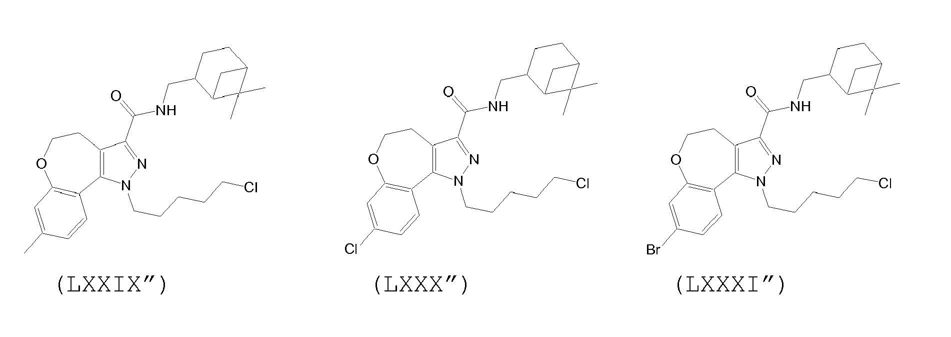

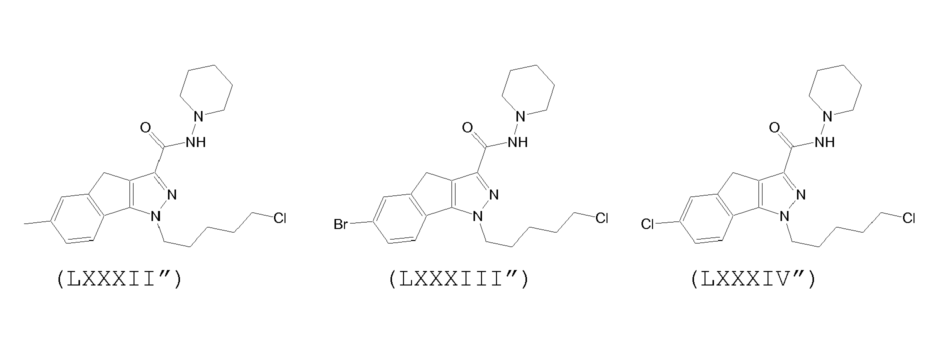

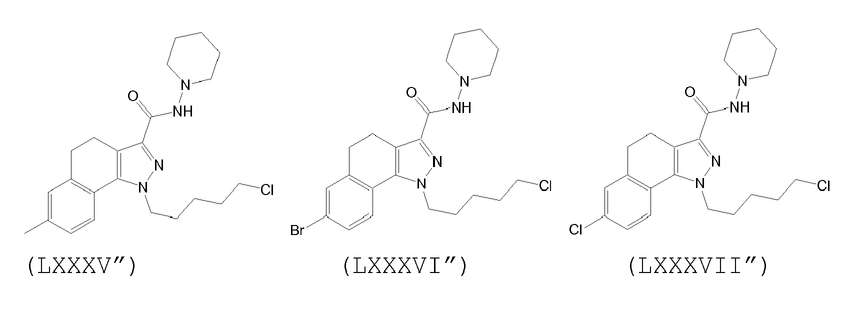

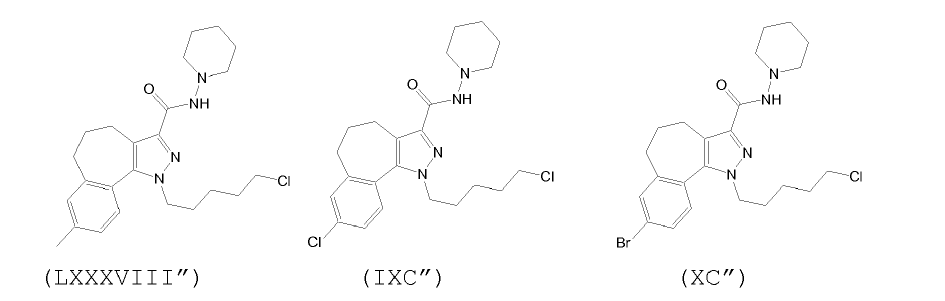

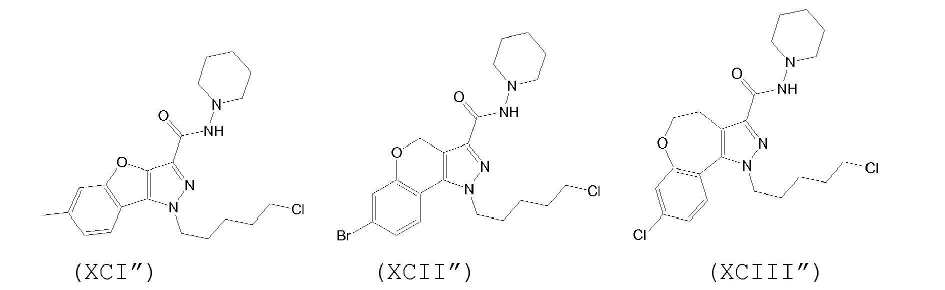

14. Compounds according to claim 13, selected from the following:
(XVI"), (XVII"), (XVIII"), (XIX"), (XX"), (XXI"), (XXXIV"), (XXXV"), (XXXVI"), (XXXVII"), (XLVI"), (XLVII"), (XLVI"), (XLVII"), (XLVIII"), (1XLVI"), (1XLVII"), (1XLVIII"), (IL"), (LVIII"), (LVIX"), (LX"), (LXXIX"), (LXXX"), (LXXXI"), (LXXXII"), (LXXXIII"), (LXXXIV"), (LXXXV"), (LXXXVI"), (LXXXVII"), (LXXXVIII"), (IXC"), (XC"), (XCI"), (XCII"), (XCII").

15. Hydrates, solvates and pharmaceutically acceptable salts of the compounds according to claims 1-14.

16. Metabolites of the compounds of claims 1-15 administered to an individual or to an animal.

17. A process for preparing the compounds of claims 1-15 comprising:
i) synthesis of the acid of the formula (II), or optionally of a reactive derivative thereof selected from acyl halides, anhydrides, mixed anhydrides, imidazolides, ester-amide adducts or linear or branched C₁-C₄ alkyl esters: 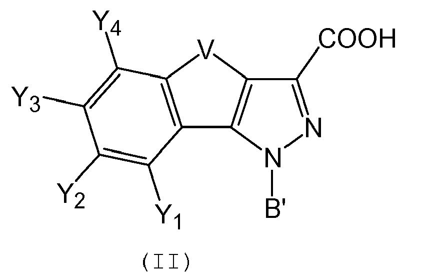 comprising the following steps:
- preparation of α-hydroxy-γ-ketoesters of formula (IV), wherein V, Y₁, Y₂, Y₃ and Y₄ are as above defined, by reacting a compound of formula (III) with sodium alkoxide (RONa) and diethyloxalate in a C₁-C₃ alcoholic solvent, at reflux: 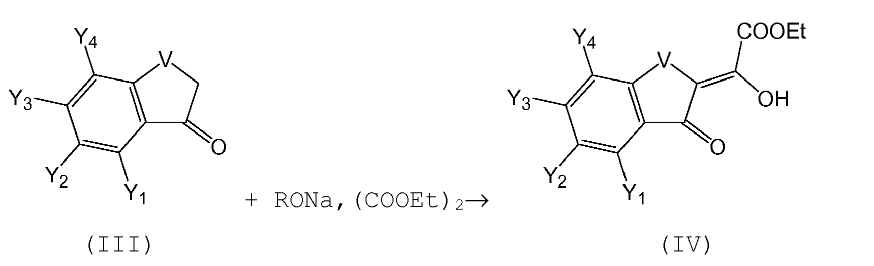
- reaction of the compounds of formula (IV) with an hydrazine of formula (VI) wherein B' is as above defined, said compound (VI) being optionally in the form of an hydrochloric salt in an alcoholic solvent or in acetic acid, at reflux, to yield the tricyclic compound of formula (VII): 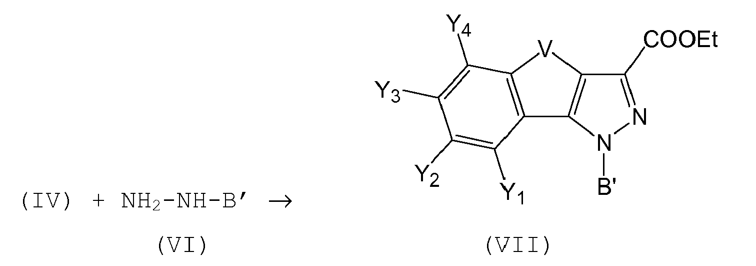
- alkaline hydrolysis with alkaline hydroxides in an hydroalcoholic solution of the compound of formula (VII), at reflux, to yield the acid of formula (II);
- optionally, preparation of a reactive derivative of the acid of formula (II),
ii) when in formula (I) D' is a substituent having an ethereal group of formula D1, the acid of formula (II) or its ester thereof, is reduced in a first step to primary alcohol with an organic metal hydride, then in a second step the primary alcohol is reacted at room temperature with an alkyl halide of formula R"-(Z')ᵥ - (CH₂)_{z}-Hal, wherein Hal is halogen and Z', v and z are as above defined, in the presence of an alkaline hydride, obtaining the compounds of formula (I) wherein D'=D1,
iii) when in formula (I) D'=D2, the compounds can be prepared according to one of the following processes:
first process, comprising:
- reaction of one ester of the acid of formula (II) with trialkylaluminum and with the hydrochloride salt of an amine, subsequent addition to the reaction mixture of R"-(2')ᵥ-MgBr, wherein Z', v and R" are as defined above, obtaining the compound of formula (I) with D'= D2, second process, comprising:
- reaction of the acid of formula (II), or a reactive derivative thereof, with a metallorganic salt of formula (R"-(Z')ᵥ)⁻ Me⁺, wherein Me⁺ is an alkaline metal cation obtaining the compound of formula (I) with D'=D2,
iiii) when in formula (I) D'=D3 the synthesis comprises the following two steps:
- preparation of the compound of formula (I)
wherein D'=D2, by using one of the two processes described in iii),
- reduction of the compound obtained in the preceding step and isolation of the final product with D'=D3,
iiiii) when in formula (I) D'=D4, the compounds are prepared by reaction of a reactive derivative of the acid of formula (II) with a compound of formula:
H₂N-(2')ᵥ-T' (VIIA)
wherein Z', v and T' are as defined.

18. Compounds of formula (II'): 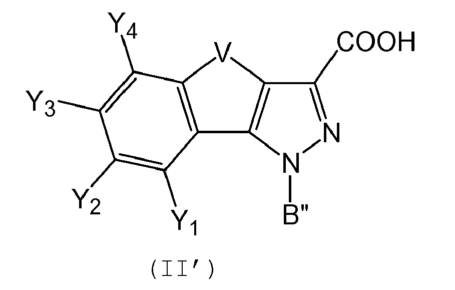 wherein:
V, Y₁, Y₂, Y₃ and Y₄ are as defined above, B" is hydrogen or a bivalent C₁-C₁₀ aliphatic chain, linear or branched when possibile, wherein the chain end not linked to the nitrogen atom is linked to W^{II}, selected from hydrogen, halogen, isothiocyanate, CN, OH, OCH₃, NH₂, SO₂NH₂ or -CH=CH₂.

19. Compounds according to claim 18, wherein:
V is selected from A1 or A2,
B" is as defined above,
Y₁, Y₂, Y₃ and Y₄, equal to or different from each other, are selected from hydrogen, halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂, isothiocyanate, phenyl, cycloalkyl, saturated or unsaturated heterocycle, heteraryl, amino optionally mono- or bisubstituted with a C₁-C₃ alkyl chain, said phenyl, cycloalkyl, saturated or unsaturated heterocycle and heteroaryl being optionally substituted with one or more groups, equal to or different from each other, selected from halogen, C₁-C₃ alkyl, C₁-C₃ alkylthio, C₁-C₃ alkoxy, C₁-C₃ haloalkyl, C₁-C₃ haloalkoxy, cyano, nitro, SO₂NH₂, phenyl, cycloalkyl, saturated or unsaturated heterocycle, heteroaryl, isothiocyanate, or amino, optionally mono- or bisubstituted with a C₁-C₇ alkyl chain.

20. Compounds according to claims 1-15 for use as a medicament.

21. Pharmaceutical compositions comprising the compounds of claims 1-15.

22. Pharmaceutical compositions for oral administration according to claim 21 comprising 0.5-20% by weight of a compound of formula (I) 0.05-0.5% by weight of sodium alkylsulphate or another surfactant, 2.5-10% by weight of a disintegrating agent, the complement to 100% given by conventional excipients.

23. Pharmaceutical compositions according to claim 21 for both oral and intraocular administration, comprising from 0.1 to 20% of the compounds of formula (I), from 0.5 to 10% of hydroxypropyl methyl cellulose, the complement to 100% given by conventional excipients, when the pharmaceutical composition is a tablet, hydroxypropyl methyl cellulose in the core and/or in the shell.

24. Pharmaceutical compositions according to claim 21 in the form of emulsions comprising (% by weight):
- from 0.005 to 20% of compounds of formula (I), their isomers or the corresponding hydrates or solvates or pharmaceutically acceptable salts,
- from 0 to 50% of one or more oils,
- from 0.01 to 50% of one or more amphiphilic compounds,
- from 0 to 50% of additives,
- from 0.01 to 99.9% of water or a saline aqueous solution, optionally buffered,
the sum of the components of the emulsions being 100%.

25. Pharmaceutical compositions according to claim 21 comprising micro- and/or nano-particles of lipids, or of pharmaceutically acceptable polymers, said particles being optionally modified on the surface, the particles comprising an amount of compounds of formula (I) between 0.01 and 60% by weight of lipids, or of polymers, the lipids or the polymers are inside and/or on the surface of the particles.

26. Use of the compounds of claims 1-15 for preparing pharmaceutical compositions for the prophylaxis and therapy in mammals and in an individual of the diseases and disorders in which the receptors of CB1 and/or CB2 cannabinoids are involved.

27. Use according to claim 26, wherein the diseases and disorders are the following: diseases involving immune system cells, immune disorders, osteoporosis, renal ischaemia, inflammatory states, pain, post operating pain, neuropathic pain, eye diseases, pulmonary diseases, asthma, chronic bronchitis, inflammation states, arthritis, allergies and allergic reactions, allergic rhinitis, contact dermatitis, allergic conjunctivitis, anxiety, behavioural problems, delirium states, psychotic problems, schizophrenia, depression, use of addiction substances, alcoholism, tabagism, vomit, nausea, vertigo, in particular for patients under chemotherapy, neuropathies, hemicrania, stress, diseases of psychosomatic origin, epilepsy, Tourette syndrome, Parkinson disease, Huntington disease, Alzheimer disease, senile dementia, cognition disorders and memory loss, pathologies associated to food intake, obesity, bulimia, gastrointestinal tract and bladder pathologies, cardiovascular diseases, urinary diseases, erection and fertility disorders, neuroinflammatory pathologies, multiple sclerosis, Guillain-Barré syndrome, viral encephalitis, amyotrophic lateral sclerosis, syndrome associated to demineralization, osteoporosis, in reducing metabolic and/or cardiovascular risk factors, also in patients with metabolic syndrome and/or dyslipidemia and in patients with type 2 diabetes, eye inflammatory conditions, eye autoimmune diseases, uveitis, uveoretinitis and retina neurodegeneration.

28. Use of the compounds according to claims 1-15 containing radioactive isotopes, or the pharmaceutical formulations thereof, for identifying and labelling the receptors of the CB1 and/or CB2 cannabinoids in mammals or in an individual.

29. Use of the compounds according to claims 1-15 comprising in the molecule an hydroxylic group for obtaining ligands of the cannabinoidergic receptors.

30. Use according to claim 28 wherein the diseases and disorders are those of claim 27.
